# EUROPEAN PATENT SPECIFICATION

(11) **EP 4 655 283 B1**
(45) Date of publication and mention of the grant of the patent: **09.12.2026**
(21) Application number: 24701632.2
(22) Date of filing: 22.01.2024
(51) Int. Cl.: C07D 209/42, A61K 31/4468, A61P 11/06, A61P 17/00, A61P 19/00, A61P 29/00, A61P 37/00, C07D 211/96, C07D 295/26, C07D 401/12, C07D 401/14, C07D 405/12, C07D 471/04, C07D 471/10, C07D 487/04, C07D 491/107, C07D 498/10

(54) **N-PHENYL-1-(PHENYLSULFONYL)PIPERIDIN-4-AMINE DERIVATIVES AS CCR6 INHIBITORS**
N-PHENYL-1-(PHENYLSULFONYL)PIPERIDIN-4-AMINDERIVATE ALS CCR6-INHIBITOREN
DÉRIVÉS DE N-PHÉNYL-1-(PHÉNYLSULFONYL)PIPÉRIDIN-4-AMINE UTILISÉS EN TANT QU'INHIBITEURS DE CCR6

(30) Priority: 24.01.2023 EP 23153061; 22.12.2023 EP 23220031
(43) Date of publication of application: 03.12.2025
(73) Proprietor: F. Hoffmann-La Roche AG, 4070 Basel (CH)
(72) Inventor: BAUTISTA SARMIENTO, Lady Johanna, 5349 AB Oss (NL); CALS, Joseph Maria Gerardus Barbara, 5349 AB Oss (NL); DE BROUWER, Johanna Hendrika Angelique, 5349 AB Oss (NL); DE KIMPE, Vera, 5349 AB Oss (NL); DERETEY, Eugen, 5349 AB Oss (NL); LAZO MORI, Alger Ayrton, 5349 AB Oss (NL); MANDERS, Daan Marten Jorrit, 5349 AB Oss (NL); NABUURS, Sander Bernardus, 5349 AB Oss (NL)
(74) Representative: Schirlin, Julien
(86) International application number: PCT/EP2024/051362
(87) International publication number: WO 2024/156630

(56) References cited:
- WO-A1-2022/173849

## Description

### BACKGROUND

Immune surveillance, the migration of immune cells throughout the body is a tightly regulated process that is involved in many aspects of health and disease. Chemokines, and their corresponding receptors, play critical roles in these trafficking patterns, they are responsible for getting the right cells into the right tissues (Griffith, J. W., Sokol, C. L. & Luster, A. D. (2014). Chemokines and Chemokine Receptors: Positioning Cells for Host Defense and Immunity. Immunology, 32(1), 659-702 and Zlotnik, A. & Yoshie, O. (2012). The Chemokine Superfamily Revisited. Immunity, 36(5), 705-716).

Chemokines, or chemotactic cytokines, are a family of around 50 small signaling proteins secreted by a variety of cell populations (David, B. A. & Kubes, P. (2019). Exploring the complex role of chemokines and chemoattractants in vivo on leukocyte dynamics. Immunological Reviews, 289(1), 9-30 and Griffith, J. W., Sokol, C. L. & Luster, A. D. (2014). Chemokines and Chemokine Receptors: Positioning Cells for Host Defense and Immunity. Immunology, 32(1), 659-702). Chemokines are divided into four main subfamilies, called CC, CXC, CX3C and C, based on the location of the canonical cysteine residues in the N-terminal region. Chemokine secretion and diffusion create concentration gradients that direct the migration of cells expressing the corresponding receptors. Chemokine receptors are a family of around 20 seven transmembrane proteins differentially expressed on the surface of immune cells and can be divided into two main subfamilies, the first is called G protein-coupled chemokine receptors, which mediate immune cell trafficking, and the second is called atypical chemokine receptors, which seem to be chemokine scavengers that influence the chemokine gradients. They are also grouped into four subfamilies according to the subfamily of their major chemokine ligands. In some cases, one chemokine can signal through multiple receptors and, in many cases, one receptor can be stimulated by multiple chemokines. These promiscuous interactions make pharmacological intervention of signaling more complicated.

CCR6, also called CD196, is a chemokine receptor expressed on a variety of adaptive and innate immune cells including B cells, T cells, dendritic cells and neutrophils. For example, TH17 cells, which play a critical role in the pathogenesis of multiple autoimmune diseases, express CCR6 and this signal has been shown to recruit these cells into inflamed peripheral tissues ( Esplugues, E., Huber, S., Gagliani, N., Hauser, A. E., Town, T., Wan, Y. Y., O'Connor, W., Rongvaux, A., Rooijen, N. V., Haberman, A. M., Iwakura, Y., Kuchroo, V. K., Kolls, J. K., Bluestone, J. A., Herold, K. C. & Flavell, R. A. (2011). Control of TH17 cells occurs in the Small Intestine. Nature, 475(7357), 514-518 and Singh, S. P., Zhang, H. H., Foley, J. F., Hedrick, M. N. & Farber, J. M. (2008). Human T Cells That Are Able to Produce IL-17 Express the Chemokine Receptor CCR6. The Journal of Immunology, 180(1), 214-221). The ligand for CCR6 is CCL20, also called macrophage inflammatory protein 3 alpha (MIP-3 alpha) and liver and activation-regulated chemokine (LARC). The CCR6/CCL20 pair is somewhat unique because they have only one binding partner and therefore form a pharmacologically selective receptor-ligand pair (Schutyser, E., Struyf, S. & Damme, J. V. (2003). The CC chemokine CCL20 and its receptor CCR6. Cytokine & Growth Factor Reviews, 14(5), 409-426).

CCL20 expression and secretion is increased in the presence of inflammatory stimuli. High levels of CCL20 can be found in the inflamed tissue associated with multiple inflammatory autoimmune diseases including psoriatic diseases, asthma, Crohn's disease, ulcerative colitis, rheumatoid arthritis, systemic lupus erythematosus and multiple sclerosis (Richmond, J. M., Strassner, J. P., Essien, K. I. & Harris, J. E. (2019). T-cell positioning by chemokines in autoimmune skin diseases. Immunological Reviews, 289(1), 186-204, Lee, A. Y. & Körner, H. (2014). CCR6 and CCL20: emerging players in the pathogenesis of rheumatoid arthritis. Immunology and Cell Biology, 92(4), 354-358, Raman, D., Sobolik-Delmaire, T. & Richmond, A. (2011). Chemokines in health and disease. Experimental Cell Research, 317(5), 575-589, Pène, J., Chevalier, S., Preisser, L., Vénéreau, E., Guilleux, M.-H., Ghannam, S., Molès, J.-P., Danger, Y., Ravon, E., Lesaux, S., Yssel, H. & Gascan, H. (2008). Chronically Inflamed Human Tissues Are Infiltrated by Highly Differentiated Th17 Lymphocytes. The Journal of Immunology, 180(11), 7423-7430 and Schutyser, E., Struyf, S. & Damme, J. V. (2003). The CC chemokine CCL20 and its receptor CCR6. Cytokine & Growth Factor Reviews, 14(5), 409-426).

Genetic linkage, clinical association and preclinical studies highlight a critical role for CCR6 in these inflammatory diseases (Hamburg, J. P. van & Tas, S. W. (2018). Molecular mechanisms underpinning T helper 17 cell heterogeneity and functions in rheumatoid arthritis. Journal of Autoimmunity, 87, 69-81 and Kurkó, J., Besenyei, T., Laki, J., Glant, T. T., Mikecz, K. & Szekanecz, Z. (2013). Genetics of Rheumatoid Arthritis - A Comprehensive Review. Clinical Reviews in Allergy & Immunology, 45(2), 170-179). For example, CCR6 gene variants have the highest risk association for Crohn's disease (CD) amongst the chemokine receptor family (Lee, A. Y. S., Eri, R., Lyons, A. B., Grimm, M. C. & Korner, H. (2013). CC Chemokine Ligand 20 and Its Cognate Receptor CCR6 in Mucosal T Cell Immunology and Inflammatory Bowel Disease: Odd Couple or Axis of Evil, Frontiers in Immunology, 4, 194).

This high selectivity renders CCR6 an attractive drug target. Selective CCR6 inhibitors would only result in on-target pharmacology.

PCT application WO 2022/173849 discloses N-(4-(phenylsulfonyl)cyclohexyl)aniline derivatives as CCR6 inhibitors useful for the treatment of autoimmune diseases.

### BRIEF SUMMARY

The invention is set out in the appended set of claims.

A first object of the present invention is a compound of formula (I) 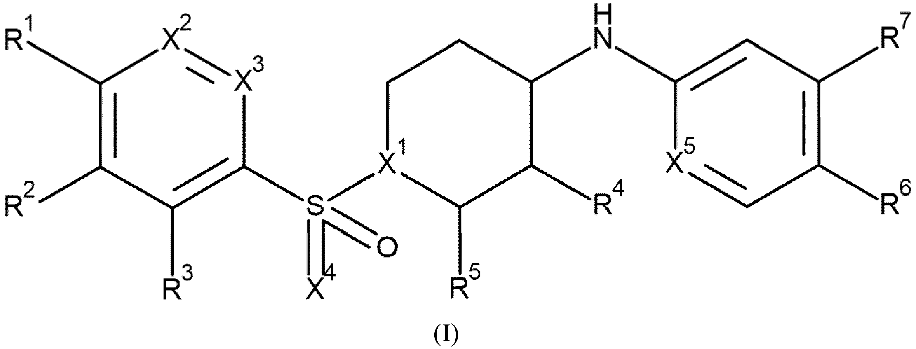 wherein
X¹ is CH or N;
X² is CH or N;
X³ is CH or N;
X⁴ is O or NH;
X⁵ is CH or N;
R¹ is aryl, heterocyclyl, or heteroaryl, wherein aryl, heterocyclyl, and heteroaryl are optionally substituted with one or two, in particular one R^{1a};
R^{1a} is C₁₋₆alkyl, C₁₋₆haloalkyl, oxo, cyano, -CONHR^{1b}, C₃₋₆cycloalkyl, or heterocyclyl;
R^{1b} is C₁₋₆alkyl, or hydrogen;
R² is hydrogen or halogen;
R³ is hydrogen or halogen;
R⁴ is hydrogen, halogen or C₁₋₆alkyl;
R⁵ is hydrogen, halogen or C₁₋₆alkyl;
R⁶ is C₃₋₆cycloalkyl or heterocyclyl, wherein C₃₋₆cycloalkyl and heterocyclyl are optionally substituted with one or two, in particular one R^{6a};
R^{6a} is C₁₋₆haloalkyl, cyano, or halogen
R⁷ is hydrogen;
or a pharmaceutically acceptable salt thereof.

An object of the present disclosure is a compound of formula (I) 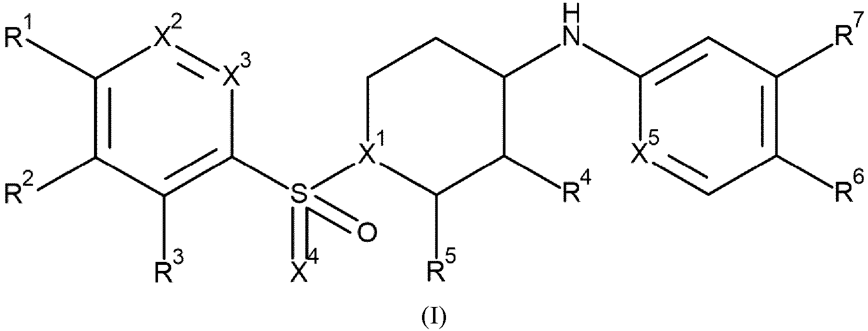 wherein
X¹ is CH or N;
X² is CH or N;
X³ is CH or N;
X⁴ is O or NH;
X⁵ is CH or N;
R¹ is aryl or heteroaryl, wherein aryl and heteroaryl are optionally substituted with one or two, in particular one R^{1a};
R^{1a} is C₁₋₆alkyl, oxo, cyano, -CONHR^{1b}, C₃₋₆cycloalkyl, or heterocyclyl;
R^{1b} is C₁₋₆alkyl, or hydrogen;
R² is hydrogen or halogen;
R³ is hydrogen or halogen;
R⁴ is hydrogen, halogen or C₁₋₆alkyl;
R⁵ is hydrogen, halogen or C₁₋₆alkyl;
R⁶ is C₃₋₆cycloalkyl or heterocyclyl, wherein C₃₋₆cycloalkyl and heterocyclyl are optionally substituted with one or two, in particular one R^{6a};
R^{6a} is C₁₋₆haloalkyl, cyano, or halogen
R⁷ is hydrogen,
and pharmaceutically acceptable salts thereof.

An object of the present disclosure is a process of preparation of a compound of formula (I) as defined above, or a pharmaceutically acceptable salt thereof, wherein X¹ is N, and X⁴ is O, comprising:
reacting compound of formula (II), wherein R⁵ and R⁴ are as defined above, 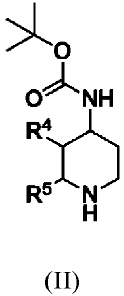
with compound of formula (III), wherein X², X³, R², and R³, are as defined above, 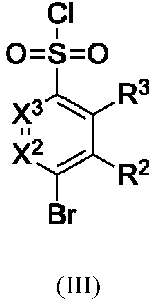
to form compound (IV), wherein R², R³, R⁴, R⁵, X², and X³ are as defined above, 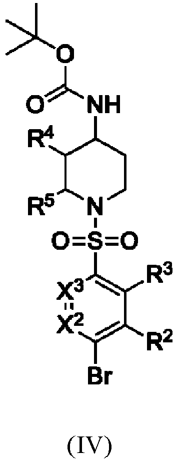
reacting said compound (IV) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 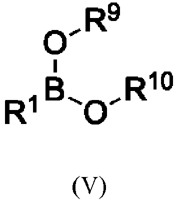
to form compound of formula (VI), wherein R¹, R², R³, R⁴, R⁵, X², and X³ are as defined above, 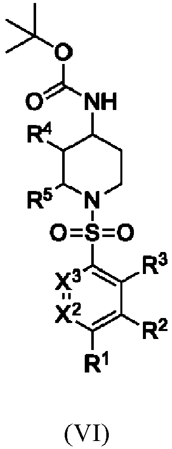
reacting said compound of formula (VI) with an acid, to form compound of formula (VII), wherein R¹, R², R³, R⁴, R⁵, X², and X³ are as defined above, 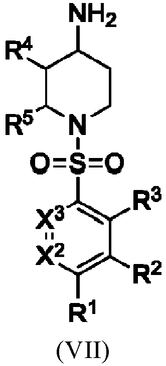
reacting said compound of formula (VII) with compound of formula (VIII), wherein R⁸ is a halogen, and R⁶, R⁷, and X⁵ are as defined above, 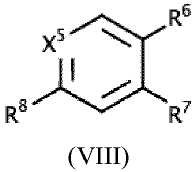
to form compound of formula (I).

An object of the present disclosure is a process of preparation of a compound of formula (I) as defined above, or a pharmaceutically acceptable salt thereof, wherein X¹ is N and X⁴ is O, comprising:
reacting compound of formula (IX), wherein R⁴ and R⁵ are as defined above, 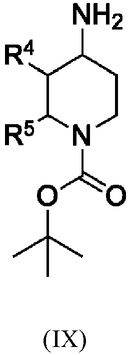
with compound of formula (VIII), wherein R⁸ is a halogen and R⁶, R⁷, and X⁵ are as defined above, 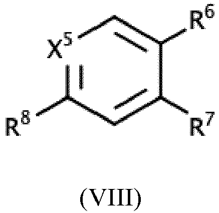
to form compound of formula (X), wherein R⁴, R⁶, R⁷, X⁵, and R⁵ are as defined above, 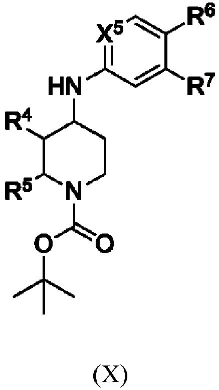
reacting said compound of formula (X) with acid to form compound of formula (XI), wherein R⁴, R⁶, R⁷, X⁵, and R⁵ are as defined above, 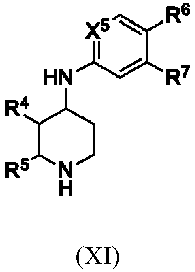
reacting said compound of formula (XI) with compound of formula (III), wherein X², X³, R², and R³, are as defined above, 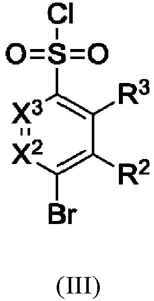
to form compound of formula (XII), wherein X², X³, R², R³, R⁴, R⁶, R⁷, X⁵, and R⁵ are as defined above, 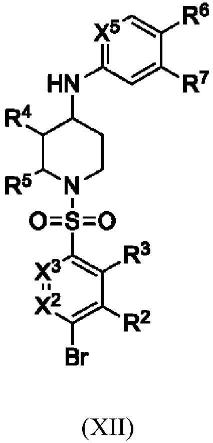
reacting said compound of formula (XII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 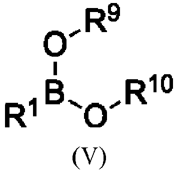
to form compound of formula (I).

An object of the present disclosure is a process of preparation of a compound of formula (I) as defined above, or a pharmaceutically acceptable salt thereof, wherein X¹ is N and X⁴ is O, comprising:
reacting compound of formula (XVIII), wherein R⁵ and R⁴ are as defined above, 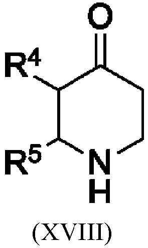
with compound of formula (XVII), wherein R¹, R², R³, X², and X³ are as defined above, 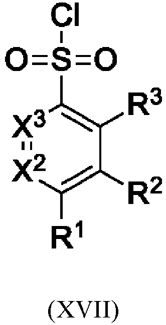
to form compound of formula (XX), wherein R¹, R², R³, R⁴, R⁵, X², and X³ are as defined above, 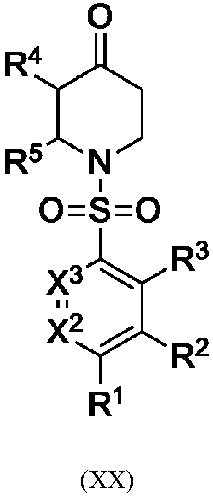
reacting said compound of formula (XX) with compound of formula (XVI), wherein X⁵, R⁶, and R⁷ are as defined above, 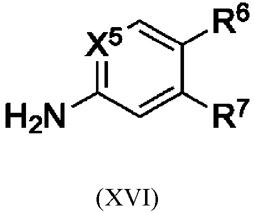
to form compound of formula (I);
   or
reacting compound of formula (XVIII), wherein R⁵ and R⁴ are as defined above, 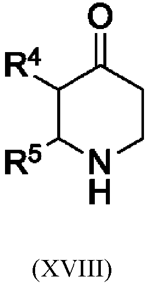
with compound of formula (III), wherein X², X³, R², and R³, are as defined above, 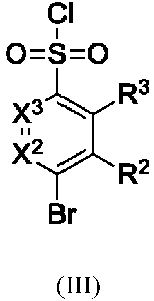
to form compound of formula (XIX), wherein R², R³, R⁴, R⁵, X², and X³ are as defined above, 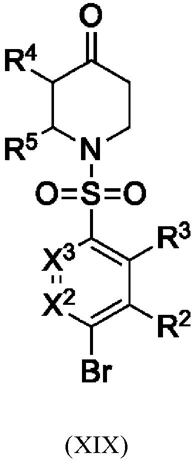
reacting said compound of formula (XIX) with said compound of formula (XVI), to form compound of formula (XII), wherein X², X³, R², R³, R⁴, R⁶, R⁷, X⁵, and R⁵ are as defined above, 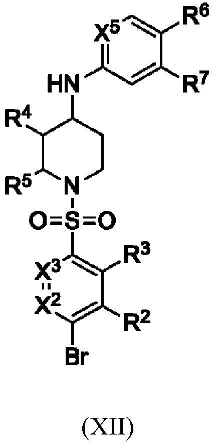
reacting said compound of formula (XII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 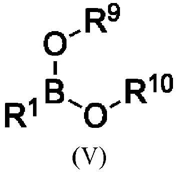
to form compound of formula (I).

An object of the present disclosure is a process of preparation of a compound of formula (I) as defined above, or a pharmaceutically acceptable salt thereof, wherein X¹ is C and X⁴ is O, comprising:
reacting compound of formula (XXI), wherein R⁴ and R⁵ are as defined above, 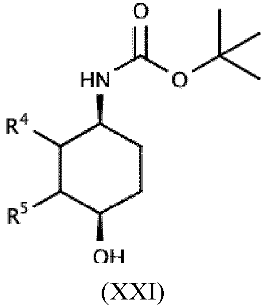
with mesyl chloride, to form compound of formula (XXII), wherein R⁴ and R⁵ are as defined above, 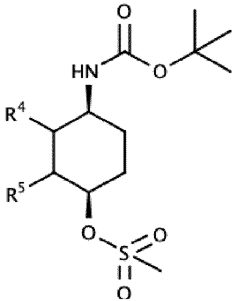 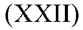
reacting said compound of formula (XXII) with compound of formula (XXIII), wherein R², R³, X², and X³ are as defined above, 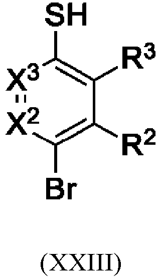
to form compound of formula (XXIV), wherein R², R³, R⁴, R⁵, X², and X³ are as defined above, 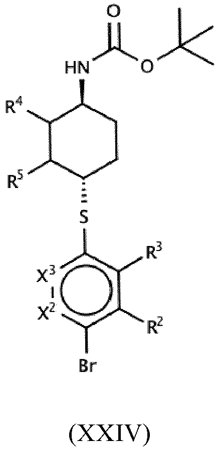
reacting said compound of formula (XXIV) with an oxidant, to form compound of formula (XXV), wherein R², R³, R⁴, R⁵, X², and X³ are as defined above, 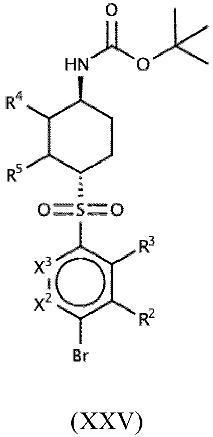
reacting said compound of formula (XXV) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 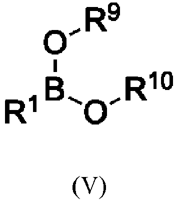
to form compound of formula (XXVI), wherein R¹, R², R³, R⁴, R⁵, X², and X³ are as defined above, 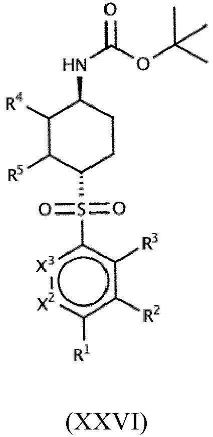
reacting said compound of formula (XXVI) with acid, to form compound of formula (XXVII), wherein R¹, R², R³, R⁴, R⁵, X², and X³ are as defined above, 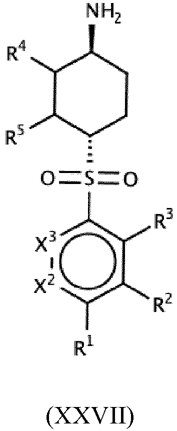
reacting said compound of formula (XXVII) with compound of formula (VIII), wherein R⁸ is a halogen and R⁶, R⁷, and X⁵ are as defined above, 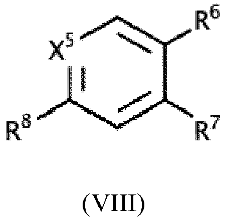
to form compound of formula (I).

An object of the present disclosure is a process of preparation of a compound of formula (I) as defined above, or a pharmaceutically acceptable salt thereof, wherein X¹ is C and X⁴ is O, comprising:
reacting compound of formula (XXV), wherein R², R³, R⁴, R⁵, X², and X³ are as defined above, 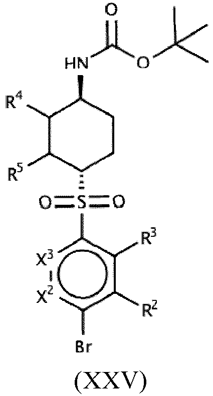
with acid to form compound of formula (XXVIII), wherein R², R³, R⁴, R⁵, X², and X³ are as defined above, 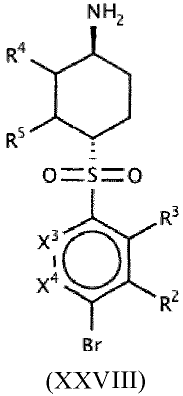
reacting said compound of formula (XXVIII) with compound of formula (VIII), wherein R⁸ is a halogen and R⁶, R⁷, and X⁵ are as defined above, 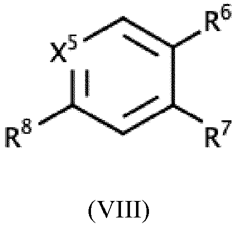
to form compound of formula (XXIX), wherein R², R³, R⁴, R⁵, R⁶, R⁷, X⁵, X², and X³ are as defined above, 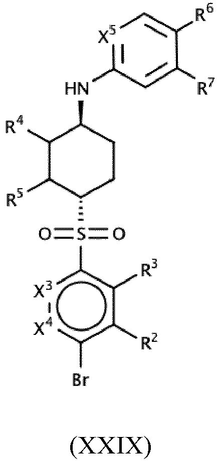
reacting said compound of formula (XXIX) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 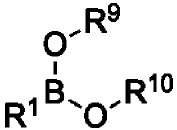 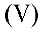
to form compound of formula (I).

An object of the present disclosure is a process of preparation of a compound of formula (I) as defined above, or a pharmaceutically acceptable salt thereof, wherein X¹ is C and X⁴ is O, comprising:
reacting compound of formula (XXXI), wherein R⁴ and R⁵ are as defined above, 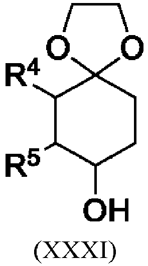
with mesyl chloride, to form compound of formula (XXXII), wherein R⁴ and R⁵ are as defined above, 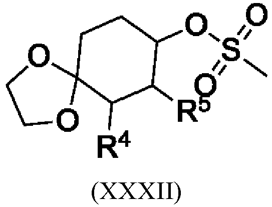
reacting said compound of formula (XXXII) with compound of formula (XXIII), wherein R², R³, X², and X³ are as defined above, 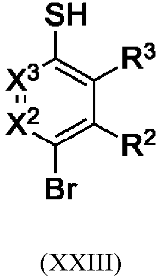
to form compound of formula (XXXIII), wherein R², R³, R⁴, R⁵, X², and X³ are as defined above, 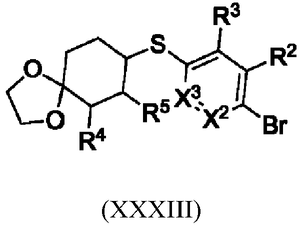
reacting said compound (XXXIII) with an oxidant, to form compound of formula (XXXIV), wherein R², R³, R⁴, R⁵, X², and X³ are as defined above, 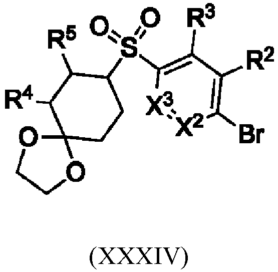
reacting said compound (XXXIV) with an acid, to form compound of formula (XXXV), wherein R², R³, R⁴, R⁵, X², and X³ are as defined above, 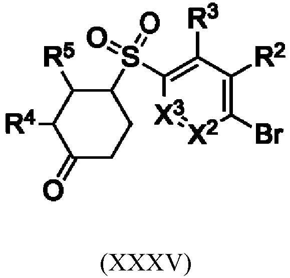
reacting said compound of formula (XXXV) with compound of formula (XVI), wherein X⁵, R⁶, and R⁷ are as defined above, 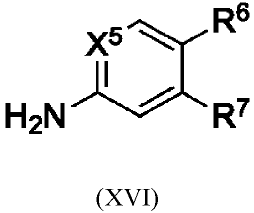
to form compound of formula (XXIX), wherein R², R³, R⁴, R⁵, R⁶, R⁷, X⁵, X², and X³ are as defined above, 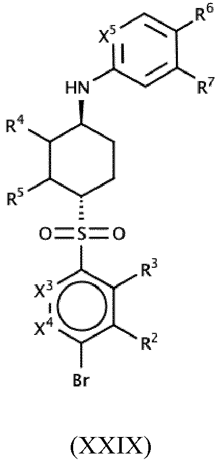
reacting said compound of formula (XXIX) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 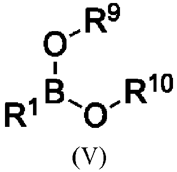
to form compound of formula (I).

An object of the present disclosure is a process of preparation of a compound of formula (I) as defined above, or a pharmaceutically acceptable salt thereof, wherein X¹ is C and X⁴ is N, comprising:
reacting compound of formula (XXXIII), wherein R², R³, R⁴, R⁵, X², and X³ are as defined above, 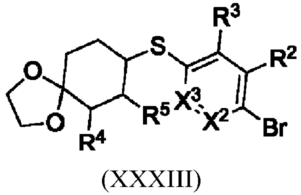
with an acid to form compound of formula (XXXVI), wherein R², R³, R⁴, R⁵, X², and X³ are as defined above, 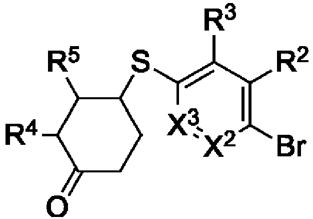 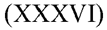
reacting said compound of formula (XXXVI) with compound of formula (XVI), wherein X⁵, R⁶, and R⁷ are as defined above, 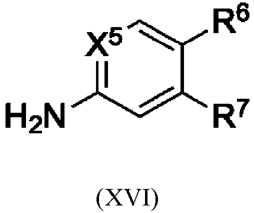
to form compound of formula (XXXVII), wherein R², R³, R⁴, R⁵, R⁶, R⁷, X⁵, X², and X³ are as defined above, 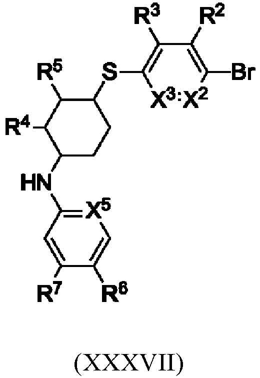
reacting said compound of formula (XXXVII) with ammonium carbamate and (diacetoxyiodo)benzene, to form compound of formula (XXXVIII), wherein R², R³, R⁴, R⁵, R⁶, R⁷, X⁵, X², and X³ are as defined above, 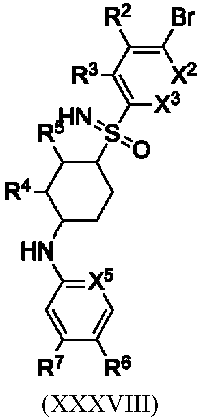
reacting said compound of formula (XXXVIII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 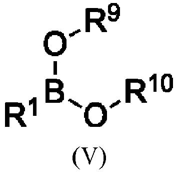
to form compound of formula (I).

An object of the present disclosure is a process of preparation of a compound of formula (I) as defined above, or a pharmaceutically acceptable salt thereof, wherein X¹ and X⁴ are N, comprising:
reacting compound of formula (XI), wherein R⁴, R⁶, R⁷, X⁵, and R⁵ are as defined above, 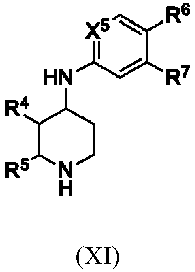
with compound of formula (XXIII), wherein R², R³, X², and X³ are as defined above, 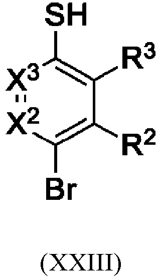
to form compound of formula (XL), wherein R², R³, X², X³, R⁴, R⁶, R⁷, X⁵, and R⁵ are as defined above, 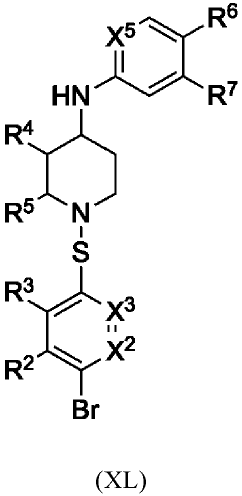
reacting said compound (XL) with ammonium carbamate and (diacetoxyiodo)benzene, to form compound of formula (XLI), wherein R², R³, X², X³, R⁴, R⁶, R⁷, X⁵, and R⁵ are as defined above, 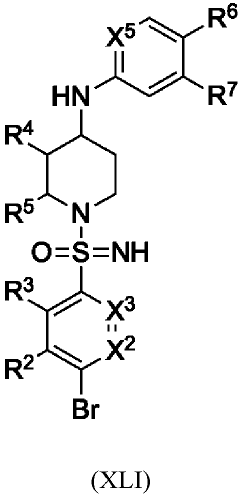
reacting said compound (XLI) with di-tert-butyl dicarbonate and a base, to form compound of formula (XLII), wherein R², R³, X², X³, R⁴, R⁶, R⁷, X⁵, and R⁵ are as defined above, 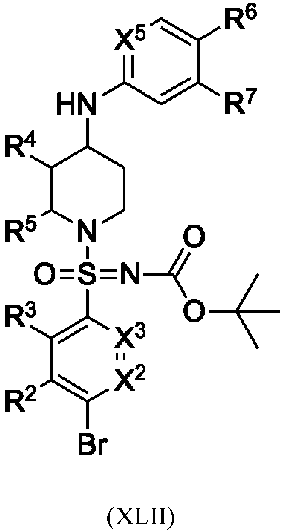
reacting said compound (XLII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 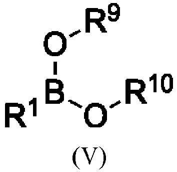
to form compound of formula (XLIII), wherein R¹, R², R³, X², X³, R⁴, R⁶, R⁷, X⁵, and R⁵ are as defined above, 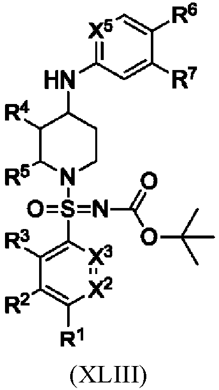
reacting said compound of formula (XLIII) with an acid to form compound of formula (I).

An object of the present invention is a pharmaceutical composition comprising a compound of formula (I) as described above, or a pharmaceutically acceptable salt thereof, and a pharmaceutically acceptable excipient.

An object of the current invention is a compound of formula (I), as described above, or a pharmaceutically acceptable salt thereof, for use in the treatment, prevention and/or delay of inflammatory autoimmune disease.

Herein disclosed is a method for the treatment, prevention and/or delay of progression of inflammatory autoimmune disease, which method comprises administering a therapeutically effective amount of a compound of formula (I), or a pharmaceutically acceptable salt thereof.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below.

The nomenclature used in this application is based on IUPAC systematic nomenclature, unless indicated otherwise.

The references to methods of treatment in the subsequent paragraphs of this description are to be interpreted as references to the compounds or pharmaceutical compositions of the present invention for use in a method for treatment of the human or animal body by therapy.

### DEFINITIONS

"Acid" refers to a compound capable of giving proton of Broensted's definition, dissociating into proton and counter ion in water at 25°C and giving a solution having neutral pH or below. Concrete examples of the acid are phosphoric acid (orthophosphoric acid), sulfuric acid, nitric acid, phosphinic acid, phosphonic acid, diphosphonic acid, hydrochloric acid, pyrophosphoric acid, metaphosphoric acid and nitrous acid. These acids may be used in the form of metal salts, ammonium salts or the like; particularly acid means hydrochloric acid.

"Amino", alone or in combination with other groups, refers to NH₂.

"Aromatic" refers to the conventional idea of aromaticity as defined in the literature, in particular in IUPAC - Compendium of Chemical Terminology, 2nd Edition, A. D. McNaught & A. Wilkinson (Eds). Blackwell Scientific Publications, Oxford (1997).

"Aryl" refers to a cyclic aromatic hydrocarbon moiety having a mono-, bi- or tricyclic aromatic ring of 5 to 14 carbon ring atoms ("C₅₋₁₄-aryl"). Bicyclic aryl ring systems include fused bicyclics having two fused five-membered aryl rings (denoted as 5-5), having a five-membered aryl ring and a fused six-membered aryl ring (denoted as 5-6 and as 6-5), and having two fused six-membered aryl rings (denoted as 6-6). The aryl group can be optionally substituted as defined herein. Examples of aryl moieties include, but are not limited to, phenyl, naphthyl, phenanthryl, fluorenyl, indenyl, pentalenyl, azulenyl, and the like. In particular aryl means phenyl.

"C₁₋₆alkyl" refers to a saturated linear (i.e. unbranched) or branched univalent hydrocarbon chain or combination thereof, having the number of carbon atoms designated (*i.e.,* C₁₋₆ means one to ten carbon atoms). Particular C₁₋₆alkyl groups are those having 1 to 6 carbon atoms, having 2 to 6 carbon atoms (a "C₂-₆alkyl"), or having 1 to 4 carbon atoms (a "C₁₋₄alkyl"). Examples of C₁₋₆alkyl group include, but are not limited to, groups such as methyl, ethyl, n-propyl, isopropyl, n-butyl, t-butyl, isobutyl, sec-butyl, homologs and isomers of, for example, n-pentyl, n-hexyl, and the like.

"Cyano", alone or in combination with other groups, refers to CN (i.e. nitrile).

"C₃₋₆ Cycloalkyl" refers to a saturated or partially unsaturated carbocyclic moiety having mono-, bi- (including bridged bicyclic and cycloalkyl spiro moieties). Examples of cycloalkyl moieties include, but are not limited to, cyclopropyl, cyclobutyl, cyclopentyl, cyclohexyl, cycloheptyl, and partially unsaturated (cycloalkenyl) derivatives thereof (e.g. cyclopentenyl, cyclohexenyl, and cycloheptenyl), bicyclo[3.1.0]hexanyl, bicyclo[3.1.0]hexenyl, bicyclo[3.1.1]heptanyl, bicyclo[3.1.1]heptenyl and bicyclo[1.1.1]pentane. The cycloalkyl moiety can be attached in a "spiro-cycloalkyl" or "cycloalkyl spiro" fashion such as "spirocyclopropyl".

"Halo" or "Halogen" means fluoro, chloro, bromo or iodo, particularly chloro or fluoro.

"Halo-C₁₋₆alkyl" refers to an C₁₋₆alkyl, as defined above, substituted with one or more halogen atoms, particularly with one to three halogen atoms. More particularly halo-C₁₋₆alkyl is the chloro- and fluoro-C₁₋₆alkyl. In some particular embodiment halo-C₁₋₆alkyl refers to perhaloC₁-₃alkyl as defined herein. More particularly halo-C₁₋₆alkyl is trifluoromethyl, difluoromethyl or fluoromethyl. Most particularly halo-C₁₋₆alkyl is trifluoroalkyl (-CF₃).

"Heteroaryl" refers to an aromatic heterocyclic mono-, bi- or tricyclic ring system of 5 to 14 ring atoms, preferably from 5 to 10 ring atoms, more preferably from 5 to 6 ring atoms, comprising 1, 2, 3 or 4 heteroatoms selected from N, O and S, the remaining ring atoms being carbon. In some aspects, monocyclic heteroaryl rings may be 5-6 membered. Bicyclic heteroaryl ring systems include fused bicyclics having two fused five-membered heteroaryl rings (denoted as 5-5), having a five-membered heteroaryl ring and a fused six-membered heteroaryl ring (denoted as 5-6 and 6-5), and having two fused six-membered heteroaryl rings (denoted as 6-6). Examples of bicyclic heteroaryl are N containing bicyclic heteroaryl, such as pyridinyl. The heteroaryl group can be optionally substituted as defined herein. Examples of heteroaryl moieties include indazolyl, indolyl, isoindolinyl, triazolopyridinyl, imidazopyridinyl, imidazopyrazinyl, indolinyl, pyridyl, triazolopyridazinyl, isoquinolinyl, pyridazinyl, triazolopyrazinyl, pyrrolotriazinyl, spirocyclopropaneindolinyl, pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, isoxazolyl, benzofuranyl, isothiazolyl, benzothienyl, benzothiophenyl, indolyl, aza-indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, pyrrolopyridinyl, furopyridinyl, thienopyridinyl, pyrrolopyridazinyl, pyrrolopyrimidinyl, pyrrolopyrazinyl, thienopyridazinyl, thienopyrimidinyl, thienopyrazinyl, furopyridazinyl, furopyrimidinyl, and furopyrazinyl. In particular examples of heteroaryl moieties include indolyl, indazolyl, imidazopyridinyl, phenyl, or pyridyl. More particular imidazopyridinyl, indolyl, imidazopyridinyl, or indazolyl.

"Heterocycle" or "heterocyclyl" refer to a 3, 4, 5, 6, 7, 8, 9, 10-membered monocyclic, 7, 8, 9 and 10-membered bicyclic (including bridged bicyclic and cycloalkyl spiro moieties) or 10, 11, 12, 13, 14 and 15-membered bicyclic heterocyclic moiety that is saturated or partially unsaturated, and has one or more (e.g., 1, 2, 3 or 4) heteroatoms selected from oxygen, nitrogen and sulfur in the ring with the remaining ring atoms being carbon. In some aspects, the heterocycle is a heterocycloalkyl. In particular aspects heterocycle or heterocyclyl refers to a 4, 5, 6 or 7-membered heterocycle. When used in reference to a ring atom of a heterocycle, a nitrogen or sulfur may also be in an oxidized form, and a nitrogen may be substituted with one or more (C₁-C₆)C₁₋₆alkyl or groups. The heterocycle can be attached to its pendant group at any heteroatom or carbon atom that results in a stable structure. Any of the heterocycle ring atoms can be optionally substituted with one or more substituents described herein. Examples of such saturated or partially unsaturated heterocycles include, without limitation, tetrahydrofuranyl, tetrahydrothienyl, pyrrolidinyl, pyrrolidonyl, piperidinyl, pyrrolinyl, isoindolinyl, quinolinyl, isoquinolinyl, tetrahydroquinolinyl, indolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, pyrrolidine 1-oxide, N-hydroxypiperidine, piperidinyl, oxa-azaspirononanyl, diazaspirodecanyl, oxa-diazaspirodecanyl, diazaspiroundecanyl, azaspirononanyl, oxetanyl, 1-methylpyrrolidine N-oxide, diazirinyl and quinuclidinyl. The term heterocycle also includes groups in which a heterocycle is fused to one or more aryl, heteroaryl, or cycloalkyl rings, such as indolinyl, 3H-indolyl, chromanyl, azabicyclo[2.2.1]heptanyl, azabicyclo[3.1.0]hexanyl, azabicyclo[3.1.1]heptanyl, octahydroindolyl, or tetrahydroquinolinyl. In particular heterocycle refers to piperidinyl, oxa-azaspirononanyl, diazaspirodecanyl, oxa-diazaspirodecanyl, diazaspiroundecanyl, azaspirononanyl, or oxetanyl.

The terms "inflammatory bowel disease" or "IBD" means several diseases associated with inflammation of the small intestine, large intestine (colon), rectum or anus (anal sphincter), and may particularly include ulcerative colitis and Crohn's disease, including proctitis in both cases. As used in the present context, the term "IBD" also includes gastrointestinal (GI) tract cancer, which is a likely result of Gl tract inflammation. As used in this specification, the terms "gastrointestinal tract" or "Gl tract" mean the small intestine, large intestine (colon), rectum or anus (anal sphincter).

"Moiety" and "Substituent" refer to an atom or group of chemically bonded atoms that is attached to another atom or molecule by one or more chemical bonds thereby forming part of a molecule.

When indicating the number of substituents, the term "one or more" refers to the range from one substituent to the highest possible number of substitution, i.e. replacement of one hydrogen up to replacement of all hydrogens by substituents, in particular wherein "one or more" refers to one, two or three, most particularly "one or more" refers to one or two.

"Obstructive pulmonary disease" refers to any disease that causes the airways of the lungs to become narrow or blocked so that a patient cannot exhale completely. Because of damage to the lungs or narrowing of the airways inside the lungs, exhaled air comes out more slowly than normal. At the end of a full exhalation, an abnormally high amount of air may still remain in the lungs. Examples of obstructive pulmonary diseases are asthma, bronchiectasis, bronchitis and chronic obstructive pulmonary disease (COPD).

"Optional" or "optionally" means that the subsequently described event or circumstance may but need not occur, and that the description includes instances where the event or circumstance occurs and instances in which it does not. For example, "aryl group optionally substituted with a C₁₋₆alkyl group" means that the C₁₋₆alkyl may but need not be present, and the description includes situations where the aryl group is substituted with a C₁₋₆alkyl group and situations where the aryl group is not substituted with the C₁₋₆alkyl group.

"Optionally substituted" means unsubstituted or substituted. Generally these substituents can be the same or different.

"Oxidant" refers to one or more suitable electron acceptors or electron sharers and may be an element, combination of elements, a compound, or combination of compounds including reducible compounds, and is a vapor, solid or liquid at the process conditions. An example of oxidant is mCPBA (meta-Chloroperoxybenzoic acid).

"Oxo", alone or in combination with other groups, refers to =O.

"Pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein.

More particularly pharmaceutically acceptable salts of compounds of formula (I) are the salts of hydrochloric acid, hydrobromic acid, sulfuric acid, phosphoric acid and methanesulfonic acid.

"Protecting group" refers to the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are Amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

"Substituted" refers to the replacement of at least one of hydrogen atoms of a compound or moiety with another substituent or moiety. For example, the term "haloalkyl" refers to the fact that one or more hydrogen atoms of a C₁₋₆alkyl (as defined below) is replaced by one or more Halogen atoms (e.g., trifluoromethyl, difluoromethyl, fluoromethyl, chloromethyl, etc.). In one aspect, substituted as used herein can refer to replacement of at least one hydrogen atom of a compound or moiety described herein with Halogen or C₁₋₆alkyl.

"Therapeutically effective amount" refers to an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

"Therapeutically inert carrier" refers to any ingredient having no therapeutic activity and being non-toxic such as disintegrators, binders, fillers, solvents, buffers, tonicity agents, stabilizers, antioxidants, surfactants or lubricants used in formulating pharmaceutical products.

### DETAILED DESCRIPTION

In one embodiment, the present invention relates a compound of formula (I), 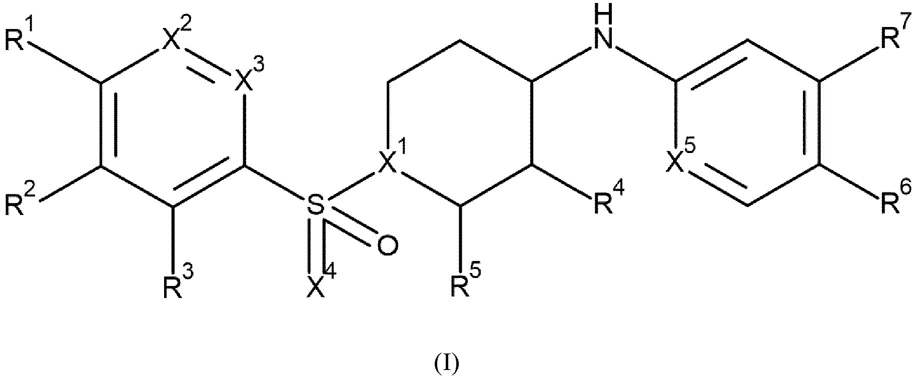 wherein
X¹ is CH or N;
X² is CH or N;
X³ is CH or N;
X⁴ is O or NH;
X⁵ is CH or N;
R¹ is aryl, heterocyclyl, or heteroaryl, wherein aryl, heterocyclyl, and heteroaryl are optionally substituted with one or two, in particular one R^{1a};
R^{1a} is C₁₋₆alkyl, C₁₋₆haloalkyl, oxo, cyano, -CONHR^{1b}, C₃₋₆cycloalkyl, or heterocyclyl;
R^{1b} is C₁₋₆alkyl, or hydrogen;
R² is hydrogen or halogen;
R³ is hydrogen or halogen;
R⁴ is hydrogen, halogen or C₁₋₆alkyl;
R⁵ is hydrogen, halogen or C₁₋₆alkyl;
R⁶ is C₃₋₆cycloalkyl or heterocyclyl, wherein C₃₋₆cycloalkyl and heterocyclyl are optionally substituted with one or two, in particular one R^{6a};
R^{6a} is C₁₋₆haloalkyl, cyano, or halogen
R⁷ is hydrogen;
or a pharmaceutically acceptable salt thereof.

In another embodiment, the present invention relates a compound of formula (I), 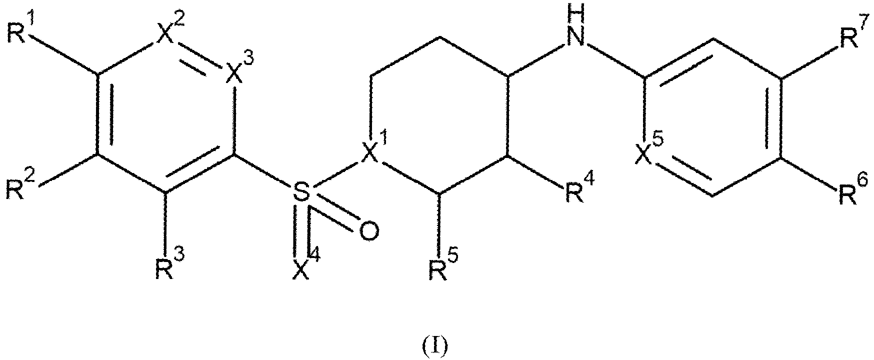 wherein
X¹ is CH or N;
X² is CH or N;
X³ is CH or N;
X⁴ is O or NH;
X⁵ is CH or N;
R¹ is aryl or heteroaryl, wherein aryl and heteroaryl are optionally substituted with one or two, in particular one R^{1a};
R^{1a} is C₁₋₆alkyl, oxo, cyano, -CONHR^{1b}, C₃₋₆cycloalkyl, or heterocyclyl;
R^{1b} is C₁₋₆alkyl, or hydrogen;
R² is hydrogen or halogen;
R³ is hydrogen or halogen;
R⁴ is hydrogen, halogen or C₁₋₆alkyl;
R⁵ is hydrogen, halogen or C₁₋₆alkyl;
R⁶ is C₃₋₆cycloalkyl or heterocyclyl, wherein C₃₋₆cycloalkyl and heterocyclyl are optionally substituted with one or two, in particular one R^{6a};
R^{6a} is C₁₋₆haloalkyl, cyano, or halogen;
R⁷ is hydrogen;
and pharmaceutically acceptable salts thereof.

A particular embodiment of the present invention relates to a compound of formula (I) or (I') as described herein, wherein
X² is CH;
X³ is CH;
X⁵ is CH;
R² is hydrogen;
R³ is hydrogen;
R⁴ is hydrogen;
R⁵ is hydrogen.

A particular embodiment of the present invention relates to a compound of formula (I), (I'), (I") or (I"') as described herein, or a pharmaceutically acceptable salt thereof, wherein X⁴ is O.

A particular embodiment of the present invention relates to a compound of formula (I), (I'), (I") or (I"') as described herein, or a pharmaceutically acceptable salt thereof, wherein X⁴ is NH.

A particular embodiment of the present invention relates to a compound of formula (I), (I'), (I") or (I"') as described herein, or a pharmaceutically acceptable salt thereof, wherein X⁵ is N.

A particular embodiment of the present invention relates to a compound of formula (I), (I'), (I") or (I"') as described herein, or a pharmaceutically acceptable salt thereof, wherein X¹ is CH.

A particular embodiment of the present invention relates to a compound of formula (I) as described herein, or a pharmaceutically acceptable salt thereof, wherein X¹ is N.

A particular embodiment of the present invention relates to a compound of formula (I , (I'), (I") or (I"') as described herein, or a pharmaceutically acceptable salt thereof, wherein R¹ is phenyl, pyridyl, or N containing bicyclic heteroaryl, wherein phenyl, pyridyl, and N containing bicyclic heteroaryl are optionally substituted with one or two, in particular one R^{1a}.

A particular embodiment of the present invention relates to a compound of formula (I), (I'), (I") or (I"') as described herein, or a pharmaceutically acceptable salt thereof, wherein R¹ is indolyl, triazolopyridyl, triazolopyrazinyl, indazolyl, imidazopyridinyl, phenyl, or pyridyl, wherein indolyl, indazolyl, imidazopyridinyl, phenyl, and pyridyl are optionally substituted with one R^{1a}.

A particular embodiment of the present invention relates to a compound of formula (I), (I'), (I") or (I"') as described herein, or a pharmaceutically acceptable salt thereof, wherein R¹ is imidazopyridinyl, cyanoindolyl, isopropylimidazopyridinyl, methylindazolyl, methylimidazopyridinyl, (methylcarbamoyl)phenyl, (methylcarbamoyl)pyridyl, carbamoylphenyl, or cyanophenyl.

A particular embodiment of the present invention relates to a compound of formula (I), (I'), (I") or (I"') as described herein, or a pharmaceutically acceptable salt thereof, wherein R¹ is imidazo[1,2-a]pyridin-6-yl, (3-cyano-1H-indol-5-yl), (3-isopropylimidazo[1,2-a]pyridin-6-yl), (3-methyl-1H-indazol-5-yl), (3-methylimidazo[1,2-a]pyridin-6-yl), [3-(methylcarbamoyl)phenyl], [4-(methylcarbamoyl)phenyl], [6-(methylcarbamoyl)-3-pyridyl], (4-carbamoylphenyl), or (4-cyanophenyl).

A particular embodiment of the present invention relates to a compound of formula (I), (I'), (I") or (I"') as described herein, or a pharmaceutically acceptable salt thereof, wherein R¹ is imidazopyridyl, indolyl, triazolopyridyl, or triazolopyrazyl, wherein imidazopyridyl, indolyl, triazolopyridyl, and triazolopyrazyl are optionally substituted with one or two R^{1a}.

A particular embodiment of the present invention relates to a compound of formula (I), (I'), (I") or (I"') as described herein, or a pharmaceutically acceptable salt thereof, wherein R¹ ismethylimidazopyridyl, cyanoindolyl, imidazopiridyl, isopropylimidazopyridinyl, isopropyltriazolopyridyl, isopropyltriazolopyrazyl, methyltriazolopyridyl, difluoromethyltriazolopyridyl, methylisopropyltriazolopyridyl.

A particular embodiment of the present invention relates to a compound of formula (I), (I'), (I") or (I"') as described herein, or a pharmaceutically acceptable salt thereof, wherein R¹ is imidazopyridinyl, cyanoindolyl, isopropylimidazopyridinyl, or methylindazolyl.

A particular embodiment of the present invention relates to a compound of formula (I), (I'), (I") or (I"') as described herein, or a pharmaceutically acceptable salt thereof, wherein R ^{1a} is methyl, isopropyl, cyano, -CONH₂, -CONH(Me), or difluoromethyl.

A particular embodiment of the present invention relates to a compound of formula (I), (I'), (I") or (I"') as described herein, or a pharmaceutically acceptable salt thereof, wherein R^{1a} is methyl, isopropyl, cyano, or difluoromethyl.

A particular embodiment of the present invention relates to a compound of formula (I), (I'), (I") or (I"') as described herein, or a pharmaceutically acceptable salt thereof, wherein R^{1a} is methyl, isopropyl, cyano, -CONH₂, -CONH(Me).

A particular embodiment of the present invention relates to a compound of formula (I), (I'), (I") or (I"') as described herein, or a pharmaceutically acceptable salt thereof, wherein R^{1b} is methyl, or hydrogen.

A particular embodiment of the present invention relates to a compound of formula (I), (I'), (I") or (I"') as described herein, or a pharmaceutically acceptable salt thereof, wherein R⁶ is cyclopropyl, or oxetanyl optionally substituted with one R^{6a}.

A particular embodiment of the present invention relates to a compound of formula (I), (I'), or (I") as described herein, or a pharmaceutically acceptable salt thereof, wherein R⁶ is (trifluoromethyl)cyclopropyl, cyanocyclopropyl, cyclopropyl, or oxetanyl.

A particular embodiment of the present invention relates to a compound of formula (I), (I'), or (I") as described herein, or a pharmaceutically acceptable salt thereof, wherein R⁶ is [1-(trifluoromethyl)cyclopropyl], (1-cyanocyclopropyl), oxetan-3-yl, (2,2-difluorocyclopropyl), cyclopropyl.

A particular embodiment of the present invention relates to a compound of formula (I), (I'), or (I") as described herein, or a pharmaceutically acceptable salt thereof, wherein R⁶ is (trifluoromethyl)cyclopropyl.

A particular embodiment of the present invention relates to a compound of formula (I), (I'), (I") or (I"') as described herein, or a pharmaceutically acceptable salt thereof, wherein R^{6a} is trifluoromethyl, cyano.

A particular embodiment of the present invention relates to a compound of formula (I), (I'), (I") or (I"') as described herein, or a pharmaceutically acceptable salt thereof, wherein R^{6a} is trifluoromethyl.

A particular embodiment of the present invention relates to a compound of formula (I) as described herein, or a pharmaceutically acceptable salt thereof, wherein
X¹ is CH or N;
X² is CH;
X³ is CH;
X⁴ is O;
X⁵ is CH or N;
R¹ is imidazopyridyl, indolyl, triazolopyridyl, or triazolopyrazyl, wherein imidazopyridyl, indolyl, triazolopyridyl, and triazolopyrazyl are optionally substituted with one or two R^{1a}.
R^{1a} is methyl, isopropyl, cyano, or difluoromethyl;
R² is hydrogen;
R³ is hydrogen;
R⁴ is hydrogen;
R⁵ is hydrogen;
R⁶ is cyclopropyl, optionally substituted with one R^{6a};
R^{6a} is trifluoromethyl;
R⁷ is hydrogen.

A particular embodiment of the present invention relates to a compound of formula (I) as described herein, or a pharmaceutically acceptable salt thereof, wherein
X¹ is CH or N;
X² is CH;
X³ is CH;
X⁴ is O;
X⁵ is CH or N;
R¹ is methylimidazopyridyl, cyanoindolyl, imidazopiridyl, isopropylimidazopyridinyl, isopropyltriazolopyridyl, isopropyltriazolopyrazyl, methyltriazolopyridyl, difluoromethyltriazolopyridyl, methylisopropyltriazolopyridyl;
R² is hydrogen;
R³ is hydrogen;
R⁴ is hydrogen;
R⁵ is hydrogen;
R⁶ is (trifluoromethyl)cyclopropyl;
R⁷ is hydrogen.

A particular embodiment of the present invention relates to a compound of formula (I) as described herein, or a pharmaceutically acceptable salt thereof, wherein the compound is of formula (I') 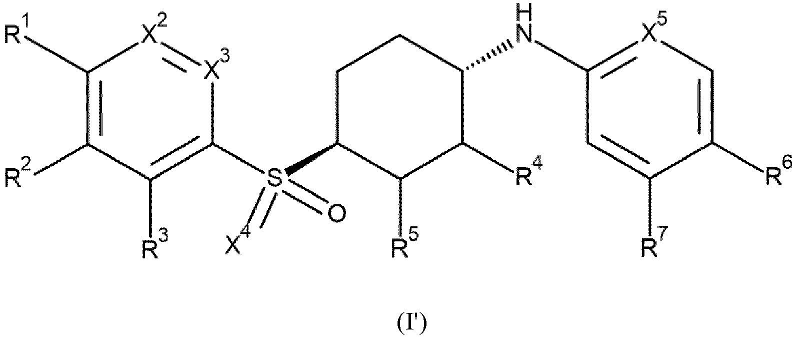 wherein R¹, R², R³ , R⁴ , R⁵, R⁶, R⁷, X², X³, X⁴, and X⁵ are as defined above.

A particular embodiment of the present invention relates to a compound of formula (I) as described herein, or a pharmaceutically acceptable salt thereof, wherein the compound is of formula (I") 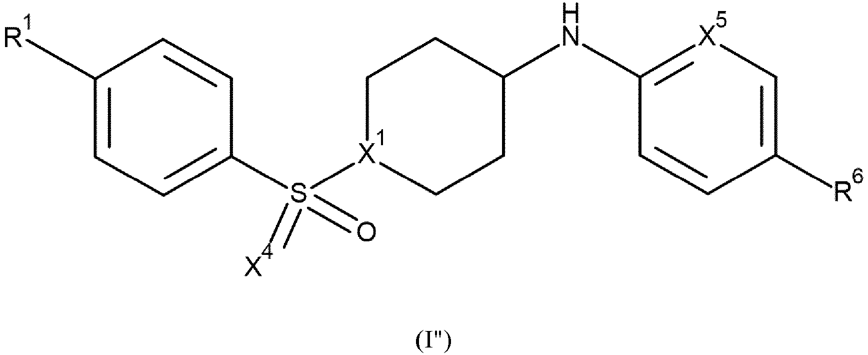 wherein R¹, R⁶, X¹, X⁴, and X⁵ are as defined above.

A particular embodiment of the present invention relates to a compound of formula (I) as described herein, or a pharmaceutically acceptable salt thereof, wherein the compound is of formula (I"') 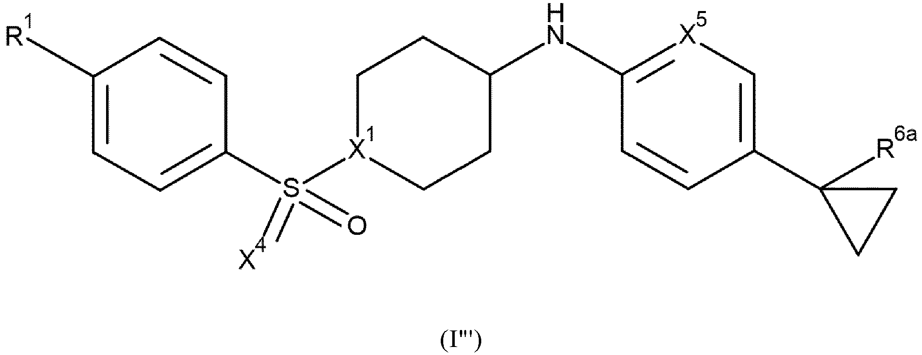 wherein R¹, R⁶, X¹, X⁴, and X⁵ are as defined above.

A particular embodiment of the present invention relates to a compound of formula (I) as described herein, or a pharmaceutically acceptable salt thereof, wherein the compound is of formula (I"') 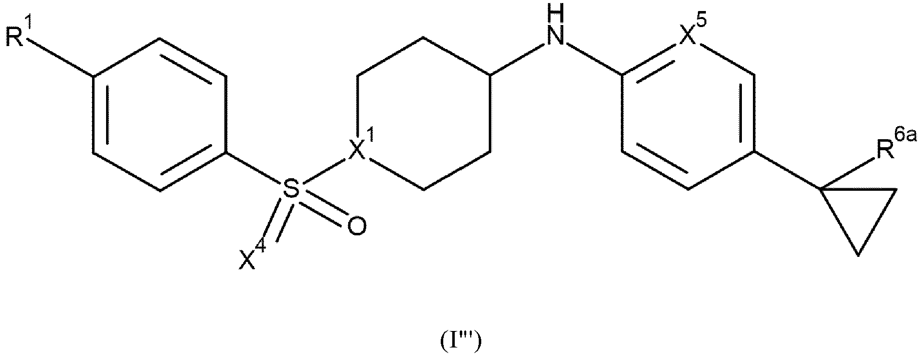 wherein
X¹ is CH or N;
X⁴ is O;
X⁵ is CH or N;
R¹ is phenyl, pyridine, or N containing bicyclic heteroaryl, wherein phenyl, pyridine, and N containing bicyclic heteroaryl are optionally substituted with one or two, in particular one R^{1a};
R^{1a} is methyl, isopropyl, cyano, -CONH₂, -CONH(Me), or difluoromethyl;
R^{6a} is trifluoromethyl or cyano.

A particular embodiment of the present invention relates to a compound of formula (I) as described herein, or a pharmaceutically acceptable salt thereof, wherein the compound is of formula (I"') 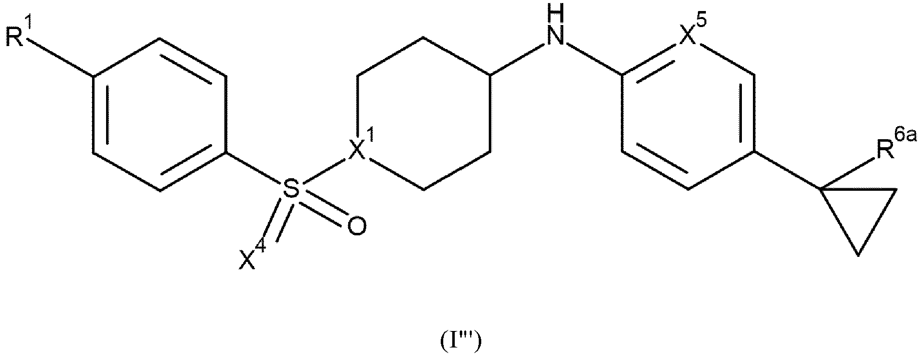 wherein
X¹ is CH or N;
X⁴ is O;
X⁵ is CH or N;
R¹ is imidazopyridyl, indolyl, triazolopyridyl, or triazolopyrazyl, wherein imidazopyridyl, indolyl, triazolopyridyl, and triazolopyrazyl are optionally substituted with one or two R^{1a};
R^{1a} is methyl, isopropyl, cyano, or difluoromethyl;
R^{6a} is trifluoromethyl.

A particular embodiment of the present invention relates to a compound of formula (I) as described herein, or a pharmaceutically acceptable salt thereof, wherein selected from:
4'-[(4-{[4-(1-cyanocyclopropyl)phenyl]Amino}piperidin-1-yl)sulfonyl]-[1,1'-biphenyl]-4-carbonitrile;
1-[4-(3-methyl-1H-indazol-5-yl)benzenesulfonyl]-N-{4-[1-(trifluoromethyl)cyclopropyl]phenyl}piperidin-4-amine;
1-(4-{3-methylimidazo[1,2-a]pyridin-6-yl}benzenesulfonyl)-N-{4-[1-(trifluoromethyl)cyclopropyl]phenyl}piperidin-4-amine;
N-methyl-4'-{[4-({4-[1-(trifluoromethyl)cyclopropyl]phenyl}Amino)piperidin-1-yl]sulfonyl}-[1,1'-biphenyl]-3-carboxamide;
N-methyl-4'-{[4-({4-[1-(trifluoromethyl)cyclopropyl]phenyl}Amino)piperidin-1-yl]sulfonyl}-[1,1'-biphenyl]-4-carboxamide;
N-methyl-5-(4-{[4-({4-[1-(trifluoromethyl)cyclopropyl]phenyl}Amino)piperidin-1-yl]sulfonyl}phenyl)pyridine-2-carboxamide;
4'-{[4-({4-[1-(trifluoromethyl)cyclopropyl]phenyl}Amino)piperidin-1-yl]sulfonyl}-[1,1'-biphenyl]-4-carboxamide;
5-(4-{[4-({4-[1-(trifluoromethyl)cyclopropyl]phenyl}Amino)piperidin-1-yl]sulfonyl}phenyl)-1H-indole-3-carbonitrile;
1-(4-{imidazo[1,2-a]pyridin-6-yl}benzenesulfonyl)-N-{4-[1-(trifluoromethyl)cyclopropyl]phenyl}piperidin-4-amine;
1-{4-[3-(propan-2-yl)imidazo[1,2-a]pyridin-6-yl]benzenesulfonyl}-N-{4-[1-(trifluoromethyl)cyclopropyl]phenyl}piperidin-4-amine;
N-(4-cyclopropylphenyl)-1-[4-(3-methyl-1H-indazol-5-yl)benzenesulfonyl]piperidin-4-amine;
5-[4-({4-[(4-cyclopropylphenyl)Amino]piperidin-1-yl}sulfonyl)phenyl]-1H-indole-3-carbonitrile;
(4-{imidazo[1,2-a]pyridin-6-yl}phenyl)[trans-4-({4-[1-(trifluoromethyl)cyclopropyl]phenyl}Amino)cyclohexyl](imino)-λ⁶-sulfanone;
4-(2,2-difluorocyclopropyl)-N-[trans-4-(4-{imidazo[1,2-a]pyridin-6-yl}benzenesulfonyl)cyclohexyl]aniline;
1-(4-{[trans-4-(4-{imidazo[1,2-a]pyridin-6-yl}benzenesulfonyl)cyclohexyl]Amino}phenyl)cyclopropane-1-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
4-(oxetan-3-yl)-N-[trans-4-(4-{imidazo[1,2-a]pyridin-6-yl}benzenesulfonyl)cyclohexyl]aniline;
5-(4-{[trans-4-{[4-(oxetan-3-yl)phenyl]Amino}cyclohexyl]sulfonyl}phenyl)-1H-indole-3-carbonitrile;
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline;
N-[(trans)-4-(4-{3-methyl-1H-pyrrolo[2,3-b]pyridin-5-yl}benzenesulfonyl)cyclohexyl]-4-[1-(trifluoromethyl)cyclopropyl]aniline;
N-[(trans)-4-(4-{3-methyl-1H-pyrazolo[3,4-b]pyridin-5-yl}benzenesulfonyl)cyclohexyl]-4-[1-(trifluoromethyl)cyclopropyl]aniline;
1-(6-{[1-(4-{2-methoxy-7-azaspiro[3.5]nonan-7-yl}benzenesulfonyl)piperidin-4-yl]amino}pyridin-3-yl)cyclopropane-1-carbonitrile;
2-(1-{4-[(4-{[5-(1-cyanocyclopropyl)pyridin-2-yl]amino}piperidin-1-yl)sulfonyl]phenyl}piperidin-4-yl)acetamide;
[(1-{4-[(4-{[5-(1-cyanocyclopropyl)pyridin-2-yl]amino}piperidin-1-yl)sulfonyl]phenyl}piperidin-4-yl)methyl]urea;
1-(6-{[1-(4-{8,10-dioxo-3,9-diazaspiro[5.5]undecan-3-yl}benzenesulfonyl)piperidin-4-yl]amino}pyridin-3-yl)cyclopropane-1-carbonitrile;
1-(6-{[1-(4-{2-oxo-3-oxa-1,8-diazaspiro[4.5]decan-8-yl}benzenesulfonyl)piperidin-4-yl]amino}pyridin-3-yl)cyclopropane-1-carbonitrile;
1-(6-{[1-(4-{1-oxo-2,8-diazaspiro[4.5]decan-8-yl}benzenesulfonyl)piperidin-4-yl]amino}pyridin-3-yl)cyclopropane-1-carbonitrile;
1-(6-{[1-(4-{2-oxa-7-azaspiro[3.5]nonan-7-yl}benzenesulfonyl)piperidin-4-yl]lamino}pyridin-3-yl)cyclopropane-1-carbonitrile;
1-(6-{[(trans)-4-{4-[3-(propan-2-yl)-[1,2,4]triazolo[4,3-a]pyridin-6-yl]benzenesulfonyl}cyclohexyl]amino}pyridin-3-yl)cyclopropane-1-carbonitrile;
1-{6-[(1-{4-[3-(propan-2-yl)-[1,2,4]triazolo[4,3-a]pyridin-6-yl]benzenesulfonyl}piperidin-4-yl)amino]pyridin-3-yl}cyclopropane-1-carbonitrile;
1-{6-[(1-{4-[8-methyl-3-(propan-2-yl)-[1,2,4]triazolo[4,3-a]pyridin-6-yl]benzenesulfonyl}piperidin-4-yl)amino]pyridin-3-yl}cyclopropane-1-carbonitrile;
1-(6-{[1-(4-{imidazo[1,2-a]pyridin-6-yl}benzenesulfonyl)piperidin-4-yl]amino}pyridin-3-yl)cyclopropane-1-carbonitrile;
1-{6-[(1-{4-[3-(propan-2-yl)imidazo[1,2-a]pyridin-6-yl]benzenesulfonyl}piperidin-4-yl)amino]pyridin-3-yl}cyclopropane-1-carbonitrile;
1-(6-{[1-(4-{3-oxo-2H,3H-[1,2,4]triazolo[4,3-a]pyridin-6-yl}benzenesulfonyl)piperidin-4-yl]amino}pyridin-3-yl)cyclopropane-1-carbonitrile;
N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine;
N-[1-(4-{imidazo[1,2-a]pyridin-6-yl}benzenesulfonyl)piperidin-4-yl]-5-[1-(trifluoromethyl)cyclopropyl]pyridin-2-amine;
N-(1-{4-[3-(propan-2-yl)imidazo[1,2-a]pyridin-6-yl]benzenesulfonyl}piperidin-4-yl)-5-[1-(trifluoromethyl)cyclopropyl]pyridin-2-amine;
N-(1-{4-[3-(propan-2-yl)-[1,2,4]triazolo[4,3-a]pyrazin-6-yl]benzenesulfonyl}piperidin-4-yl)-5-[1-(trifluoromethyl)cyclopropyl]pyridin-2-amine;
1-{4-[3-(propan-2-yl)-[1,2,4]triazolo[4,3-a]pyridin-6-yl]benzenesulfonyl}-N-{4-[1-(trifluoromethyl)cyclopropyl]phenyl}piperidin-4-amine;
1-{4-[3-(propan-2-yl)-[1,2,4]triazolo[4,3-a]pyrazin-6-yl]benzenesulfonyl}-N-{4-[1-(trifluoromethyl)cyclopropyl]phenyl}piperidin-4-amine;
1-(4-{3-methyl-[1,2,4]triazolo[4,3-a]pyridin-6-yl}benzenesulfonyl)-N-{4-[1-(trifluoromethyl)cyclopropyl]phenyl}piperidin-4-amine;
6-(4-{[4-({4-[1-(trifluoromethyl)cyclopropyl]phenyl}amino)piperidin-1-yl]sulfonyl}phenyl)-2H,3H-[1,2,4]triazolo[4,3-a]pyridin-3-one
N-(1-{4-[3-(difluoromethyl)-[1,2,4]triazolo[4,3-a]pyridin-6-yl]benzenesulfonyl}piperidin-4-yl)-5-[1-(trifluoromethyl)cyclopropyl]pyridin-2-amine
4-(4-{[4-({5-[1-(trifluoromethyl)cyclopropyl]pyridin-2-yl}amino)piperidin-1-yl]sulfonyl}phenyl)pyridine-2-carboxamide
N-(1-{4-[8-methyl-3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine
   or
N-[1-(4-{3-methyl-[1,2,4]triazolo[4,3-a]pyridin-6-yl}benzenesulfonyl)piperidin-4-yl]-5-[1-(trifluoromethyl)cyclopropyl]pyridin-2-amine.

A particular embodiment of the present invention relates to a compound of formula (I) as described herein, or a pharmaceutically acceptable salt thereof, wherein selected from:
4'-[(4-{[4-(1-cyanocyclopropyl)phenyl]Amino}piperidin-1-yl)sulfonyl]-[1,1'-biphenyl]-4-carbonitrile;
1-[4-(3-methyl-1H-indazol-5-yl)benzenesulfonyl]-N-{4-[1-(trifluoromethyl)cyclopropyl]phenyl}piperidin-4-amine;
1-(4-{3-methylimidazo[1,2-a]pyridin-6-yl}benzenesulfonyl)-N-{4-[1-(trifluoromethyl)cyclopropyl]phenyl}piperidin-4-amine;
N-methyl-4'-{[4-({4-[1-(trifluoromethyl)cyclopropylphenyl}Amino)piperidin-1-yl]sulfonyl}-[1,1'-biphenyl]-3-carboxamide;
N-methyl-4'-{[4-({4-[1-(trifluoromethyl)cyclopropyl]phenyl}Amino)piperidin-1-yl]sulfonyl}-[1,1'-biphenyl]-4-carboxamide;
N-methyl-5-(4-{[4-({4-[1-(trifluoromethyl)cyclopropyl]phenyl}Amino)piperidin-1-yl]sulfonyl}phenyl)pyridine-2-carboxamide;
4'-{[4-({4-[1-(trifluoromethyl)cyclopropyl]phenyl}Amino)piperidin-1-yl]sulfonyl}-[1,1'-biphenyl]-4-carboxamide;
5-(4-{[4-({4-[1-(trifluoromethyl)cyclopropyl]phenyl}Amino)piperidin-1-yl]sulfonyl}phenyl)-1H-indole-3-carbonitrile;
1-(4-{imidazo[1,2-a]pyridin-6-yl}benzenesulfonyl)-N-{4-[1-(trifluoromethyl)cyclopropyl]phenyl}piperidin-4-amine;
1-{4-[3-(propan-2-yl)imidazo[1,2-a]pyridin-6-yl]benzenesulfonyl}-N-{4-[1-(trifluoromethyl)cyclopropyl]phenyl}piperidin-4-amine;
N-(4-cyclopropylphenyl)-1-[4-(3-methyl-1H-indazol-5-yl)benzenesulfonyl]piperidin-4-amine;
5-[4-({4-[(4-cyclopropylphenyl)Amino]piperidin-1-yl}sulfonyl)phenyl]-1H-indole-3-carbonitrile;
(4-{imidazo[1,2-a]pyridin-6-yl}phenyl)[trans-4-({4-[1-(trifluoromethyl)cyclopropyl]phenyl}Amino)cyclohexyl](imino)-λ⁶-sulfanone;
4-(2,2-difluorocyclopropyl)-N-[trans-4-(4-{imidazo[1,2-a]pyridin-6-yl}benzenesulfonyl)cyclohexyl]aniline;
1-(4-{[trans-4-(4-{imidazo[1,2-a]pyridin-6-yl}benzenesulfonyl)cyclohexyl]Amino}phenyl)cyclopropane-1-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
4-(oxetan-3-yl)-N-[trans-4-(4-{imidazo[1,2-a]pyridin-6-yl}benzenesulfonyl)cyclohexyl]aniline;
5-(4-{[trans-4-{[4-(oxetan-3-yl)phenyl]Amino}cyclohexyl]sulfonyl}phenyl)-1H-indole-3-carbonitrile;
   or
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline.

A particular embodiment of the present invention relates to a compound of formula (I) as described herein, or a pharmaceutically acceptable salt thereof, wherein selected from:
1-[4-(3-methyl-1H-indazol-5-yl)benzenesulfonyl]-N-{4-[1-(trifluoromethyl)cyclopropyl]phenyl}piperidin-4-amine;
5-(4-{[4-({4-[1-(trifluoromethyl)cyclopropyl]phenyl}Amino)piperidin-1-yl]sulfonyl}phenyl)-1H-indole-3-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline;
N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine;
1-{4-[3-(propan-2-yl)-[1,2,4]triazolo[4,3-a]pyridin-6-yl]benzenesulfonyl}-N-{4-[1-(trifluoromethyl)cyclopropyl]phenyl}piperidin-4-amine;
1-{4-[3-(propan-2-yl)-[1,2,4]triazolo[4,3-a]pyrazin-6-yl]benzenesulfonyl}-N-{4-[1-(trifluoromethyl)cyclopropyl]phenyl}piperidin-4-amine;
1-(4-{3-methyl-[1,2,4]triazolo[4,3-a]pyridin-6-yl}benzenesulfonyl)-N-{4-[1-(trifluoromethyl)cyclopropyl]phenyl}piperidin-4-amine
N-(1-{4-[3-(difluoromethyl)-[1,2,4]triazolo[4,3-a]pyridin-6-yl]benzenesulfonyl}piperidin-4-yl)-5-[1-(trifluoromethyl)cyclopropyl]pyridin-2-amine
N-(1-{4-[8-methyl-3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine
   or
N-[1-(4-{3-methyl-[1,2,4]triazolo[4,3-a]pyridin-6-yl}benzenesulfonyl)piperidin-4-yl]-5-[1-(trifluoromethyl)cyclopropyl]pyridin-2-amine.

A particular embodiment of the present invention relates to a compound of formula (I) as described herein, or a pharmaceutically acceptable salt thereof, wherein selected from:
1-[4-(3-methyl-1H-indazol-5-yl)benzenesulfonyl]-N-{4-[1-(trifluoromethyl)cyclopropyl]phenyl}piperidin-4-amine;
5-(4-{[4-({4-[1-(trifluoromethyl)cyclopropyl]phenyl}Amino)piperidin-1-yl]sulfonyl}phenyl)-1H-indole-3-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
   or
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline.

Further objects of the present invention are all forms of optically pure enantiomers, racemates or diastereomeric mixtures for compounds of formula (I) or (I').

### Process of manufacturing

Processes for the manufacture of compounds of formula (I), or pharmaceutically acceptable salt thereof, are described herein.

Here is provided a process of preparation of a compound as described herein, or a pharmaceutically acceptable salt thereof, wherein X¹ is N, and X⁴ is O, comprising:
reacting compound of formula (II), wherein R⁵ and R⁴ are as defined above, 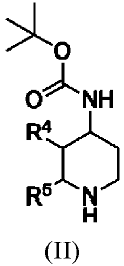
with compound of formula (III), wherein X², X³, R², and R³, are as defined above, 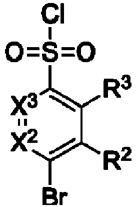 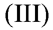
to form compound (IV), wherein R², R³, R⁴, R⁵, X², and X³ are as defined above, 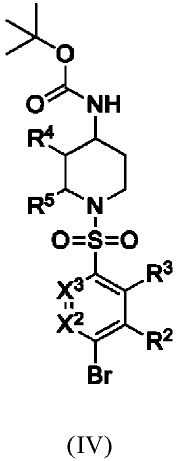
reacting said compound (IV) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3 - to 14-membered heterocyclyl optionally substituted with one or more C₁₋₆alkyl, in particular optionally substituted with four C₁₋₆alkyl, 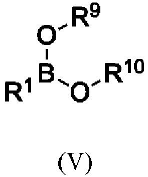
to form compound of formula (VI), wherein R¹, R², R³, R⁴, R⁵, X², and X³ are as defined above, 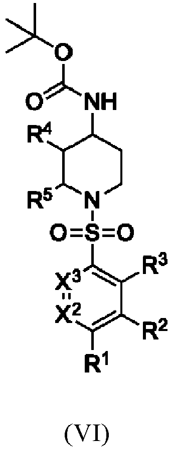
reacting said compound of formula (VI) with an acid, to form compound of formula (VII), wherein R¹, R², R³, R⁴, R⁵, X², and X³ are as defined above, 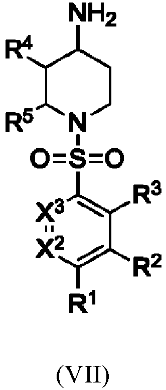
reacting said compound of formula (VII) with compound of formula (VIII), wherein R⁸ is a halogen and R⁶, R⁷, and X⁵ are as defined above, 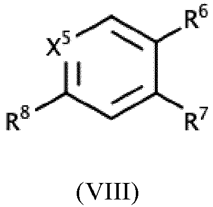
to form compound of formula (I).

Here is provided a process of preparation of a compound as described herein, or a pharmaceutically acceptable salt thereof, wherein X¹ is N and X⁴ is O, comprising:
reacting compound of formula (IX), wherein R⁴ and R⁵ are as defined above, 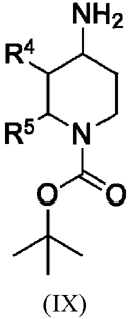
with compound of formula (VIII), wherein R⁸ is a halogen and, R⁶, R⁷, and X⁵ are as defined above, 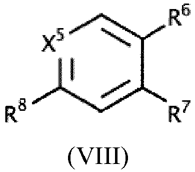
to form compound of formula (X), wherein R⁴, R⁶, R⁷, X⁵, and R⁵ are as defined above, 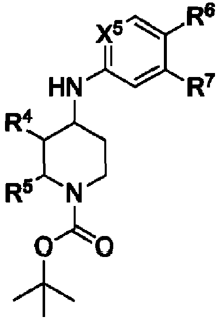 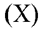
reacting said compound of formula (X) with acid to form compound of formula (XI), wherein R⁴, R⁶, R⁷, X⁵, and R⁵ are as defined above, 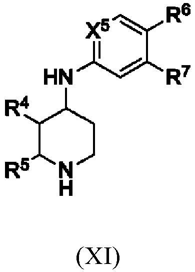
reacting said compound of formula (XI) with compound of formula (III), wherein X², X³, R², and R³, are as defined above, 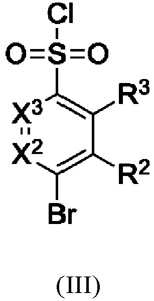
to form compound of formula (XII), wherein X², X³, R², R³, R⁴, R⁶, R⁷, X⁵, and R⁵ are as defined above, 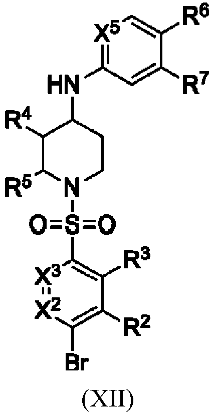
reacting said compound of formula (XII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C ₁₋₆alkyl, in particular optionally substituted with four C₁₋₆alkyl, 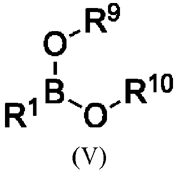
to form compound of formula (I).

Here is provided a process of preparation of a compound as described herein, or a pharmaceutically acceptable salt thereof, wherein X¹ is N and X⁴ is O, comprising:
reacting compound of formula (XVIII), wherein R⁵ and R⁴ are as defined above, 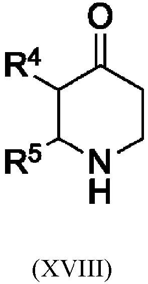
with compound of formula (XVII), wherein R¹, R², R³, X², and X³ are as defined above, 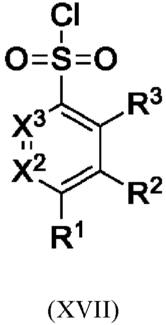
to form compound of formula (XX), wherein R¹, R², R³, R⁴, R⁵, X², and X³ are as defined above, 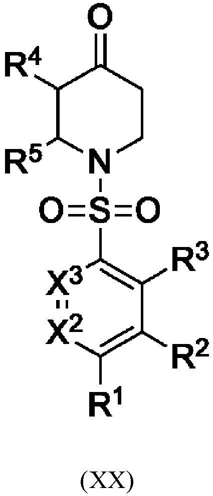
reacting said compound of formula (XX) with compound of formula (XVI), wherein X⁵, R⁶, and R⁷ are as defined above, 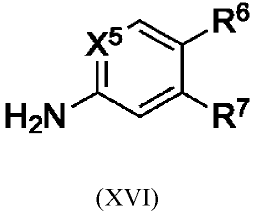
to form compound of formula (I);
   or
reacting compound of formula (XVIII), wherein R⁵ and R⁴ are as defined above, 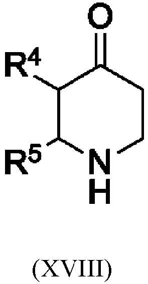
with compound of formula (III), wherein X², X³, R², and R³, are as defined above, 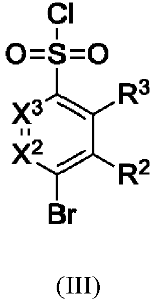
to form compound of formula (XIX), wherein R², R³, R⁴, R⁵, X², and X³ are as defined above, 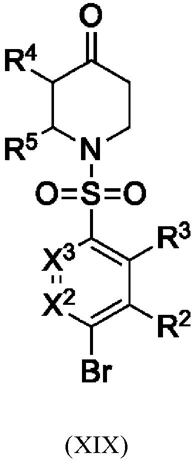
reacting said compound of formula (XIX) with said compound of formula (XVI), to form compound of formula (XII), wherein X², X³, R², R³, R⁴, R⁶, R⁷, X⁵, and R⁵ are as defined above, 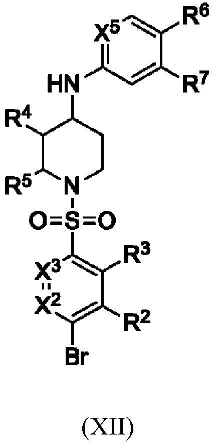
reacting said compound of formula (XII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C ₁₋₆alkyl, in particular optionally substituted with four C₁₋₆alkyl, 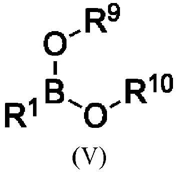
to form compound of formula (I).

Here is provided a process of preparation of a compound as described herein, or a pharmaceutically acceptable salt thereof, wherein X¹ is C and X⁴ is O, comprising:
reacting compound of formula (XXI), wherein R⁴ and R⁵ are as defined above, 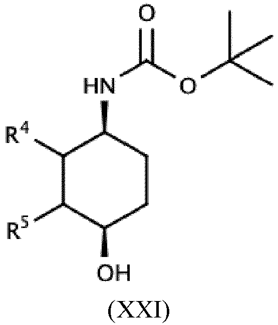
with mesyl chloride, to form compound of formula (XXII), wherein R⁴ and R⁵ are as defined above, 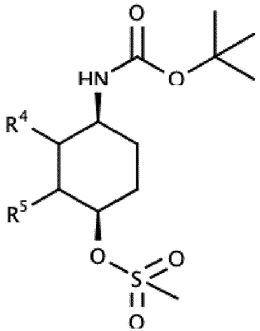 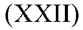
reacting said compound of formula (XXII) with compound of formula (XXIII), wherein R², R³, X², and X³ are as defined above, 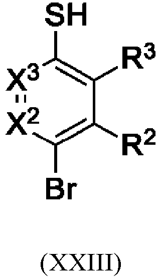
to form compound of formula (XXIV), wherein R², R³, R⁴, R⁵, X², and X³ are as defined above, 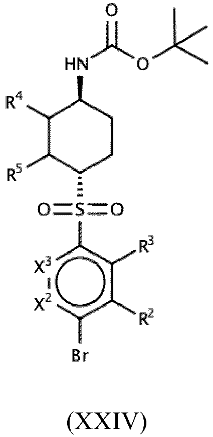
reacting said compound of formula (XXIV) with an oxidant, to form compound of formula (XXV), wherein R², R³, R⁴, R⁵, X², and X³ are as defined above, 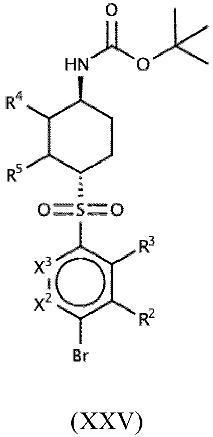
reacting said compound of formula (XXV) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C ₁₋₆alkyl, in particular optionally substituted with four C₁₋₆alkyl, 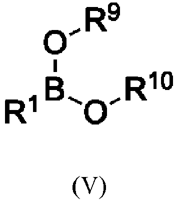
to form compound of formula (XXVI), wherein R¹, R², R³, R⁴, R⁵, X², and X³ are as defined above, 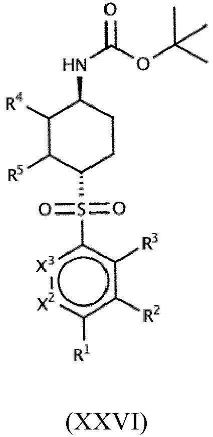
reacting said compound of formula (XXVI) with acid, to form compound of formula (XXVII), wherein R¹, R², R³, R⁴, R⁵, X², and X³ are as defined above, 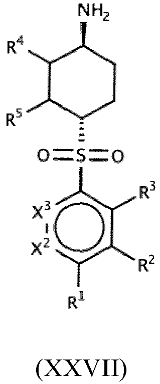
reacting said compound of formula (XXVII) with compound of formula (VIII), wherein R⁸ is a halogen and R⁶, R⁷, and X⁵ are as defined above, 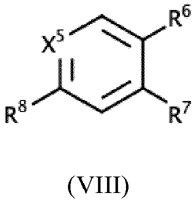
to form compound of formula (I).

Here is provided a process of preparation of a compound as described herein, or a pharmaceutically acceptable salt thereof, wherein X¹ is C and X⁴ is O, comprising:
reacting compound of formula (XXV), wherein R², R³, R⁴, R⁵, X², and X³ are as defined above, 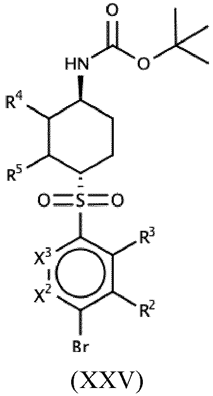
with acid to form compound of formula (XXVIII), wherein R², R³, R⁴, R⁵, X², and X³ are as defined above, 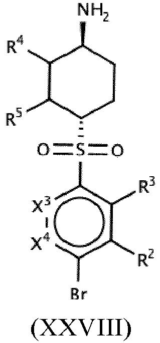
reacting said compound of formula (XXVIII) with compound of formula (VIII), wherein R⁸ is a halogen and R⁶, R⁷, and X⁵ are as defined above, 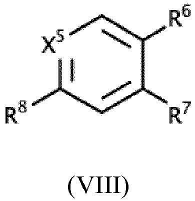
to form compound of formula (XXIX), wherein R², R³, R⁴, R⁵, R⁶, R⁷, X⁵, X², and X³ are as defined above, 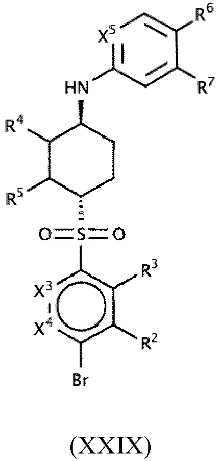
reacting said compound of formula (XXIX) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C ₁₋₆alkyl, in particular optionally substituted with four C₁₋₆alkyl, 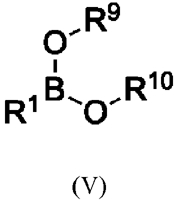
to form compound of formula (I).

Here is provided a process of preparation of a compound as described herein, or a pharmaceutically acceptable salt thereof, wherein X¹ is C and X⁴ is O, comprising:
reacting compound of formula (XXXI), wherein R⁴ and R⁵ are as defined above, 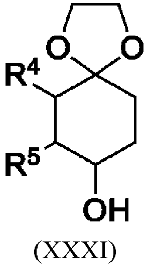
with mesyl chloride, to form compound of formula (XXXII), wherein R⁴ and R⁵ are as defined above, 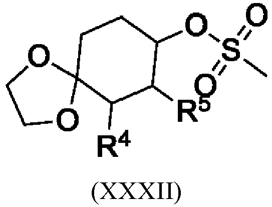
reacting said compound of formula (XXXII) with compound of formula (XXIII), wherein R², R³, X², and X³ are as defined above, 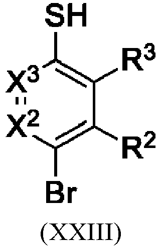
to form compound of formula (XXXIII), wherein R², R³, R⁴, R⁵, X², and X³ are as defined above, 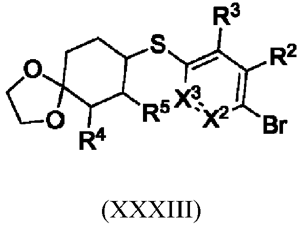
reacting said compound (XXXIII) with an oxidant, to form compound of formula (XXXIV), wherein R², R³, R⁴, R⁵, X², and X³ are as defined above, 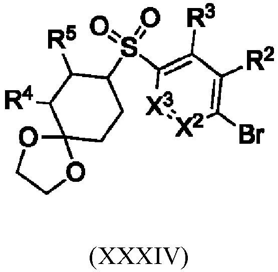
reacting said compound (XXXIV) with an acid, to form compound of formula (XXXV), wherein R², R³, R⁴, R⁵, X², and X³ are as defined above, 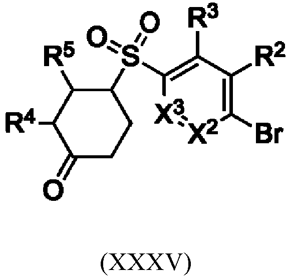
reacting said compound of formula (XXXV) with compound of formula (XVI), wherein X⁵, R⁶, and R⁷ are as defined above, 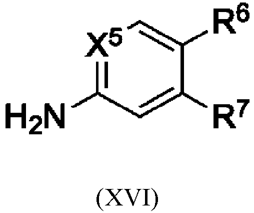
to form compound of formula (XXIX), wherein R², R³, R⁴, R⁵, R⁶, R⁷, X⁵, X², and X³ are as defined above, 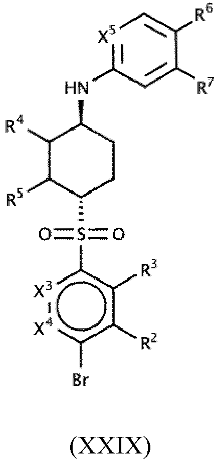
reacting said compound of formula (XXIX) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C ₁₋₆alkyl, in particular optionally substituted with four C₁₋₆alkyl, 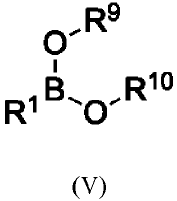
to form compound of formula (I).

Here is provided a process of preparation of a compound as described herein, or a pharmaceutically acceptable salt thereof, wherein X¹ is C and X⁴ is N, comprising:
reacting compound of formula (XXXIII), wherein R², R³, R⁴, R⁵, X², and X³ are as defined above, 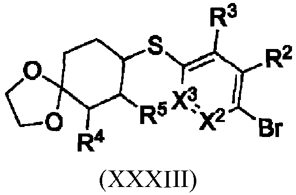
with an acid to form compound of formula (XXXVI), wherein R², R³, R⁴, R⁵, X², and X³ are as defined above, 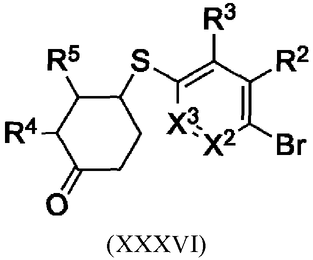
reacting said compound of formula (XXXVI) with compound of formula (XVI), wherein X⁵, R⁶, and R⁷ are as defined above, 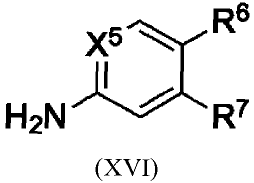
to form compound of formula (XXXVII), wherein R², R³, R⁴, R⁵, R⁶, R⁷, X⁵, X², and X³ are as defined above, 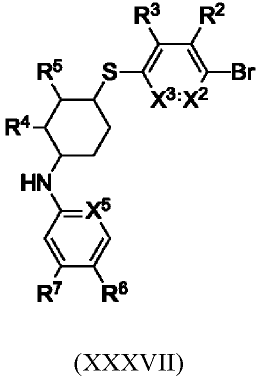
reacting said compound of formula (XXXVII) with ammonium carbamate and (diacetoxyiodo)benzene, to form compound of formula (XXXVIII), wherein R², R³, R⁴, R⁵, R⁶, R⁷, X⁵, X², and X³ are as defined above, 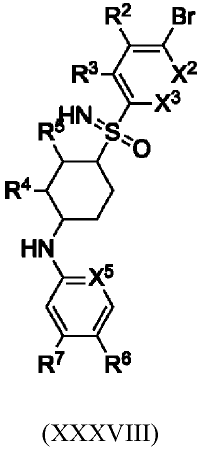
reacting said compound of formula (XXXVIII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3- to 14-membered heterocyclyl optionally substituted with one or more C₁₋₆alkyl, in particular optionally substituted with four C₁₋₆alkyl, 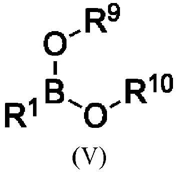
to form compound of formula (I).

Here is provided a process of preparation of a compound as described herein, or a pharmaceutically acceptable salt thereof, wherein X¹ and X⁴ are N, comprising:
reacting compound of formula (XI), wherein R⁴, R⁶, R⁷, X⁵, and R⁵ are as defined above, 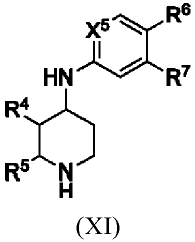
with compound of formula (XXIII), wherein R², R³, X², and X³ are as defined above, 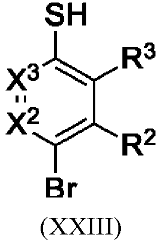
to form compound of formula (XL), wherein R², R³, X², X³, R⁴, R⁶, R⁷, X⁵, and R⁵ are as defined above, 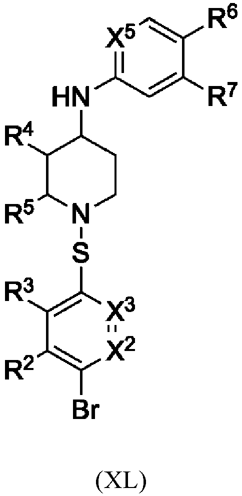
reacting said compound (XL) with ammonium carbamate and (diacetoxyiodo)benzene, to form compound of formula (XLI), wherein R², R³, X², X³, R⁴, R⁶, R⁷, X⁵, and R⁵ are as defined above, 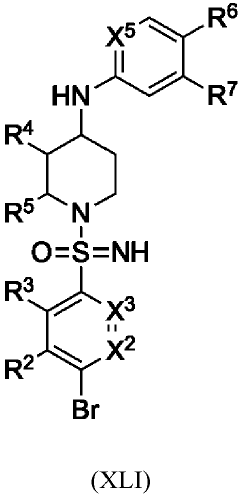
reacting said compound (XLI) with di-tert-butyl dicarbonate and a base, to form compound of formula (XLII), wherein R², R³, X², X³, R⁴, R⁶, R⁷, X⁵, and R⁵ are as defined above, 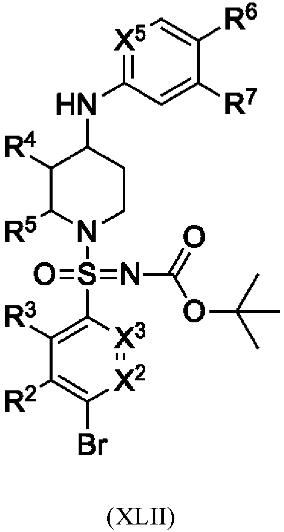
reacting said compound (XLII) with compound of formula (V), wherein R¹ is as defined above, and R⁹ and R¹⁰ are hydrogen, or R⁹ and R¹⁰ are independently selected from C₁₋₆ alkyl, or R⁹ and R¹⁰ taken together with the oxygen atom to which they are attached, form a 3 - to 14-membered heterocyclyl optionally substituted with one or more C₁₋₆alkyl, in particular optionally substituted with four C₁₋₆alkyl, 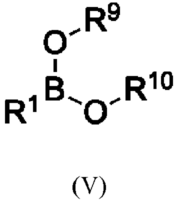
to form compound of formula (XLIII), wherein R¹, R², R³, X², X³, R⁴, R⁶, R⁷, X⁵, and R⁵ are as defined above, 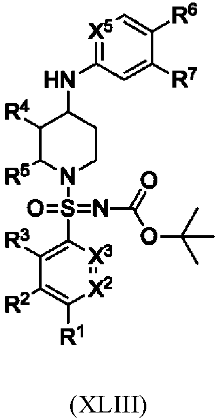
reacting said compound of formula (XLIII) with an acid to form compound of formula (I).

### EXEMPLIFICATION

As depicted in the Examples below, in certain exemplary embodiments, compounds are prepared according to the following general procedures. It will be appreciated that, although the general methods depict the synthesis of certain compounds of the invention, the following general methods, and other methods known to one skilled in the art, can be applied to all compounds and subclasses and species of each of these compounds, as described herein.

### General methods of preparation

The compounds described herein, including compounds of general Formula (I), can be readily prepared according to the following reaction schemes and Examples, or modifications thereof, using readily available starting materials, reagent, and conventional synthesis procedures. Many of the reactions can also be carried out under microwave conditions or using conventional heating or utilizing other technologies such as solid phase reagents/scavengers or flow chemistry. In these reactions, it is also possible to make use of variants which are themselves known to those skilled in the art but are not mentioned in greater detail. For Example, where specific acids, bases, reagents, coupling agents, solvents, etc. are mentioned, it is understood that other suitable acids, bases, reagents, coupling agents, solvents etc. may be used and are included within the scope of the present invention. Furthermore, other methods for preparing compounds of the invention will be readily apparent to a person of ordinary skill in the art in light of the following reaction schemes and Examples. In cases where synthetic intermediates and final products contain potentially reactive functional groups, for Example Amino, hydroxyl, thiol and carboxylic acid groups that may interfere with the desired reaction, it may be advantageous to employ protected forms of the intermediate. Methods for the selection, introduction, and subsequent removal of protecting groups are well known to those skilled in the art. The compounds obtained by using the general reaction sequences may be of insufficient purity. The compounds can be purified by using any of the methods of purification of organic compounds, for Example, crystallization or silica gel, alumina or C18 column chromatography, using different solvents in suitable ratios. All possible stereoisomers are envisioned within the scope of the invention. In the discussion below variables have the meaning indicated above unless otherwise indicated. All final compounds have been characterized using for example LC-MS, NMR and/or Specific Optical Rotation.

The abbreviations used in these experimental details are listed below and additional ones should be considered known to a person skilled in the art of synthetic chemistry.

Abbreviations used herein are as follow: **r.t.:** room temperature; **TFA:** Trifluoroacetic acid; **THF:** Tetrahydrofuran; **EtOH:** Ethanol; **TEA:** triethyl amine; **HOAc:** Acetic acid; **BINAP:** 2,2'-bis(difenylfosfino)-1,1'-binaftyl; **BOC:** tert-Butyloxycarbonyl; **N:** Normal; **DMF:** Dimethylformamide; ***t*-BuONa:** Sodium tertiar butoxide.

Chemical names are preferred IUPAC names.

If a chemical compound is referred to using both a chemical structure and a chemical name, and an ambiguity exists between the structure and the name, the structure predominates.

### General procedures

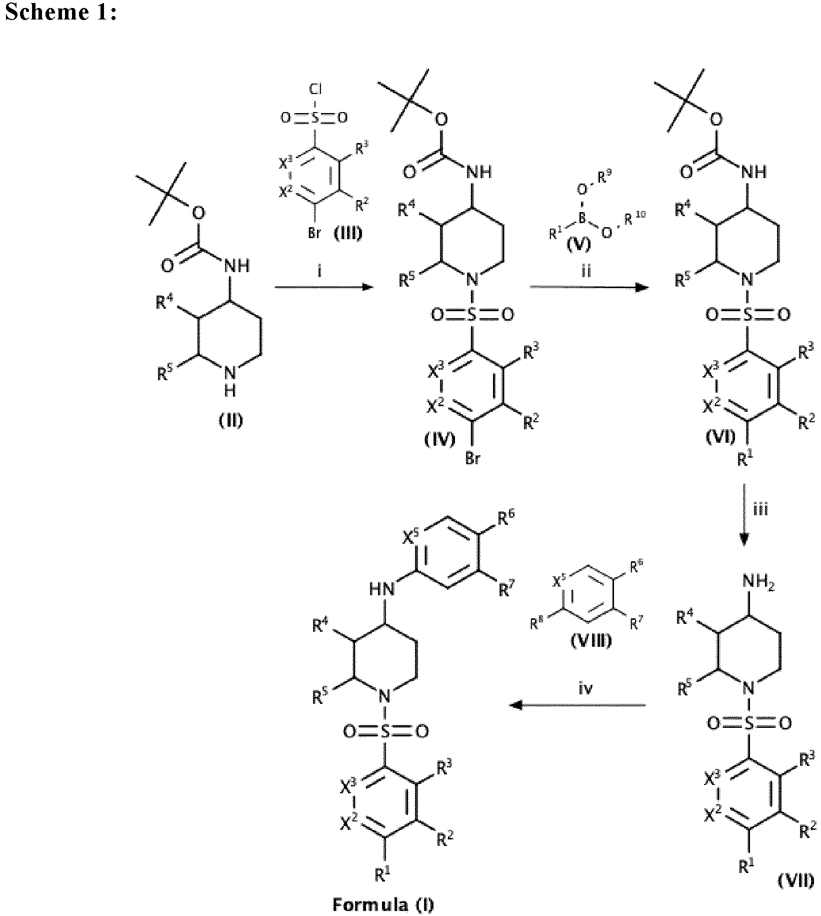

Conditions: i) TEA, CH₂Cl₂, sulfonyl chloride (III), r.t.; ii) R¹-boronic acid/ester (**V**), Pd(PPh₃)₄, NaHCO₃, 1,4-dioxane/water, 110 °C; iii) TFA, CH₂Cl₂, r.t.; iv) Pd₂(dba)₃, aryl halide (VIII), Cs₂CO₃, BINAP.

Scheme 1 describes a route to synthesize derivatives of the invention having Formula (I), when X¹ = N and X⁴ = O.

These compounds can for example be obtained by starting from readily available protected 4-aminopiperidines of formula **(II),** wherein R⁴ and R⁵ have the meaning as previously described, which can be coupled with sulfonyl chlorides of formula **(III),** wherein X², X³, R² and R³ have the meaning as previously described, to form the corresponding sulfonamides of formula **(IV).** Derivatives of formula **(VI)** can be prepared by coupling of derivatives of formula **(IV)** with commercially available boronic acids or boronic esters of formula **(V),** wherein R₁ has the meaning as previously described, under Suzuki conditions using for example Pd(PPh₃)₄ and NaHCO₃ in dioxane/water mixture. After deprotection of the amine under acidic conditions, using for example TFA or an aqueous HCl solution, the obtained aminopiperidines derivatives of formula **(VII)** can be converted, under Buchwald conditions, using for example Pd₂(dba)₃, BINAP, and Cs₂CO₃ as the base, into derivatives of **Formula (I),** wherein X¹, X², X³, X⁴, X⁵, R¹, R², R³, R⁴, R⁵, R⁶ and R⁷ have the meaning as previously described.

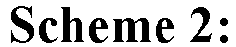

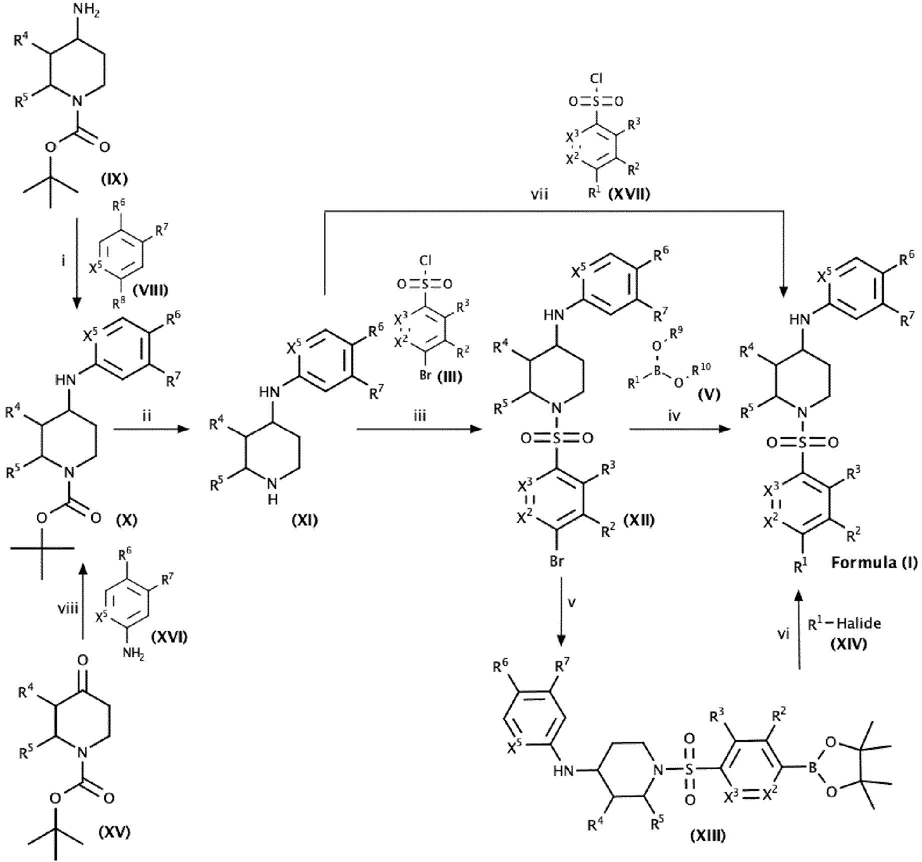

Conditions: i) Pd₂(dba)₃, aryl halide **(VIII),** Cs₂CO₃, BINAP; ii) TFA, CH₂Cl₂, r.t.; iii) TEA, CH₂Cl₂, sulfonyl chloride **(III),** r.t.; iv) R¹-boronic acid/ester **(V),** Pd(PPh₃)₄, NaHCO₃, 1,4-dioxane/water, 110 °C; v) Bis(pinacolato)diboron, KOAc, Pd(dppf)Cl₂, DMF, 75 °C; vi) R¹-halide **(XIV),** Pd(PPh₃)₄, 2N K₂CO₃, toluene/ethanol, 90 °C; vii) TEA, CH₂Cl₂, sulfonyl chloride **(XVII),** r.t.; viii) Aniline **(XVI),** 2-methylpyridine borane complex, CH₃OH, HOAc, r.t..

Scheme 2 describes an alternative route to synthesize derivatives of the invention having **Formula (I),** when X¹ = N and X⁴ = O.

Compounds of the invention can for example be obtained by coupling under Buchwald conditions, of commercially available protected 4-aminopiperidine derivatives of formula (IX), wherein X², X³, R², R³, R⁴ and R⁵ have the meaning as previously described, and aryl halide derivatives of formula (VIII), wherein R⁸ is a halogen and X⁵, R⁶ and R⁷ have the meaning as previously described, to give the corresponding protected N-phenylpiperidin-4-amine derivatives of formula (X), which after deprotection under acidic conditions, using for example TFA, give 4-aminopiperidine derivatives of formula (XI). In an alternative way, protected N-phenylpiperidin-4-amine derivatives of formula (X) can be prepared under reductive amination conditions, starting from the N-protected piperidin-4-one derivatives of formula (XV), wherein R⁴ and R⁵ have the meaning as previously described, and an aniline derivative of formula **(XVI),** wherein X⁵, R⁶ and R⁷ have the meaning as previously described. Derivatives of **Formula (I)** can be prepared, by direct coupling of derivatives of 4-aminopiperidine derivatives **(XI)** with commercially available sulfonyl chlorides **(XVII),** wherein R¹, R², R³, X² and X³ have the meaning as previously described. Alternatively, derivatives of 4-aminopiperidine derivatives **(XI)** can be coupled with 4-bromosulfonyl derivatives of formula **(III)** to give 1-(4-bromobenzenesulfonyl)-N-phenylpiperidin-4-amine derivatives of formula **(XII)** from which derivatives of **Formula** (I) can be prepared, either by direct coupling of derivatives of formula **(XII)** with commercially available boronic acids or boronic esters of formula **(V),** wherein R¹ has the meaning as previously described, under Suzuki conditions using for example Pd(PPh₃)₄ and NaHCO₃ in dioxane/water mixture or, by first converting the piperidin derivatives of formula **(XII)** into the corresponding boronic ester derivatives of formula **(XIII),** which then can be reacted under Suzuki conditions, with R₁-halides of formula **(XIV),** wherein R₁ has the meaning as previously described.

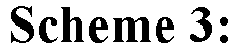

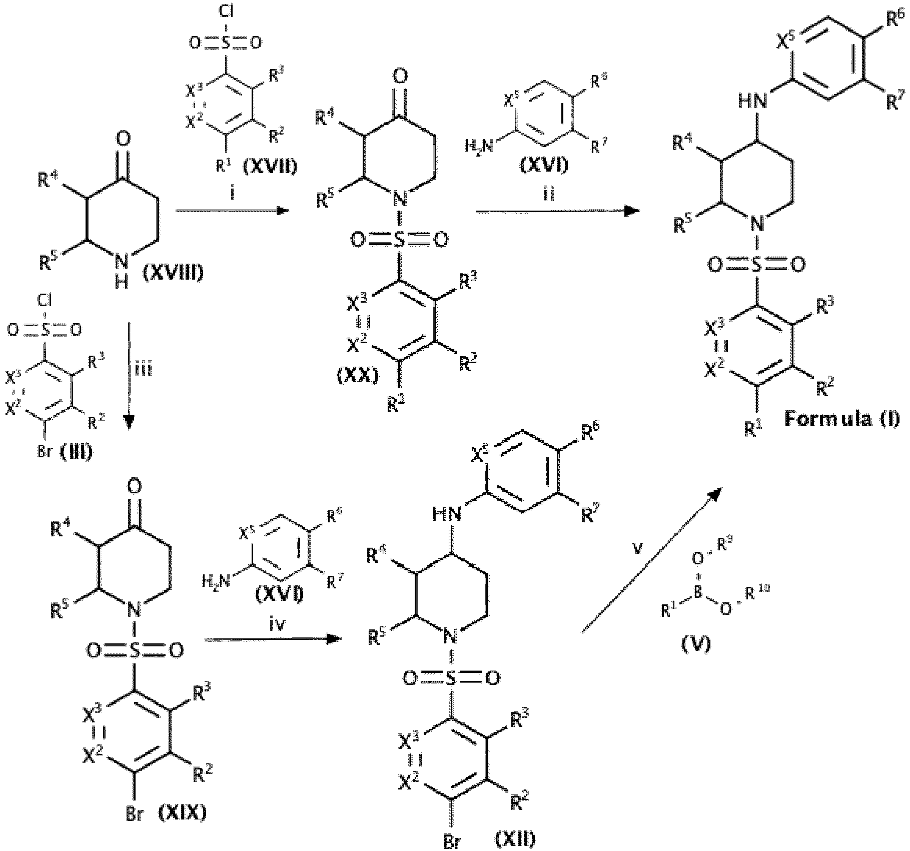

Conditions: i) TEA, CH₂Cl₂, benzenesulfonylchloride **(XVII)** r.t.; ii) Aniline **(XVI),** 2-methylpyridine borane complex, CH₃OH, HOAc, r.t.; iii) 4-Bromosulfonylchloride **(III),** TEA, r.t.; iv) Aniline **(XVI),** 2-methylpyridine borane complex, CH₃OH, HOAc, r.t.; v) R¹-boronic acid/ester **(V),** Pd(PPh₃)₄, NaHCO₃, 1,4-dioxane/water, 110 °C.

Scheme 3 describes another route to synthesize derivatives of the invention having **Formula (I),** when X¹ = N and X⁴ = O.

Starting from commercially available piperidine-4-one derivatives of formula **(XVIII),** wherein R⁴ and R⁵ have the meaning as previously described, 1-(benzenesulfonyl)piperidin-4-one derivatives of formula **(XX)** can be prepared by coupling derivatives of formula **(XVIII)** with benzenesulfonylchloride derivatives of formula **(XVII),** wherein R¹, R², R³, X² and X³ have the meaning as previously described. 1-(benzenesulfonyl)piperidin-4-one derivatives of formula **(XX)** can then be coupled under reductive amination conditions, with suitable aniline derivatives of formula **(XVI),** wherein X⁵, R⁶ and R⁷ have the meaning as previously described, to give the corresponding derivatives of **Formula (I).**

Alternatively, derivatives of piperidine-4-one derivatives of formula **(XVIII)** can be coupled with 4-bromosulfonyl derivatives of formula **(III),** wherein X², X³, R² and R³ have the meaning as previously described, to give 1-(4-bromobenzenesulfonyl)piperidin-4-one derivatives of formula **(XIX),** which, under reductive amination conditions, can be converted to the corresponding derivatives of formula **(XII),** using appropriate aniline derivatives of formula **(XVI),** wherein X⁵, R⁶ and R⁷ have the meaning as previously described. Under Suzuki conditions, using for example Pd(PPh₃)₄ and NaHCO₃ in dioxane/water mixture, the bromophenyl derivatives of formula **(XII),** can be coupled with commercially available boronic acids or boronic esters of formula **(V),** wherein R¹ has the meaning as previously described, to give the corresponding derivatives of **Formula (I).**

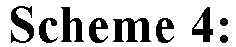

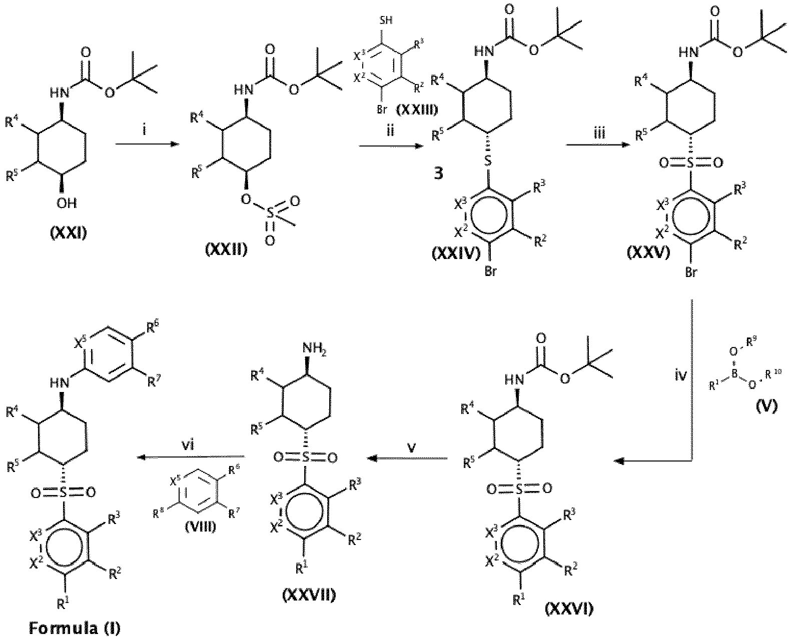

Conditions: i) Mesyl chloride, TEA, 0 °C to r.t.; ii) 4-bromobenzenethiol **(XXIII),** Cs₂CO₃, acetone, 60 °C; iii) mCPBA, ethyl acetate, r.t.; iv) R¹-boronic acid/ester **(V),** Pd(PPh₃)₄, 2N K₂CO₃, toluene/ethanol, 90 °C; v) TFA, CH₂Cl₂, r.t.; vi) Pd₂(dba)₃, aryl halide **(VIII),** Cs₂CO₃, BINAP.

Scheme 4 describes a route to synthesize derivatives of the invention having **Formula (I),** when X¹ = C and X⁴ = O.

As depicted in scheme 4, the derivatives of the invention having **Formula (I)** can be obtained by coupling of N-Boc protected cis-4-aminocyclohexan-1-ol derivatives of formula **(XXI),** wherein R⁴ and R⁵ have the meaning as previously described, which can easily be prepared by someone skilled in the art of organic chemistry, and mesylchloride under basic conditions, to give the corresponding N-Boc protected cis-4-aminocyclohexyl methanesulfonate derivatives of formula **(XXII).** These sulfonates derivatives of formula **(XXII)** can be converted into N-Boc protected 4-[(4-bromophenyl)sulfanyl]cyclohexan-1-amine derivatives of formula **(XXIV)** via a nucleophilic substitution reaction with 4-bromobenzene-1-thiol derivatives of formula **(XXIII),** using a suitable base e.g. Cs₂CO₃, which can be oxidized to the corresponding N-Boc protected sulfonyl derivatives of formula **(XXV)** using for example mCPBA_as the oxidizing agent. These sulfonyl derivatives of formula **(XXV)** can then be coupled with the appropriate boronic acids or boronic esters of formula **(V)** under Suzuki conditions, using for example Pd(PPh₃)₄ as the catalyst, to form the corresponding derivatives of formula **(XXVI).** After removing the Boc-group of derivatives of formula **(XXVI)** under acidic conditions, using for example TFA, the obtained derivatives of formula **(XXVII)** can be converted, for example, under Buchwald conditions, using the appropriate aryl halide **(VIII),** wherein R⁸ is a halogen and X⁵, R⁶ and R⁷ have the meaning as previously described, using for example Pd₂(dba)₃, BINAP and a suitable base e.g. Cs₂CO₃, into derivatives of **Formula (I),** wherein X², X³, X⁵, R¹, R², R³, R⁴, R⁵, R⁶ and R⁷ have the meaning as previously described.

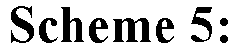

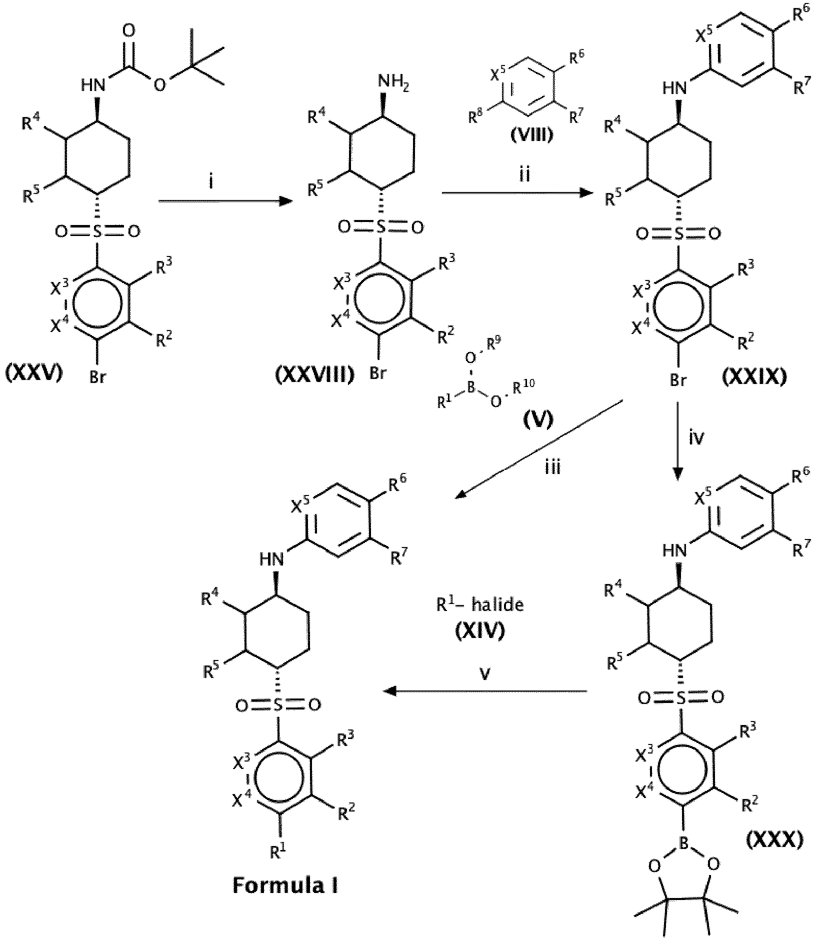

Conditions: i) TFA, CH₂Cl₂, r.t.; ii) Pd₂(dba)₃, aryl halide (VIII), Cs₂CO₃, BINAP; iii) R¹-boronic acid/ester (V), Pd(PPh₃)₄, 2N K₂CO₃, toluene/ethanol, 90 °C; iv) Bis(pinacolato)diboron, KOAc, Pd(dppf)Cl₂, DMF, 75 °C; v) R¹-halide (XIV), Pd(PPh₃)₄, 2N K₂CO₃, toluene/ethanol, 90 °C.

Scheme 5 describes an alternative route to synthesize derivatives of the invention having Formula (I), when X¹ = C and X⁴ = O.

In an alternative way, first the Boc group of derivatives of formula **(XXV)** can be removed under acidic conditions, using for example TFA to obtain the Amino derivatives of formula **(XXVIII)** which can be converted, under Buchwald conditions, as previously described, into derivatives of formula **(XXIX).** Derivatives of formula **(XXIX)** can be converted into derivatives of **Formula (I)** either by reaction with suitable boronic acids or boronic esters of formula **(V),** wherein R¹ has the meaning as previously described, under Suzuki conditions using for example Pd(PPh₃)₄ as the catalyst or by first converting derivatives of formula **(XXIX)** into their corresponding boronic esters of formula **(XXX)** which can further be reacted with suitable R¹-halides of formula **(XIV),** under Suzuki conditions, as previously described.

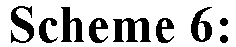

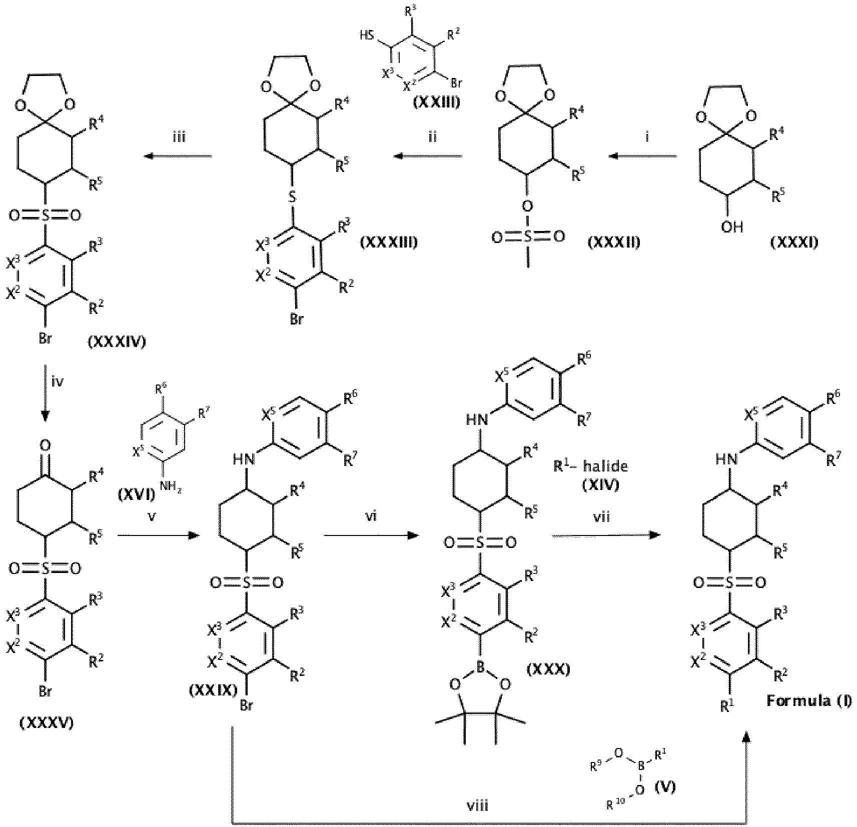

Conditions: i) CH₃SO₂Cl, Et₃N, CH₂Cl₂, r.t.; ii) 4-bromobenzenethiol **(XXIII),** Cs₂CO₃, acetone, 60 °C; iii) mCPBA, ethyl acetate, r.t.; iv) 2N HCl, THF, r.t.; v) Aniline **(XVI),** 2-methylpyridine borane complex, CH₃OH, HOAc, r.t.; vi) Bis(pinacolato)diboron, KOAc, Pd(dppf)Cl₂, DMF, 75 °C; vii) R¹-halide **(XIV),** 2N K₂CO₃, toluene/ethanol, Pd(PPh₃)₄, 90 °C; viii) R¹-boronic acid/ester **(V),** Pd(PPh₃)₄, 2N K₂CO₃, toluene/ethanol, 90 °C.

Scheme 6 describes another route to synthesize derivatives of the invention having **Formula (I),** when X¹ = C and X⁴= O.

These compounds can for example be obtained by starting from readily available keto-protected 4-hydroxycyclohexan-1-ones derivatives of formula **(XXXI),** wherein R⁴ and R⁵ have the meaning as previously described, in which the hydroxyl group is converted into a suitable leaving group, e.g. a mesyl or tosyl group, to give derivates of formula **(XXXII),** which can react with 4-bromobenzenethiol derivatives of formula **(XXIII),** to form the phenylsulfanyl derivatives of formula **(XXXIII).** After oxidation of the sulfur, using for example mCPBA, the formed sulfonyl derivatives of formula **(XXXIV)** can then be deprotected under acid conditions to get the sulfonylcyclohexanone derivatives of formula **(XXXV).** These sulfonylcyclohexanone derivatives of formula **(XXXV)** can be coupled to an aniline derivative of formula **(XVI),** wherein X⁵, R⁶ and R⁷ have the meaning as previously described, under reductive amination conditions to give the corresponding N-phenylpiperidin-4-amine derivatives of formula **(XXIX).** Derivatives of **Formula (I)** can then be prepared, either by direct coupling of derivatives of formula **(XXIX)** with commercially available boronic acids or boronic esters of formula **(V),** wherein R¹ has the meaning as previously described, under Suzuki conditions using for example Pd(PPh₃)₄ and NaHCO₃ in dioxane/water mixture or, by first converting derivatives of formula **(XXIX)** into the corresponding boronic acids or boronic esters of formula **(XXX),** which then can be reacted under Suzuki conditions, with R¹-halides of formula **(XIV),** wherein R¹ has the meaning as previously described.

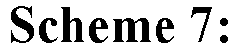

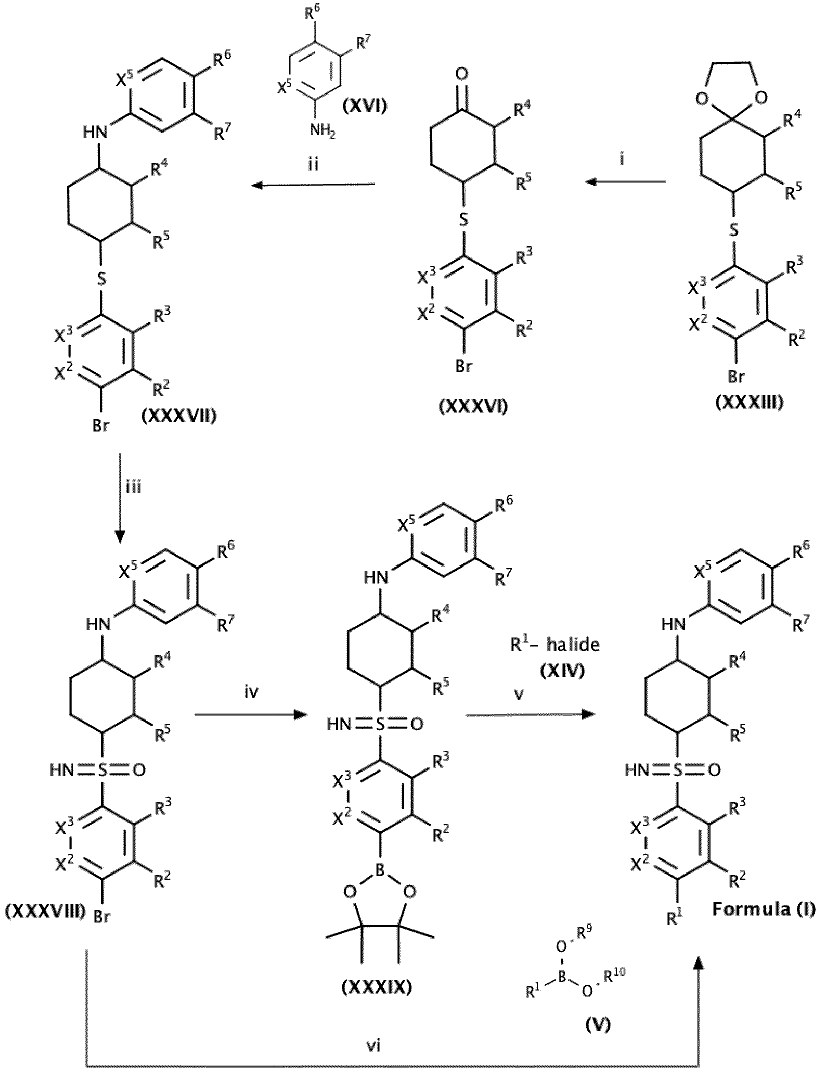

Conditions: i) 2N HCl, THF, r.t.; ii) Aniline **(XVI),** 2-methylpyridine borane complex, CH₃OH, HOAc, r.t.; iii) Ammonium carbamate, (diacetoxyiodo)benzene, CH₃OH, CH₃CN, r.t.; iv) Bis(pinacolato)diboron, KOAc, Pd(dppf)Cl₂, DMF, 75 °C; v) R¹-halide **(XIV),** 2N K₂CO₃, toluene/ethanol, Pd(PPh₃)₄, 90 °C; vi) R¹-boronic acid/ester **(V),** Pd(PPh₃)₄, 2N K₂CO₃, toluene/ethanol, 90 °C.

Scheme 7 describes a route to synthesize derivatives of the invention having **Formula (I),** when X¹ = C and X⁴ = N.

Keto-protected sulfanyl derivatives of formula **(XXXIII)** can be deprotected under acidic conditions, using for example HCl or TFA, to give 4-(phenylsulfanyl)cyclohexan-1-one derivatives of formula **(XXXVI).** Under reductive amination conditions, derivatives of formula **(XXXVI)** can be coupled with suitable aniline derivatives of formula **(XVI),** wherein X⁵, R⁶ and R⁷ have the meaning as previously described, to give the corresponding N-[4-(phenylsulfanyl)cyclohexyl]aniline derivatives of formula **(XXXVII).** Derivatives of formula **(XXXVIII)** can be prepared by converting derivatives of formula **(XXXVII)** into the corresponding sulfoximines derivatives, by using ammonium carbamate, (diacetoxyiodo)benzene and CH₃CN in CH₃OH. Finally, derivatives of formula **(XXXVIII)** can be converted into derivatives of **Formula (I)** either by reaction with suitable boronic acids or boronic esters of formula **(V),** wherein R₁ has the meaning as previously described, under Suzuki conditions using for example Pd(PPh₃)₄ as the catalyst or by first converting derivatives of formula **(XXXVIII)** into their corresponding boronic ester of formula **(XXXIX),** which can further be reacted with suitable R¹-halides of formula **(XIV)** under Suzuki conditions, as previously described.

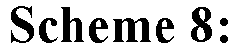

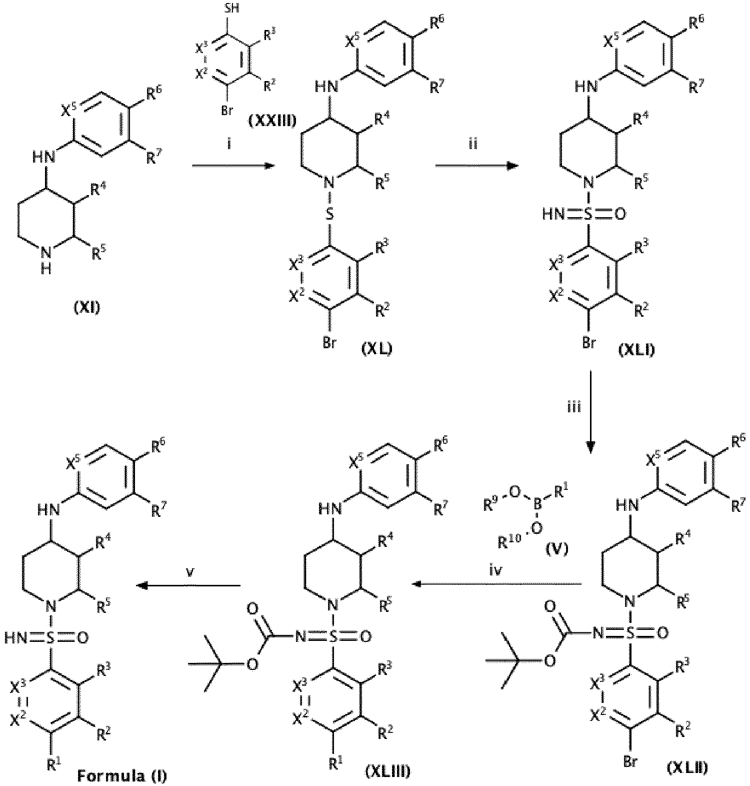

Conditions: i) 4-bromobenzenethiol **(XXIII),** Cs₂CO₃, acetone, 60 °C; ii) Ammonium carbamate, (diacetoxyiodo)benzene, CH₃OH, CH₃CN, r.t.; iii) NaH, di-tert-butyl dicarbonate, THF, r.t.; iv) R¹-boronic acid/ester **(V),** Pd(PPh₃)₄, 2N K₂CO₃, toluene/ethanol, 90 °C; v) 5N HCl, 2-propanol, r.t..

Scheme 8 describes a route to synthesize derivatives of the invention having **Formula (I),** when X₁ and X₄ are N.

N-phenylpiperidin-4-amine derivatives of formula **(XI),** wherein X⁵, R⁴, R⁵, R⁶ and R⁷ have the meaning as previously described, can be converted into the N-phenyl-1-(phenylsulfanyl)piperidin-4-amine derivatives of formula **(XL)** by reaction with 4-bromobenzene-1-thiol derivatives of formula **(XXIII),** wherein X², X³, R² and R³ have the meaning as previously described, using for example Cs₂CO₃ as the base. Derivatives of formula **(XL)** can be converted into the corresponding sulfoniminamide derivatives of formula **(XLI),** by using ammonium carbamate, (diacetoxyiodo)benzene and CH₃CN in CH₃OH. After protecting the sulfonimidamide nitrogen with for example a Boc-group, derivatives of formula **(XLII)** can react with suitable boronic acids or boronic acids of formula **(V)** under Suzuki conditions, as previously described, to give derivatives of formula **(XLIII).** In the final step, the Boc-group can be removed, under acidic conditions, to give the corresponding derivatives of **Formula (I),** wherein X², X³, X⁵, R¹, R², R³, R⁴, R⁵, R⁶ and R⁷ have the meaning as previously described.

### Pharmaceutical compositions and administration

Another object of the present invention is a pharmaceutical composition comprising a compound of formula (I) as described herein, or a pharmaceutically acceptable salt thereof, and a pharmaceutically acceptable excipient.

Another embodiment of the present invention is a pharmaceutical composition comprising a compound as described herein, or a pharmaceutically acceptable salt thereof, and a pharmaceutically acceptable excipient.

Another embodiment of the present invention is the pharmaceutical composition as described herein, further comprising an additional therapeutic agent.

### Indications

The compounds of formula (I) or (I'), as described herein, can be used in an effective amount to treat a subject, in particular a human, affected by an inflammatory autoimmune disease.

In one embodiment of the present invention is the compound as described herein, or a pharmaceutically acceptable salt thereof, for use as therapeutically active substance.

In one embodiment of the present invention is the compound as described herein, or a pharmaceutically acceptable salt thereof, for use in the treatment, prevention and/or delay of an inflammatory autoimmune disease.

In one embodiment of the present invention is the compound as described herein, or a pharmaceutically acceptable salt thereof, for use in the treatment, prevention and/or delay of progression of psoriatic diseases, asthma, Inflammatory Bowel Diseases (IBD), Crohn's disease, Obstructive Lung Diseases ulcerative colitis, rheumatoid arthritis, systemic lupus erythematosus and multiple sclerosis.

In one embodiment of the present invention is the compound as described herein, or a pharmaceutically acceptable salt thereof, for use in the treatment, prevention and/or delay of progression of psoriatic diseases, asthma, Crohn's disease, ulcerative colitis, rheumatoid arthritis, systemic lupus erythematosus and multiple sclerosis.

Herein disclosed is the compound as described herein, or a pharmaceutically acceptable salt thereof, for the preparation of a medicament for the treatment, prevention and/or delay of progression of psoriatic diseases, asthma, Crohn's disease, ulcerative colitis, rheumatoid arthritis, systemic lupus erythematosus and multiple sclerosis.

Herein disclosed is the compound as described herein, or a pharmaceutically acceptable salt thereof, for the preparation of a medicament for the treatment, prevention and/or delay of an inflammatory autoimmune disease.

Herein disclosed is a method for the treatment, prevention and/or delay of progression of an inflammatory autoimmune disease, which method comprises administering a therapeutically effective amount of a compound as described herein, or a pharmaceutically acceptable salt thereof.

Herein disclosed is a method for the treatment, prevention and/or delay of progression of psoriatic diseases, asthma, Inflammatory Bowel Diseases (IBD), Crohn's disease, Obstructive Lung Diseases ulcerative colitis, rheumatoid arthritis, systemic lupus erythematosus or multiple, which method comprises administering a therapeutically effective amount of a compound as described herein, or a pharmaceutically acceptable salt thereof.

By the term "treatment" or "treating" and grammatical variations thereof as used herein, is meant therapeutic therapy. In reference to a particular condition, treating means: (1) to ameliorate the condition or one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy using the methods and/or compositions of the invention is also contemplated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing psoriatic diseases, asthma, Inflammatory Bowel Diseases (IBD), Crohn's disease, Obstructive Lung Diseases ulcerative colitis, rheumatoid arthritis, systemic lupus erythematosus or multiple, such as when a subject has a strong family history of psoriatic diseases, asthma, Inflammatory Bowel Diseases (IBD), Crohn's disease, Obstructive Lung Diseases ulcerative colitis, rheumatoid arthritis, systemic lupus erythematosus or multiple.

### Examples

### Example 1: 4'-[(4-{[4-(1-cyanocyclopropyl)phenyl]Amino}piperidin-1-yl)sulfonyl]-[1,1'-biphenyl]-4-carbonitrile.

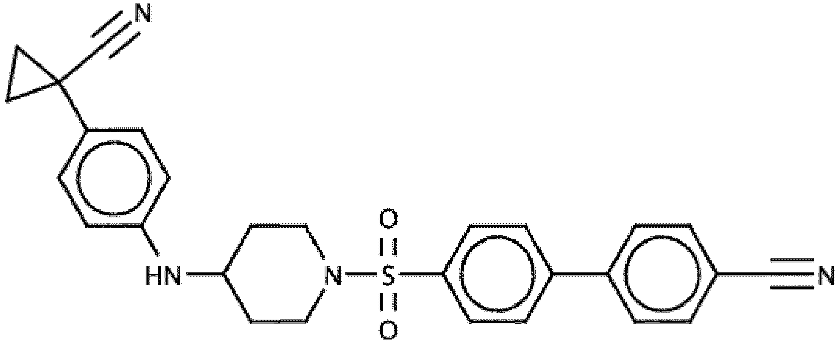

**i)** To a solution of tert-butyl N-(piperidin-4-yl)carbamate (1.0 g). and triethylamine (2.1 mL) in THF (40 mL) was added at room temperature 4-bromobenzene-1-sulfonyl chloride (1.4 g). The reaction mixture was stirred overnight at room temperature and concentrated under reduced pressure to give tert-butyl N-[1-(4-bromobenzenesulfonyl)piperidin-4-yl]carbamate (2.0 g), which was used in the next step without further purification.
**ii)** Under a nitrogen atmosphere, Pd(PPh₃)₄ (14 mg) was added to a suspension of the product obtained in the previous step (500 mg), (4-cyanophenyl)boronic acid (180 mg), and an aqueous 2M K₂CO₃ solution (6.0 mL) in a mixture of toluene (13 mL) and ethanol (1.5 mL). The reaction mixture was stirred for 1 hour at 90 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure to give tert-butyl N-[1-({4'-cyano-[1,1'-biphenyl]-4-yl}sulfonyl)piperidin-4-yl]carbamate (500 mg) as a brown oil, which was used in the next step without further purification.
**iii)** To a solution of the product obtained in the previous step (500 mg) in ethyl acetate (20 mL) was added at room temperature a 2N HCl solution in diethyl ether (5.7 mL). The reaction mixture was stirred overnight at room temperature and concentrated under reduced pressure to give 4'-[(4-aminopiperidin-1-yl)sulfonyl]-[1,1'-biphenyl]-4-carbonitrile hydrochloride (500 mg), which was used in the next step without further purification.
**iv)** Under a nitrogen atmosphere, Pd₂(dba)₃ (6.1 mg) was added to a suspension of the product obtained in the previous step (50 mg), 1-(4-bromophenyl)cyclopropane-1-carbonitrile (29 mg), Cs₂CO₃ (216 mg) and BINAP (9.1 mg) in toluene (3 mL). The reaction mixture was stirred overnight at 90 °C in a microwave. After cooling to room temperature, the reaction mixture was dried over MgSO₄, filtered, and concentrated under reduced pressure. The residue was purified on C18, using 10% to 90% CH₃CN in water as the eluent, to give the title compound 4'-[(4-{[4-(1-cyanocyclopropyl)phenyl]Amino}piperidin-1-yl)sulfonyl]-[1,1'-biphenyll-4-carbonitrile (9 mg) as a white solid. MS(ES⁺) *m*/*z* 483.1 (M+H)⁺.

¹H NMR (400 MHz, DMSO) δ 8.09 - 7.96 (m, 6H), 7.92 - 7.84 (m, 2H), 7.04 - 6.96 (m, 2H), 6.57 - 6.49 (m, 2H), 5.63 (d, *J =* 8.2 Hz, 1H), 3.64 - 3.56 (m, 2H), 3.30 - 3.22 (m, 1H), 2.62 - 2.53 (m, 2H), 1.99 - 1.91 (m, 2H), 1.59 - 1.51 (m, 2H), 1.48 - 1.32 (m, 2H), 1.30 - 1.23 (m, 2H).

### Example 2: 1-[4-(3-methyl-1H-indazol-5-yl)benzenesulfonyl]-N-{4-[1-(trifluoromethyl)cyclopropyl]phenyl}piperidin-4-amine.

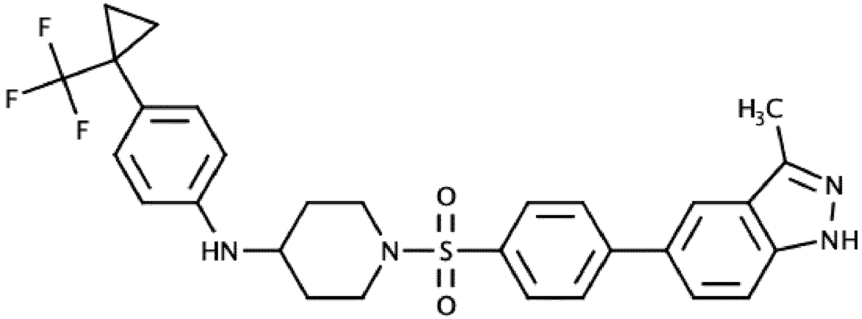

**i)** Under a nitrogen atmosphere, Pd₂(dba)₃ (460 mg) was added to a suspension of tert-butyl 4-aminopiperidine-1-carboxylate (1.0 g), *t*-BuONa (2.88 g), 1-bromo-4-[1-(trifluoromethyl)cyclopropyl]benzene (1.59 g) and Xantphos (690 mg) toluene (6 mL). The reaction mixture was stirred overnight hours at 90 °C in a microwave. After cooling to room temperature, the reaction mixture was filtered over Celite and concentrated under reduced pressure. The residue was purified on SiO₂, using 10% to 100% ethyl acetate in heptane as the eluent, to give tert-butyl 4-({4-[1-(trifluoromethyl)cyclopropyl]phenyl}Amino)piperidine-1-carboxylate (0.7 g) as a yellow solid.
**ii)** A solution of 5N HCl in 2-propanol (3.6 mL) was added to a solution of the product obtained in the previous step (0.7 g) in ethyl acetate (10 mL) and the reaction mixture was stirred overnight at room temperature. After completion the reaction mixture was concentrated under reduced pressure to give N-{4-[1-(trifluoromethyl)cyclopropyl]phenyl}piperidin-4-amine hydrochloride (0.7 g) as a white solid, which was used in the next step without further purification.
**iii)** To a suspension of the product obtained in the previous step (0.58 g) and triethyl amine (1.3 mL) in ethyl acetate (15 mL) was added portion wise at room temperature 4-bromobenzene-1-sulfonyl chloride (506 mg). The reaction mixture was stirred overnight at room temperature and quenched by addition of water. The product was extracted into ethyl acetate and the combined organic layers were dried over MgSO₄, filtered, and concentrated under reduced pressure. The residue was purified on SiO₂, using 40% ethyl acetate in heptane as the eluent, to give 1-(4-bromobenzenesulfonyl)-N-{4-[1-(trifluoromethyl)cyclopropyl]phenyl}piperidin-4-amine (1.0 g) as a white solid.
**iv)** Under a nitrogen atmosphere, Pd(PPh₃)₄ (11 mg) was added to a suspension of the product obtained in the previous step (100 mg), {3-methylimidazo[1,2-a]pyridin-6-yl}boronic acid (38 mg), and NaHCO₃ (83 mg) in a mixture of 1,4-dioxane (4 mL) and water (1 mL). The reaction mixture was stirred for 2 hours at 100 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 10% to 90% CH₃CN in water as the eluent, to give the title compound [(4-{3-methylimidazo[1,2-a]pyridin-6-yl}phenyl)[trans-4-{[4-(pentafluoro-λ⁶-sulfan_{yl})phenyllAmino }cyclohexyllimino-À,⁶-sulfan_{yll}one (45 mg) as a white solid. MS(ES⁺) *m*/*z* 555.4 (M+H)⁺.

¹H NMR (400 MHz, DMSO) δ 12.79 (s, 1H), 8.13 (s, 1H), 8.06 - 7.90 (m, 2H), 7.88 - 7.78 (m, 2H), 7.74 (dd, *J =* 8.8, 1.7 Hz, 1H), 7.60 (dd, *J =* 8.7, 0.8 Hz, 1H), 7.11 - 7.02 (m, 2H), 6.56 - 6.48 (m, 2H), 5.66 (d, *J=* 8.0 Hz, 1H), 3.63 - 3.56 (m, 2H), 3.32 - 3.19 (m, 1H), 2.59 - 2.55 (m, 5H), 2.03 - 1.92 (m, 2H), 1.50 - 1.36 (m, 2H), 1.23 - 1.12 (m, 2H), 0.94 (s, 2H).

Following a procedure analogous to that described for **Example 2,** using in step I the appropriate (hetero)aryl halide and in step iv the appropriate boronic ester or boronic acid, **Examples 3 - 9** have been prepared.

### Examples 3 - 9

### Example 3: 1-(4-{3-methylimidazo[1,2-a]pyridin-6-yl}benzenesulfonyl)-N-{4-[1-(trifluoromethyl)cyclopropyl]phenyl}piperidin-4-amine.

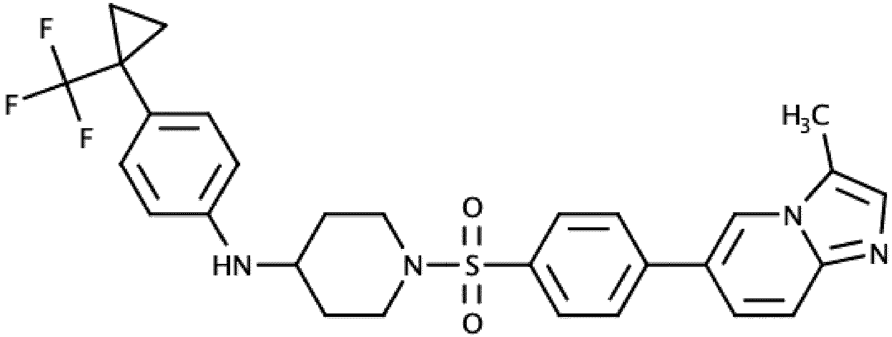

MS(ES⁺) *m*/*z* 555.4.2 (M+H)⁺.

¹H NMR (400 MHz, DMSO) δ 8.71 (s, 1H), 8.14 - 8.06 (m, 2H), 7.91 - 7.83 (m, 2H), 7.77 - 7.68 (m, 2H), 7.51 (s, 1H), 7.12 - 7.05 (m, 2H), 6.56 - 6.47 (m, 2H), 5.67 (d, *J =* 8.0 Hz, 1H), 3.64 - 3.56 (m, 2H), 3.45 - 3.21 (m, 1H), 2.62 - 2.52 (m, 5H), 2.00 - 1.93 (m, 2H), 1.49 - 1.34 (m, 2H), 1.23 - 1.16 (m, 2H), 0.94 (s, 2H).

Building block: **step i:** 1-bromo-4-[1-(trifluoromethyl)cyclopropyl]benzene; **step iv:** {3-methylimidazo[1,2-a]pyridin-6-yl}boronic acid.

### Example 4: N-methyl-4'-{[4-({4-[1-(trifluoromethyl)cyclopropylphenyl}Amino)piperidin-1-yl]sulfonyl}-[1,1'-biphenyl]-3-carboxamide.

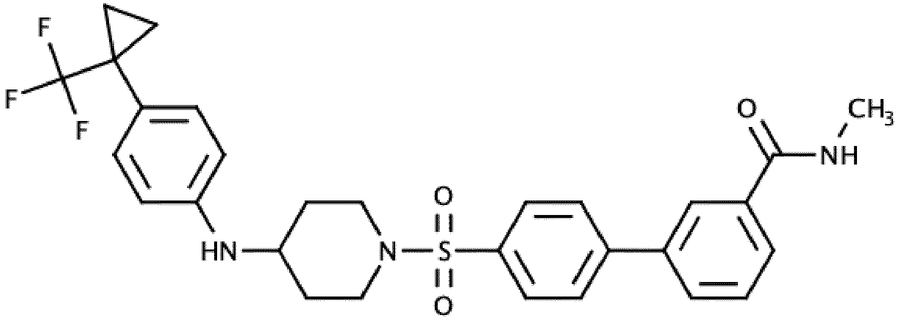

MS(ES⁺) *m*/*z* 558.4 (M+H)⁺.

¹H NMR (400 MHz, DMSO) δ 8.65 - 8.59 (m, 1H), 8.21 (t, *J=* 1.8, 1.8 Hz, 1H), 8.06 - 7.99 (m, 2H), 7.95 - 7.90 (m, 2H), 7.90 - 7.84 (m, 2H), 7.63 (t, *J=* 7.7, 7.7 Hz, 1H), 7.08 (d, *J* = 8.4 Hz, 2H), 6.55 - 6.48 (m, 2H), 5.67 (d, *J =* 8.1 Hz, 1H), 3.63 - 3.56 (m, 2H), 3.29 - 3.20 (m, 1H), 2.84 (d, *J=* 4.5 Hz, 3H), 2.62 - 2.54 (m, 2H), 1.99 - 1.92 (m, 2H), 1.48 - 1.36 (m, 2H), 1.26 - 1.12 (m, 2H), 0.94 (s, 2H).

Building block: **step i:** 1-bromo-4-[1-(trifluoromethyl)cyclopropyl]benzene; **step iv:** [3-(methylcarbamoyl)phenyl]boronic acid.

### Example 5: N-methyl-4'-{[4-({4-[1-(trifluoromethyl)cyc1opropyl]phenyl}Amino)piperidin-l-yl]sulfonyl}-[1,1'-biphenyl]-4-carboxamide.

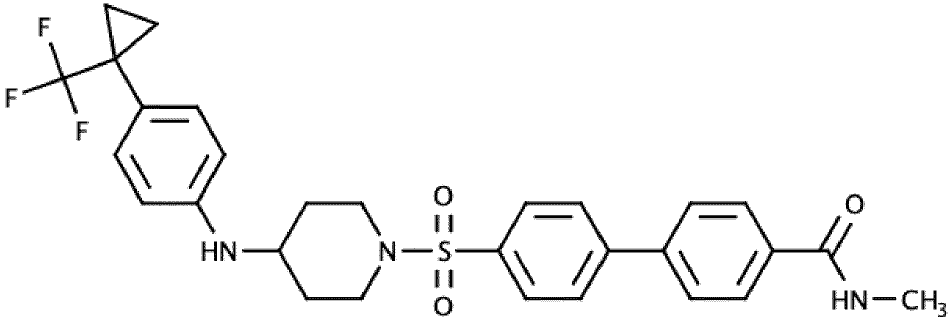

MS(ES⁺) *m*/*z* 558.4 (M+H)⁺.

¹H NMR (400 MHz, DMSO) δ 8.57 - 8.53 (m, 1H), 8.05 - 7.96 (m, 4H), 7.94 - 7.81 (m, 4H), 7.08 (d, *J=* 8.5 Hz, 2H), 6.55 - 6.47 (m, 2H), 5.66 (d, *J=* 8.1 Hz, 1H), 3.63 - 3.55 (m, 2H), 3.31 - 3.23 (m, 1H), 2.82 (d, *J=* 4.5 Hz, 3H), 2.61 - 2.54 (m, 2H), 1.99 - 1.92 (m, 2H), 1.48 - 1.36 (m, 2H), 1.23 - 1.16 (m, 2H), 0.94 (s, 2H).

Building blocks: **step i:** 1-bromo-4-[1-(trifluoromethyl)cyclopropyl]benzene; **step iv:** [4-(methylcarbamoyl)phenyl]boronic acid.

### Example 6: N-methyl-5-(4-{[4-({4-[1-(trifluoromethyl)cyclopropyl]phenyl}Amino)piperidin-1-yl]sulfonyl}phenyl)pyridine-2-carboxamide.

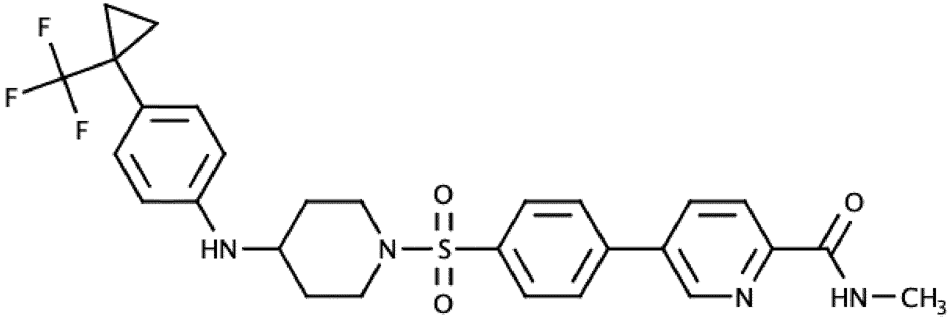

MS(ES⁺) *m*/*z* 559.4 (M+H)⁺

¹H NMR (400 MHz, DMSO) δ 9.04 (dd, *J= 2.3,* 0.8 Hz, 1H), 8.86 (q, *J* = 5.0, 4.8, 4.8 Hz, 1H), 8.38 (dd, *J* = 8.2, 2.3 Hz, 1H), 8.16 (dd, *J* = 8.2, 0.8 Hz, 1H), 8.14 - 8.09 (m, 2H), 7.96 - 7.86 (m, 2H), 7.11 - 7.05 (m, 2H), 6.55 - 6.47 (m, 2H), 5.67 (d, *J =* 8.0 Hz, 1H), 3.64 - 3.56 (m, 2H), 3.30 - 3.21 (m, 1H), 2.86 (d, *J =* 4.8 Hz, 3H), 2.62 - 2.53 (m, 2H), 1.99 - 1.92 (m, 2H), 1.48 - 1.36 (m, 2H), 1.23 - 1.16 (m, 2H), 0.94 (s, 2H).

Building blocks: **step i:** 1-bromo-4-[1-(trifluoromethyl)cyclopropyl]benzene; **step iv:** N-methyl-5-(4,4,5,5-tetramethyl-1,3,2-dioxaborolan-2-yl)pyridine-2-carboxamide.

### Example 7: 4'-{[4-({4-[1-(trifluoromethyl)cyc1opropyl]phenyl}Amino)piperidin-1-yl]sulfonyl}-[1,1'-biphenyl]-4-carboxamide.

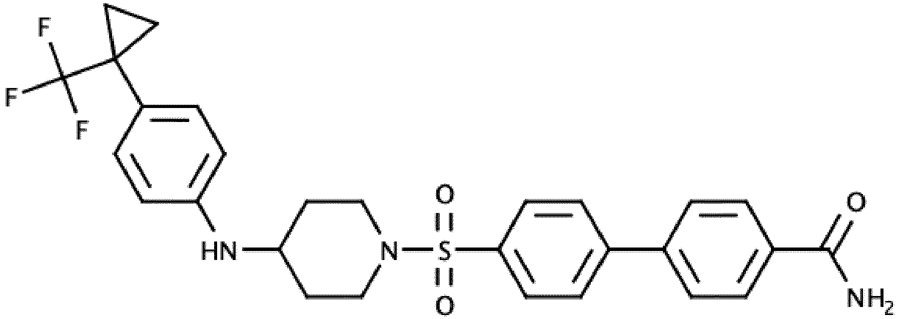

MS(ES⁺) *m*/*z* 544.3 (M+H)⁺

¹H NMR (400 MHz, DMSO) δ 8.09 (s, 1H), 8.06 - 8.00 (m, 4H), 7.91 - 7.81 (m, 4H), 7.46 (s, 1H), 7.11 - 7.05 (m, 2H), 6.55 - 6.47 (m, 2H), 5.66 (d, *J* = 8.1 Hz, 1H), 3.63 - 3.56 (m, 2H), 3.30 - 3.21 (m, 1H), 2.62 - 2.55 (m, 2H), 1.99 - 1.92 (m, 2H), 1.48 - 1.35 (m, 2H), 1.26 - 1.16 (m, 2H), 0.94 (s, 2H).

Building blocks: **step i:** 1-bromo-4-[1-(trifluoromethyl)cyclopropyl]benzene; **step iv: 4-**(4,4,5,5-tetramethyl-1,3,2-dioxaborolan-2-yl)benzamide.

### Example 8: 5-(4-{[4-({4-[1-(trifluoromethyl)cyclopropyl]phenyl}Amino)piperidin-1-yl]sulfonyl}phenyl)-1H-indole-3-carbonitrile.

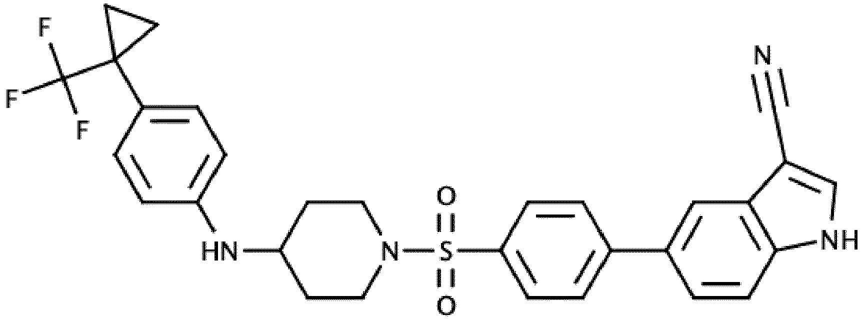

MS(ES⁺) *m*/*z* 565.4 (M+H)⁺.

¹H NMR (400 MHz, DMSO) δ 12.33 (s, 1H), 8.35 (s, 1H), 8.06 - 8.01 (m, 2H), 8.00 (t, *J* = 1.3, 1.3 Hz, 1H), 7.88 - 7.79 (m, 2H), 7.70 (t, *J=* 1.1, 1.1 Hz, 2H), 7.12 - 7.02 (m, 2H), 6.56 - 6.48 (m, 2H), 5.66 (d, *J* = 8.1 Hz, 1H), 3.64 - 3.56 (m, 2H), 3.32 - 3.21 (m, 1H), 2.61 - 2.52 (m, 2H), 2.00 - 1.92 (m, 2H), 1.50 - 1.36 (m, 2H), 1.23 - 1.15 (m, 2H), 0.94 (s, 2H)

Building blocks: **step i:** 1-bromo-4-[1-(trifluoromethyl)cyclopropyl]benzene; **step iv:** 5-(4,4,5,5-tetramethyl-1,3,2-dioxaborolan-2-yl)-1H-indole-3-carbonitrile.

### Example 9: 1-(4-{imidazo[1,2-a]pyridin-6-yl }benzenesulfonyl)-N-{4-[1-(trifluoromethyl)cyclopropyl]phenyl}piperidin-4-amine.

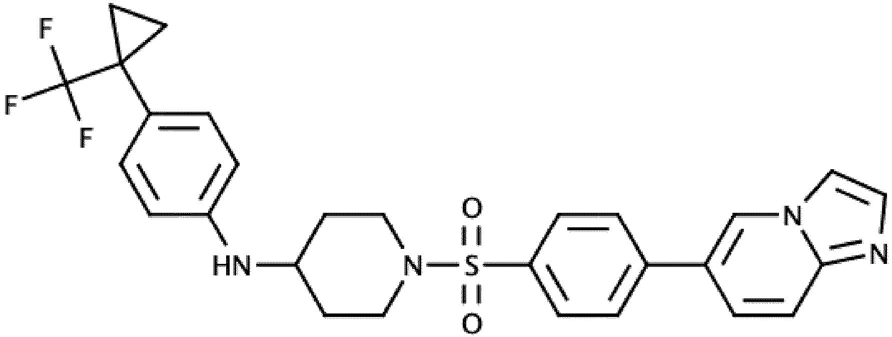

MS(ES⁺) *m*/*z* 541.4 (M+H)⁺.

¹H NMR (400 MHz, DMSO) δ 9.10 (dd, *J* = 1.9, 1.1 Hz, 1H), 8.04 - 7.98 (m, 3H), 7.89 - 7.84 (m, 2H), 7.72 (d, *J* = 9.4 Hz, 1H), 7.67 (dd, *J=* 9.7, 1.6 Hz, 2H), 7.12 - 7.05 (m, 2H), 6.56 - 6.48 (m, 2H), 5.67 (d, *J=* 8.1 Hz, 1H), 3.64 - 3.55 (m, 2H), 3.31 - 3.23 (m, 1H), 2.62 - 2.52 (m, 2H), 1.99 - 1.92 (m, 2H), 1.49 - 1.36 (m, 2H), 1.26 - 1.14 (m, 2H), 0.94 (s, 2H).

Building blocks: **step i:** 1-bromo-4-[1-(trifluoromethyl)cyclopropyl]benzene; **step iv:** 6-(4,4,5,5-tetramethyl-1,3,2-dioxaborolan-2-yl)imidazo[1,2-a]pyridine.

### Example 10: 1-{4-[3-(propan-2-yl)imidazo[1,2-a]pyridin-6-yl]benzenesulfonyl}-N-{4-[1-(trifluoromethyl)cyc1opropyl]phenyl}piperidin-4-amine.

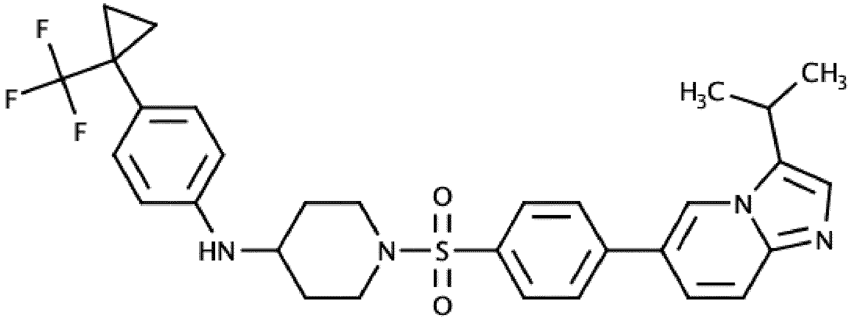

**i)** To a suspension of 1-(4-bromobenzenesulfonyl)-N-{4-[1-(trifluoromethyl)cyclopropyl]phenyl }piperidin-4-amine **(Example 2, step iii,** 176 mg), bis(pinacolato)diboron (133 mg) and potassium acetate (103 mg) in 1,4-dioxane (4 mL), purged with N₂ gas, was added PdCl₂(dppf).CH₂Cl₂ (13 mg). The reaction mixture was stirred overnight at 100 °C. After cooling to room temperature, the reaction mixture was poured into water and the product was extracted into CH₂Cl₂. The organic layer was washed with brine, dried over MgSO₄ and concentrated under reduced pressure giving N-[4-(pentafluoro-λ⁶-sulfanyl)phenyl]-1-[4-(4,4,5,5-tetramethyl-1,3,2-dioxaborolan-2-yl)benzenesulfonyl]piperidin-4-amine (190 mg) as a black solid, which was used in the next step without further purification.
**ii)** Under a nitrogen atmosphere, Pd(PPh₃)₄ (11 mg) was added to a suspension of the product obtained in the previous step (190 mg), 6-bromo-3-(propan-2-yl)imidazo[1,2-a]pyridine (117 mg), and NaHCO₃ (148 mg) in a mixture of 1,4-dioxane (4 mL) and water (1 mL). The reaction mixture was stirred for 1 hour at 120 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 10% to 60% CH₃CN in water as the eluent, to give the title compound 1-{4-[3-(propan-2-yl)imidazo[1,2-a]pyridin-6-yl]benzenesulfonyl}-N-{4-[1-(trifluoromethyl)cyclopropyl]phenyl}piperidin-4-amine (55 mg) as a white solid. MS(ES⁺) *m*/*z* 583.4 (M+H)⁺.

¹H NMR (400 MHz, DMSO) δ 8.74 - 8.69 (m, 1H), 8.12 - 8.05 (m, 2H), 7.89 - 7.82 (m, 2H), 7.70 (dd, *J=* 9.4, 0.9 Hz, 1H), 7.63 (dd, *J* = 9.4, 1.8 Hz, 1H), 7.46 (d, *J=* 0.8 Hz, 1H), 7.08 (d, *J* = 8.3 Hz, 2H), 6.56 - 6.48 (m, 2H), 5.67 (d, *J* = 8.1 Hz, 1H), 3.65 - 3.58 (m, 2H), 3.52 (p, *J* = 6.9, 6.9, 6.8, 6.8 Hz, 1H), 3.33 - 3.22 (m, 1H), 2.61 - 2.54 (m, 2H), 2.00 - 1.93 (m, 2H), 1.49 - 1.40 (m, 2H), 1.37 (s, 3H), 1.36 (s, 3H), 1.26 - 1.12 (m, 2H), 0.94 (s, 2H).

### Example 11: N-(4-cyclopropylphenyl)-1-[4-(3-methyl-1H-indazol-5-yl)benzenesulfonyl]piperidin-4-amine.

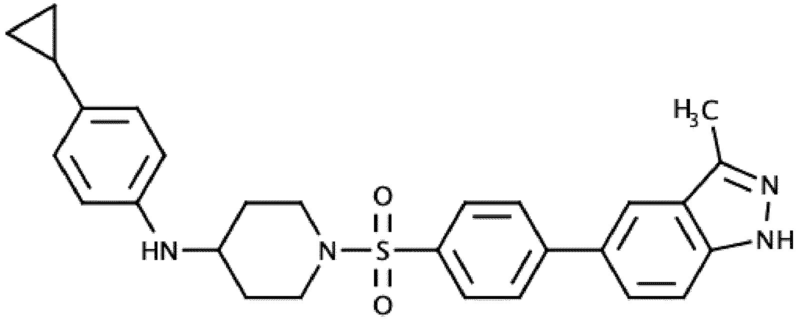

**i)** To a suspension of piperidin-4-one (10.0 g) and triethyl amine (27 mL) in CH₂Cl₂ (200 mL) was added portion wise at room temperature 4-bromobenzene-1-sulfonyl chloride (16.6 g). The reaction mixture was stirred over weekend at room temperature and quenched by addition of water. The product was extracted into CH₂Cl₂ and the combined organic layers were washed with brine, dried over MgSO₄, filtered and concentrated under reduced pressure. The obtained brown oil was dissolved in a little ethyl acetate and heptane was added. Overnight the product precipitated and the solid was filtered off and dried under reduced pressure to give 1-(4-bromobenzenesulfonyl)piperidin-4-one (15.1 g) as an off-white solid, which was used in the next step without further purification.
**ii)** Under a nitrogen atmosphere, 2-Methylpyridine borane complex (51 mg) was added at 0 °C, to a solution of the product obtained in the previous step (150 mg), 4-cyclopropylaniline hydrochloride (80 mg) and trifluoroacetic acid (300 uL) in methanol (3 mL). The reaction mixture was stirred overnight at room temperature. After the reaction mixture was concentrated under reduced pressure, an aqueous 2N HCl solution (20 mL) was added at 0 °C, and the resulting mixture was stirred for 1 hour at room temperature. The mixture was basified by the addition of an aqueous 5N NaOH solution (20 mL) and the product was extracted into CH₂Cl₂. The combined organic layers were washed with brine, dried over MgSO₄, filtered, and concentrated under reduced pressure. The obtained brown oil was purified on SiO₂, using 30% ethyl acetate in heptane as the eluent, to give 1-(4-bromobenzenesulfonyl)-N-(4-cyclopropylphenyl)piperidin-4-amine (146 mg) as a brown solid, which was used in the next step without further purification.
**iii)** Under a nitrogen atmosphere, Pd(PPh₃)₄ (19 mg) was added to a suspension of the product obtained in the previous step (146 mg), (3-methyl-1H-indazol-5-yl)boronic acid (65 mg), and NaHCO₃ (169 mg) in a mixture of 1,4-dioxane (3 mL) and water (3 mL). The reaction mixture was stirred for 2 hours at 110 °C in a microwave. After cooling to room temperature, the reaction mixture was filtered over Celite, diluted with water and ethyl acetate and the product was extracted into ethyl acetate. The combined organic layers were washed with water, brine, dried over MgSO₄, filtered, and concentrated under reduced pressure. The obtained brown oil was purified on C18, using 10% to 90% acetonitrile in water as the eluent, to give the title compound N-(4-cyclopropylphenyl)-1-[4-(3-methyl-1H-indazol-5-yl)benzenesulfonyl]piperidin-4-amine (27 mg) as a white solid. MS(ES⁺) *m*/*z* 487.3 (M+H)⁺.

¹H NMR (400 MHz, DMSO) δ 12.78 (s, 1H), 8.13 (dd, *J* = 1.8, 0.8 Hz, 1H), 8.05 - 7.98 (m, 2H), 7.85 - 7.80 (m, 2H), 7.75 (dd, *J* = 8.8, 1.7 Hz, 1H), 7.59 (dd, *J* = 8.7, 0.8 Hz, 1H), 6.79 - 6.71 (m, 2H), 6.49 - 6.41 (m, 2H), 5.22 (d, *J=* 8.3 Hz, 1H), 3.63 - 3.55 (m, 2H), 3.25 - 3.16 (m, 1H), 2.57 (s, 3H), 2.55 - 2.52 (m, 2H), 1.99 - 1.92 (m, 2H), 1.71 (tt, *J* = 8.4, 8.4, 5.1, 5.1 Hz, 1H), 1.46 - 1.33 (m, 2H), 0.81 - 0.70 (m, 2H), 0.50 - 0.41 (m, 2H).

Following a procedure analogous to that described for **Example 11,** using in **step iii** the appropriate boronic ester or boronic acid, **Example 12** has been prepared.

### Example 12: 5-[4-({4-[(4-cyclopropylphenyl)Amino]piperidin-1-yl}sulfonyl)phenyl]-1H-indole-3-carbonitrile.

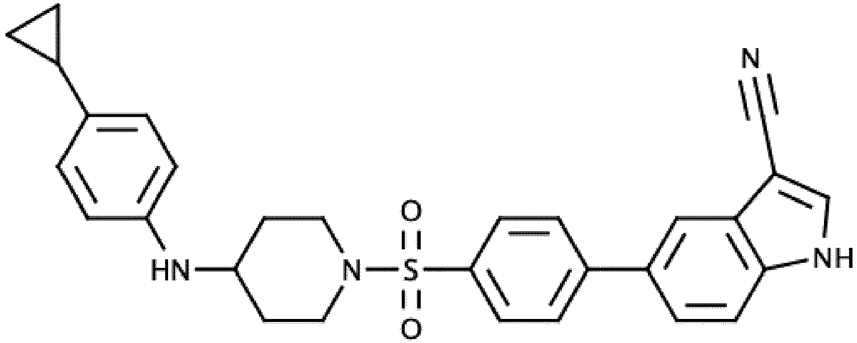

MS(ES⁺) *m*/*z* 497.3 (M+H)⁺.

¹H NMR (400 MHz, DMSO) δ 12.36 (s, 1H), 8.34 (s, 1H), 8.06 - 8.01 (m, 2H), 7.99 (t, *J=* 1.3, 1.3 Hz, 1H), 7.86 - 7.78 (m, 2H), 7.70 (d, *J=* 1.3 Hz, 2H), 6.79 - 6.71 (m, 2H), 6.49 - 6.41 (m, 2H), 5.22 (d, *J* = 8.4 Hz, 1H), 3.63 - 3.56 (m, 2H), 3.27 - 3.16 (m, 1H), 2.62 - 2.53 (m, 2H), 1.99 - 1.91 (m, 2H), 1.71 (tt, *J* = 8.4, 8.4, 5.1, 5.1 Hz, 1H), 1.46 - 1.34 (m, 2H), 0.81 - 0.70 (m, 2H), 0.50 - 0.41 (m, 2H).

Building block: step ii: 5-(4,4,5,5-tetramethyl-1,3,2-dioxaborolan-2-yl)-1H-indole-3-carbonitrile.

### Example 13: (4-{imidazo[1,2-a]pyridin-6-yl }phenyl)[trans-4-({4-[1-(trifluoromethyl)cyclopropyl]phenyl}Amino)cyclohexyl](imino)-λ⁶-sulfanone.

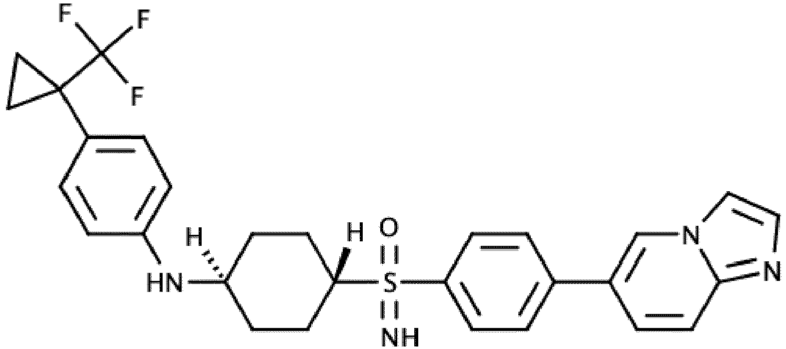

**i)** To a 0 °C cold solution of 4-hydroxycyclohexanone monoethylene ketal (50 g) and Et₃N (57 mL) in CH₂Cl₂ (100 mL) was added dropwise a solution of methanesulfonyl chloride (32 mL) in CH₂Cl₂ (100 mL). The reaction mixture was stirred overnight at room temperature. After completion the reaction mixture was poured into a saturated aqueous NaHCO₃ solution, and the product was extracted into CH₂Cl₂. The combined organic layers were washed with brine, dried over MgSO₄, filtered and concentrated under reduced pressure to give 1,4-dioxaspiro[4.5]decan-8-yl methanesulfonate as a yellow oil (82 g), which was used in the next step without further purification.
**ii)** To a suspension of the product obtained in the previous step (74.7 g) and Cs₂CO₃ (257.4 g) in acetone (1500 mL) was added at room temperature 4-bromothiophenol (89.6 g). The reaction mixture was stirred overnight at 60 °C. After the reaction mixture was concentrated under reduced pressure, water and ethyl acetate were added. The product was extracted into ethyl acetate and the combined organic layers were washed with water, brine, dried over MgSO₄, filtered, and concentrated under reduced pressure. The obtained brown oil (130 g) was purified on SiO₂, using 0% to 100% EtOAc in heptane as the eluent, giving 8-[(4-bromophenyl)sulfanyl]-1,4-dioxaspiro[4.5]decane as a clear oil (64.7 g), which slowly solidified.
**iii)** To a suspension of the product obtained in the previous step (5 g) and ammonium carbamate (1.8 g) in a mixture of methanol (10 mL) and acetonitrile (10 mL) was added at room temperature (diacetoxyiodo)benzene (10.2 g). The reaction mixture was stirred at room temperature for 1 hour in a flask open to the atmosphere. After completion, the reaction mixture was concentrated under reduced pressure. At this stage, the cis/trans mixture could be separated on C18, using 10% to 100% acetonitrile in water, to give [(4-bromophenyl)({1,4-dioxaspiro[4.5]decan-8-yl})imino-λ⁶-sulfanyl]one (5.3 g).
**iv)** To a solution of the product obtained in the previous step (5.3 g) in THF (100 mL) was added an aqueous 2N HCl solution (110 mL) and the reaction mixture was stirred overnight at room temperature. After completion, the organic solvent was removed under reduced pressure and the remaining aqueous layer was washed with EtOAc. The combined organic layers were washed with water, brine, dried over MgSO₄, filtered and concentrated under reduced pressure to give 4-[(4-bromophenyl)oxo-λ⁶-sulfanyl]cyclohexan-1-one as an yellowish oil (4.6 g), which was used in the next step without further purification.
**v)** To a solution of 4-[1-(trifluoromethyl)cyclopropyl]aniline hydrochloride (950 mg) in a mixture of CH₂Cl₂ (9 ml) and methanol (1 mL) was added SillaBond^{®} Carbonate (Si-CO₃) and the resulting mixture was stirred for 30 minutes at room temperature. The mixture was filtered, concentrated under reduced pressure to give 4-[1-(trifluoromethyl)cyclopropyl]aniline. To a solution of the product obtained in the previous step (998 mg), 4-[1-(trifluoromethyl)cyclopropyl]aniline and HOAc (2 mL) in methanol (10 mL) was added at 0 °C, 2-methylpyridine borane complex (1.46 g). The reaction mixture was stirred overnight at room temperature. After completion the reaction mixture was concentrated under reduced pressure and after cooling to 0 °C, an aqueous solution of 2N HCl (5 mL) was added. After stirring for 1 hour at 0 °C, an aqueous solution of 5N NaOH was added. The product was extracted into EtOAc and the combined organic layers were washed with water, brine, dried over MgSO₄, filtered and concentrated under reduced pressure. The residue was purified on SiO₂, using 0% to 95% ethyl acetate in heptane as the eluent, giving (4-bromophenyl)[*trans*-4-({4-[1-(trifluoromethyl)cyclopropyl]phenyl}Amino)cyclohexyl](imino)-λ⁶-sulfanone (400 mg) as a white solid.
**vi)** Under a nitrogen atmosphere, Pd(PPh₃)₄ (12 mg) was added to a suspension of the product obtained in the previous step (100 mg), 6-(4,4,5,5-tetramethyl-1,3,2-dioxaborolan-2-yl)imidazo[1,2-a]pyridine (61 mg) and NaHCO₃ (84 mg) in a mixture of 1,4-dioxane (4 mL) and water (1 mL). The reaction mixture was stirred for 1hour at 120 °C in a microwave. After cooling to room temperature, the product was extracted into EtOAc and the organic layer was dried over MgSO₄, filtered, and concentrated under reduced pressure. The obtained brown solid was purified on C18, using 60% to 95% acetonitrile in water as the eluent, giving the title compound (4-{imidazo[1,2-a]pyridin-6-yl}phenyl)[trans-4-({4-[1-(trifluoromethyl)cyclopropyl]phenyl}Amino)cyclohexyl](imino)-λ⁶-sulfanone as a white solid (48 mg). MS(ES⁺) *m*/*z* 539.4 (M+H)⁺.

¹H NMR (400 MHz, DMSO) δ 9.09 (t, *J =* 1.5, 1.5 Hz, 1H), 8.02 - 8.00 (m, 1H), 8.00 - 7.93 (m, 4H), 7.71 (d, *J=* 9.4 Hz, 1H), 7.69 - 7.64 (m, 2H), 7.12 - 7.05 (m, 2H), 6.55 - 6.47 (m, 2H), 5.57 (d, *J=* 7.9 Hz, 1H), 4.26 (s, 1H), 3.13 - 3.03 (m, 2H), 2.06 - 1.95 (m, 4H), 1.58 - 1.40 (m, 2H), 1.26 - 1.10 (m, 4H), 0.94 (s, 2H).

### Example 14: 4-(2,2-difluorocyclopropyl)-N-[trans-4-(4-{imidazo[1,2-a]pyridin-6-yl}benzenesulfonyl)cyclohexyl]aniline.

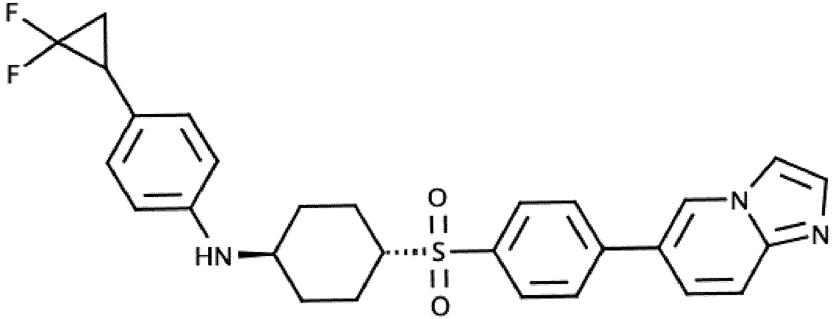

**i)** To a suspension of tert-butyl N-[4-(methanesulfonyloxy)cyclohexyl]carbamate (43.7 g) and Cs₂CO₃ (120.6 g) in acetone (400 mL) was added at room temperature, 4-bromothiophenol (42.0 g). The reaction mixture was stirred overnight at 60°C. After completion the reaction mixture was filtered, and the filtrate was concentrated under reduced pressure. The residue was dissolved in a water/ethyl acetate mixture and the product was extracted into ethyl acetate. The combined organic layers were washed with brine, dried over MgSO₄, filtered, and concentrated under reduced pressure. The obtained brown oil was crystalized from acetonitrile to give a white solid. The solid was washed with cold heptane to give tert-butyl N-[trans-4-[(4-bromophenyl)sulfanyl]cyclohexyl]carbamate (36.5 g) as a white solid.
**ii)** To a solution of the product obtained in the previous step (23 g) in ethyl acetate (200 mL) was added mCPBA (51.4 g) and the reaction mixture was stirred overnight at room temperature. After completion the reaction mixture was poured into water and the product was extracted into ethyl acetate. The combined organic layers were washed with aqueous 1N NaOH solution, water, brine, dried over MgSO₄ filtered, and concentrated under reduced pressure. To remove last traces of 3-chlorobenzoic acid, the obtained white solid was dissolved in ethyl acetate and stirred for 30 minutes in the presence of silica bound carbonate. The mixture was filtered and the filtrate was concentrated under reduced pressure to give tert-butyl N-[trans-4-(4-bromobenzenesulfonyl)cyclohexyl]carbamate (12.9 g) as a white solid.
**iii)** Under a nitrogen atmosphere, Pd(PPh₃)₄ (276 mg) was added to a solution of the product obtained in the previous step (2.0 g), 6-(4,4,5,5-tetramethyl-1,3,2-dioxaborolan-2-yl)imidazo[1,2-a]pyridine (1.51) and NaHCO₃ (2.4 g) in a mixture of 1,4-dioxane (12 mL) and water (3 mL). The reaction mixture was stirred for 2 hours at 100 °C in a microwave. After cooling to room temperature, the reaction mixture was poured into water and the product was extracted into ethyl acetate. The combined organic layers were washed with water, brine, dried over MgSO₄, filtered, and concentrated under reduced pressure. The obtained brown oil was purified on C18, using 10% to 100% acetonitrile in water as the eluent, to give tert-butyl N-[trans-4-(4-limidazo[1,2-alpyridin-6-yllbenzenesulfonyl)cyclohexyllcarbamate (2.2 g) as a clear solid.
**iv)** To a solution of the product obtained in the previous step (2.0 g) in ethyl acetate (40 mL) was added at room temperature, a 5N HCl solution in 2-propanol (8.9 mL). The reaction mixture was stirred overnight at room temperature. After completion the reaction mixture concentrated under reduced pressure to give trans-4-(4-{imidazo[1,2-a]pyridin-6-yl}benzenesulfonyl)cyclohexan-1-amine hydrochloride (1.9 g) as a yellow solid, which was used in the next step without further purification.
**v)** Under a nitrogen atmosphere, Pd₂(dba)₃ (23 mg) was added to a suspension of the product obtained in the previous step (100 mg), 1-bromo-4-(2,2-difluorocyclopropyl)benzene (71 mg), sodium tert-butoxide (147 mg) and xantphos (37 mg) in toluene (15 mL). The reaction mixture was stirred overnight at 100 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The obtained brown oil was purified on C18, using 10% to 90% acetonitrile in water as the eluent, to give the title compound 4-(2,2-difluorocyclopropyl)-N-[trans-4-(4-{imidazo[1,2-a]pyridin-6-yl}benzenesulfonyl)cyclohexyl]aniline (10 mg) as a white solid. MS(ES⁺) *m*/*z* 508.3 (M+H)⁺.

¹H NMR (400 MHz, DMSO) δ 9.11 (t, *J =* 1.4, 1.4 Hz, 1H), 8.06 - 8.02 (m, 2H), 8.01 (s, 1H), 7.99 - 7.94 (m, 2H), 7.72 (d, *J=* 9.4 Hz, 1H), 7.68 (dd, *J* = 9.5, 1.9 Hz, 1H), 7.66 (d, *J* = 1.2 Hz, 1H), 6.95 - 6.89 (m, 2H), 6.57 - 6.49 (m, 2H), 5.42 (d, *J =* 8.1 Hz, 1H), 3.38 - 3.34 (m, 1H), 3.20 - 3.09 (m, 1H), 2.76 (td, *J=* 12.7, 12.7, 8.1 Hz, 1H), 2.10 - 1.95 (m, 4H), 1.89 - 1.75 (m, 1H), 1.74 - 1.61 (m, 1H), 1.61 - 1.47 (m, 2H), 1.22 - 1.10 (m, 2H).

Following a procedure analogous to that described for **Example 14,** using in **step iii** the appropriate boronic ester or boronic acid and in **step v** the appropriate (hetero)aryl halide, **Example 15** has been prepared

### Example 15: 1-(4-{[trans-4-(4-{imidazo[1,2-a]pyridin-6-yl}benzenesulfonyl)cyclohexyl]Amino}phenyl)cyclopropane-1-carbonitrile.

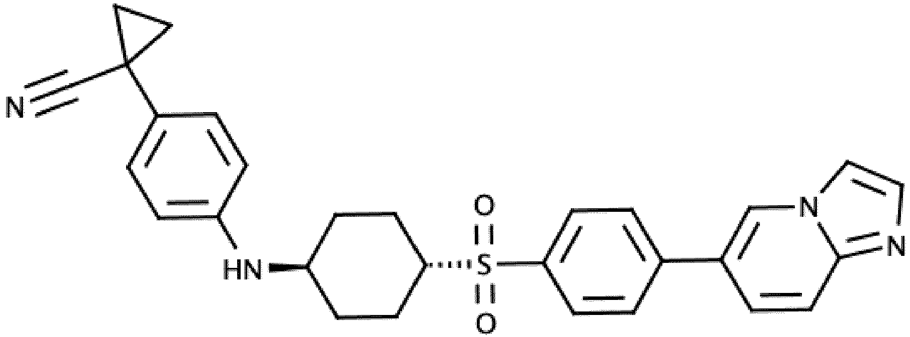

MS(ES⁺) *m*/*z* 497.3 (M+H)⁺

¹H NMR (400 MHz, DMSO) δ 9.11 (dd, *J* = 1.9, 1.1 Hz, 1H), 8.05 - 8.02 (m, 2H), 8.01 (t, *J=* 0.9, 0.9 Hz, 1H), 7.99 - 7.94 (m, 2H), 7.72 (dt, *J=* 9.5, 0.9, 0.9 Hz, 1H), 7.68 (dd, *J* = 9.5, 1.9 Hz, 1H), 7.66 (d, *J=* 1.2 Hz, 1H), 7.05 - 6.97 (m, 2H), 6.59 - 6.51 (m, 2H), 5.58 (d, *J* = 8.1 Hz, 1H), 3.38 - 3.33 (m, 1H), 3.22 - 3.11 (m, 1H), 2.10 - 1.95 (m, 4H), 1.62 - 1.47 (m, 4H), 1.31 - 1.25 (m, 2H), 1.21 - 1.10 (m, 2H).

Building block: **step iii:** 6-(4,4,5,5-tetramethyl-1,3,2-dioxaborolan-2-yl)imidazo[1,2-a]pyridine; **step v:** 1-(4-bromophenyl)cyclopropane-1-carbonitrile.

### Example 16: 5-(4-{[trans-4-({4-[1-(trifluoromethyl)cyclopropyl]phenyl}Amino)cyclohexyl]sulfonyl}phenyl)-1H-indole-3-carbonitrile.

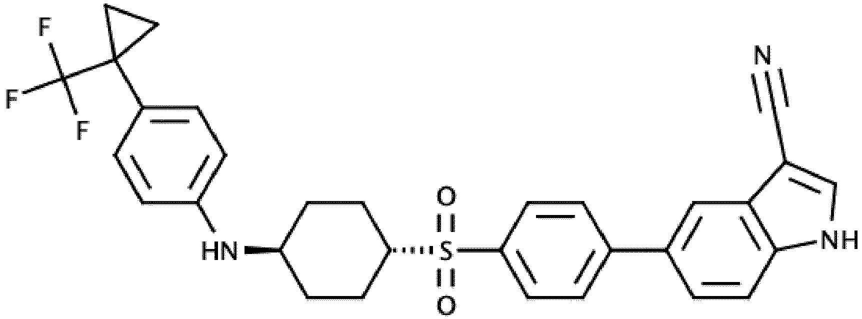

**i)** A solution of methanesulfonyl chloride (1.3 mL) in CH₂Cl₂ (10 mL) was added dropwise to a solution of 4-hydroxycyclohexanone monoethylene ketal (2.0 g) and triethyl amine (2.3 mL) in CH₂Cl₂ (15 mL) at 0 °C. The reaction mixture was stirred overnight at room temperature. After completion the reaction mixture was quenched by pouring into a saturated aqueous NaHCO₃ solution. The layers were separated, and the aqueous layers were washed with CH₂Cl₂. The combined organic layers were washed brine, dried over MgSO₄, filtered, and concentrated under reduced pressure. The obtained yellow oil was dissolved in heptane, the precipitated solid was filtered off and the filtrate was reduced under reduced pressure to give 1,4-dioxaspiro[4.5]decan-8-yl methanesulfonate (3.3 g) as an oil, which slowly solidified. The product was used in the next step without further purification.
**ii)** 4-bromobenzene-1-thiol (2.64 g) was added to a suspension of the product obtained in the previous step (3.3 g) and cesium carbonate (5.46 g) in acetone (50 mL). The reaction mixture was stirred overnight at 60 °C. After cooling to room temperature, the reaction mixture was filtered, and the solids were washed with ethyl acetate. The filtrate was concentrated under reduced pressure and the obtained oil was purified on SiO₂, using 0% to 60% ethyl acetate in heptane as the eluent, giving 8-[(4-bromophenyl)sulfanyll-1,4-dioxaspiro[4.5]decane (3.68 g) as a white solid.
**iii)** 3-chloroperbenzoic acid (9.64 g) was added to a solution of the product obtained in the previous step (3.68 g) in ethyl acetate (50 mL) and the reaction mixture was stirred overnight at room temperature. The reaction mixture was washed with a saturated NaHCO₃ solution, and the combined organic layers were washed with brine, dried over MgSO₄, filtered, and concentrated under reduced pressure. The residue was purified on SiO₂, using 0% to 100% ethyl acetate in heptane as the eluent, giving 8-(4-bromobenzenesulfonyl)-1,4-dioxaspiro[4.5]decane (3.68 g) as a white solid.
**iv)** To a solution of the product obtained in the previous step (3.5 g) in THF (50 mL) was added at room temperature an aqueous 2N HCl solution (39 mL) and the reaction mixture was stirred overnight at room temperature. After completion, the reaction mixture was washed with ethyl acetate and the combined organic layers were washed with brine, dried over MgSO₄, filtered and concentrated under reduced pressure to give 4-(4-bromobenzenesulfonyl)cyclohexan-1-one (3.0 g) as a white solid which was used in the next step without further purification.
**v)** To a suspension of the product obtained in the previous step (501 mg) and 4-[1-(trifluoromethyl)cyclopropyl]aniline hydrochloride (498 mg) in a mixture of HOAc (1 mL) in CH₃OH (10 mL) was added 2-methylpyridine borane complex (183 mg) at 0 °C. After removal of the ice bath the reaction mixture was stirred overnight at room temperature. The reaction mixture was concentrated under reduced pressure and an aqueous 2N HCl solution (5 mL) was added at 0 °C. After stirring for 1h at 0 °C, an aqueous 5N NaOH solution (5 mL) was added, and the product was extracted into ethyl acetate. The organic layer was washed with brine, dried over MgSO₄, and concentrated under reduced pressure. The residue was purified on SiO₂, using 0% to 50% ethyl acetate in heptane as the eluent, giving N-[trans-4-(4-bromobenzenesulfonyl)cyclohexyl]-4-[1-(trifluoromethyl)cyclopropyl]aniline (69 mg) as a white solid.
**vi)** Under a nitrogen atmosphere, Pd(PPh₃)₄ (10 mg) was added to a solution of the product obtained in the previous step (69 mg), 5-(4,4,5,5-tetramethyl-1,3,2-dioxaborolan-2-yl)-1H-indole-3-carbonitrile (53 mg) and NaHCO₃ (62 mg) in a mixture of 1,4-dioxane (4 mL) and ethanol (1 mL). The reaction mixture was stirred for 1 hours at 120 °C in a microwave. After cooling to room temperature, the reaction mixture was poured into water and the product was extracted into ethyl acetate. The combined organic layers were washed with water, brine, dried over MgSO₄, filtered, and concentrated under reduced pressure. The residue was purified on C18, using 60% to 95% acetonitrile in water as the eluent, giving the title compound 5-(4-{[trans-4-({4-[1-(trifluoromethyl)cyclopropyl]phenyl}Amino)cyclohexyl]sulfonyl}phenyl)-1H-indole-3-carbonitrile (48 mg) as a white solid. MS(ES⁺) *m*/*z* 564.4 (M+H)⁺ .

¹H NMR (400 MHz, DMSO) δ 12.37 (s, 1H), 8.35 (s, 1H), 8.09 - 8.03 (m, 2H), 8.01 (t, *J=* 1.2, 1.2 Hz, 1H), 7.96 - 7.89 (m, 2H), 7.71 (t, *J=* 1.0, 1.0 Hz, 2H), 7.09 (d, *J=* 8.5 Hz, 2H), 6.58 - 6.49 (m, 2H), 5.60 (d, *J =* 8.0 Hz, 1H), 3.40 - 3.34 (m, 1H), 3.21 - 3.11 (m, 1H), 2.09 - 1.94 (m, 4H), 1.61 - 1.46 (m, 2H), 1.27 - 1.10 (m, 4H), 1.00 - 0.90 (m, 2H).

Following a procedure analogous to that described for **Example 16,** using in **step v** the appropriate aniline and in **step vi** the appropriate boronic ester or boronic acid, **Examples 17** - **19** have been prepared.

### Examples 17 -19

### Example 17: N-[trans-4-(4-{imidazo[1,2-a]pyridin-6-yl}benzenesulfonyl)cyclohexyl]-4-[1-(trifluoromethyl)cyclopropyl]aniline.

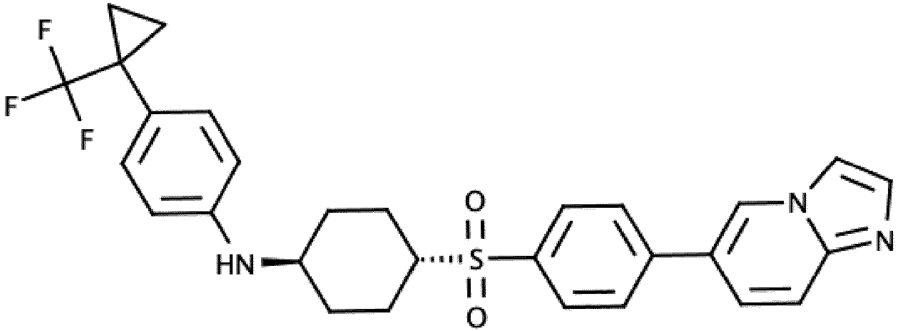

MS(ES⁺) *m*/*z* 540.4 (M+H)⁺

¹H NMR (400 MHz, DMSO) δ 9.11 (dd, *J* = 1.9, 1.1 Hz, 1H), 8.06 - 8.02 (m, 2H), 8.02 - 8.00 (m, 1H), 7.99 - 7.94 (m, 2H), 7.72 (dt, *J* = 9.4, 0.9, 0.9 Hz, 1H), 7.68 (dd, *J* = 9.5, 1.9 Hz, 1H), 7.66 (d, *J* = 1.2 Hz, 1H), 7.13 - 7.05 (m, 2H), 6.57 - 6.49 (m, 2H), 5.60 (d, *J* = 8.0 Hz, 1H), 3.39 - 3.34 (m, 1H), 3.22 - 3.11 (m, 1H), 2.11 - 1.95 (m, 4H), 1.61 - 1.47 (m, 2H), 1.26 - 1.10 (m, 4H), 0.95 (s, 2H).

Building blocks: **step v:** 4-[1-(trifluoromethyl)cyclopropyl]aniline; **step vi:** 6-(4,4,5,5-tetramethyl-1,3,2-dioxaborolan-2-yl)imidazo[1,2-a]pyridine.

### Example 18: 4-(oxetan-3-yl)-N-[trans-4-(4-{imidazo[1,2-a]pyridin-6-yl}benzenesulfonyl)cyclohexyl]aniline.

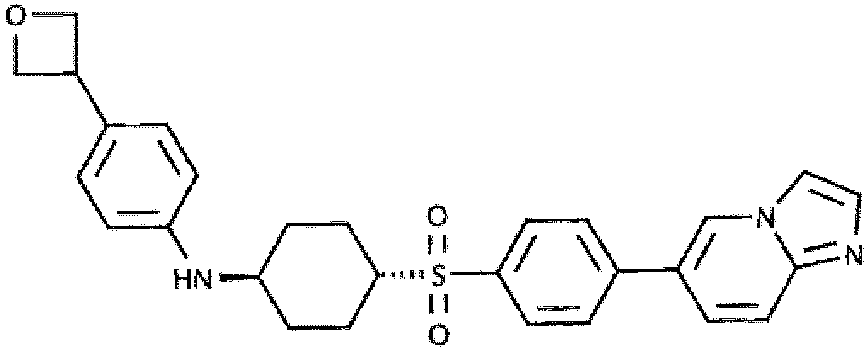

MS(ES⁺) *m*/*z* 488.4 (M+H)⁺

¹H NMR (400 MHz, DMSO) δ 9.11 (dd, *J* = 1.9, 1.1 Hz, 1H), 8.05 - 8.02 (m, 2H), 8.01 (s, 1H), 7.98 - 7.95 (m, 2H), 7.72 (d, *J=* 9.4 Hz, 1H), 7.68 (dd, *J=* 9.5, 1.9 Hz, 1H), 7.66 (d, *J* = 1.2 Hz, 1H), 7.11 - 7.03 (m, 2H), 6.60 - 6.52 (m, 2H), 5.36 (d, *J* = 8.1 Hz, 1H), 4.84 (dd, *J*= 8.4, 5.7 Hz, 2H), 4.52 (dd, *J* = 7.0, 5.7 Hz, 2H), 4.05 (ddd, *J=* 15.4, 8.4, 7.0 Hz, 1H), 3.37 - 3.27 (m, 1H), 3.21 - 3.10 (m, 1H), 2.11 - 1.95 (m, 4H), 1.61 - 1.47 (m, 2H), 1.22 - 1.10 (m, 2H)

Building block: **step v:** 4-(oxetan-3-yl)aniline; **step vi:** 6-(4,4,5,5-tetramethyl-1,3,2-dioxaborolan-2-yl)imidazo[1,2-a]pyridine.

### Example 19: 5-(4-{[trans-4-{[4-(oxetan-3-yl)phenyl]Amino}cyclohexyl]sulfonyl}phenyl)-1H-indole-3-carbonitrile.

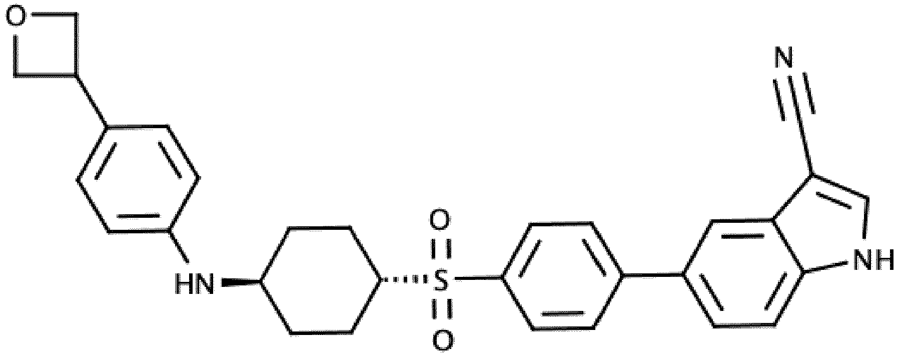

MS(ES⁺) *m*/*z* 512.3 (M+H)⁺

¹H NMR (400 MHz, DMSO) δ 12.37 (s, 1H), 8.35 (s, 1H), 8.09 - 8.04 (m, 2H), 8.01 (t, 1H), 7.96 - 7.89 (m, 2H), 7.75 - 7.66 (m, 2H), 7.11 - 7.03 (m, 2H), 6.61 - 6.52 (m, 2H), 5.36 (d, *J=* 8.2 Hz, 1H), 4.84 (dd, *J* = 8.4, 5.7 Hz, 2H), 4.52 (dd, *J* = 7.0, 5.7 Hz, 2H), 4.11 - 3.99 (m, 1H), 3.32 - 3.24 (m, 1H), 3.21 - 3.10 (m, 1H), 2.11 - 1.93 (m, 4H), 1.61 - 1.47 (m, 2H), 1.22 - 1.11 (m, 2H).

Building block: **step v:** 4-(oxetan-3-yl)aniline; **step vi:** 5-(4,4,5,5-tetramethyl-1,3,2-dioxaborolan-2-yl)-1H-indole-3-carbonitrile.

### Example 20: N-[trans-4-{4-[3-(propan-2-yl)imidazo[1,2-a]pyridin-6-yl]benzenesulfonyl}cyclohexyl]-4-[1-(trifluoromethyl)cyclopropyl]aniline.

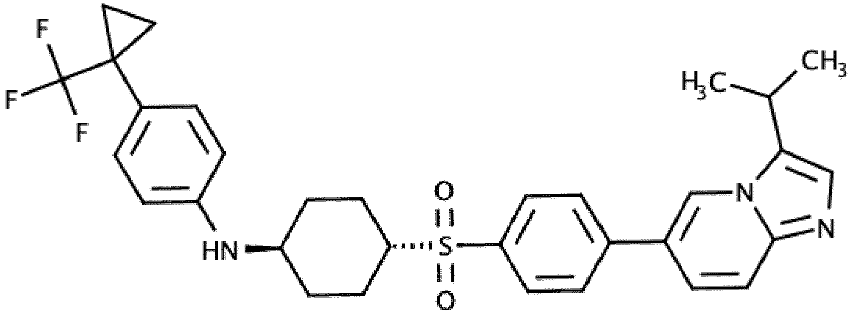

i) To a suspension of N-[trans-4-(4-bromobenzenesulfonyl)cyclohexyl]-4-[1-(trifluoromethyl)cyclopropyl]aniline **(Example 16, step v,** 300 mg), bis(pinacolato)diboron (133 mg) and potassium acetate (228 mg) in 1,4-dioxane (10 mL), purged with N₂ gas, was added PdCl₂(dppf).CH₂Cl₂ (30 mg). The reaction mixture was heated overnight at 100 °C in a microwave. After cooling to room temperature, the reaction mixture was poured into water and the product was extracted into CH₂Cl₂. The organic layer was washed with brine, dried over MgSO₄ and concentrated under reduced pressure giving N-[trans-4-[4-(4,4,5,5-tetramethyl-1,3,2-dioxaborolan-2-yl)benzenesulfonyl]cyclohexyl]-4-[1-(trifluoromethyl)cyclopropyl]aniline (210 mg) as a black solid, which was used in the next step without further purification.
**ii)** Under a nitrogen atmosphere, Pd(PPh₃)₄ (11 mg) was added to a suspension of the product obtained in the previous step (210 mg), 6-bromo-3-(propan-2-yl)imidazo[1,2-a]pyridine (114 mg), and NaHCO₃ (173 mg) in a mixture of 1,4-dioxane (4 mL) and water (1 mL). The reaction mixture was stirred for 30 minutes at 120 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 20% to 95% CH₃CN in water as the eluent, to give the title compound N-[trans-4-{4-[3-(propan-2-yl)imidazo[1,2-a]pyridin-6-yl]benzenesulfonyl}cyclohexyl]-4-[1-(trifluoromethyl)cyclopropyl]aniline (55 mg) as a white solid. MS(ES⁺) *m*/*z* 582.4 (M+H)⁺.

¹H NMR (400 MHz, DMSO) δ 8.77 (t, *J* = 1.4, 1.4 Hz, 1H), 8.14 - 8.07 (m, 2H), 8.00 - 7.93 (m, 2H), 7.73 (dd, *J* = 9.5, 1.0 Hz, 1H), 7.69 (dd, *J* = 9.5, 1.7 Hz, 1H), 7.51 (s, 1H), 7.13 - 7.06 (m, 2H), 6.57 - 6.49 (m, 2H), 5.61 (d, *J=* 7.9 Hz, 1H), 3.53 (p, *J* = 6.8, 6.8, 6.8, 6.8 Hz, 1H), 3.42 - 3.27 (m, 1H), 3.22 - 3.09 (m, 1H), 2.09 - 1.95 (m, 4H), 1.62 - 1.47 (m, 2H), 1.37 (s, 3H), 1.36 (s, 3H), 1.24 - 1.10 (m, 4H), 0.97 - 0.93 (m, 2H).

Following a procedure analogous to that described for **Example 20,** using in **step ii** the appropriate (hetero)aryl halide, **Examples 21** and 22 have been prepared.

### Example 21: N-[(trans)-4-(4-{3-methyl-1H-pyrrolo[2,3-b]pyridin-5-yl}benzenesulfonyl)cyclohexyl]-4-[1-(trifluoromethyl)cyclopropyl]aniline.

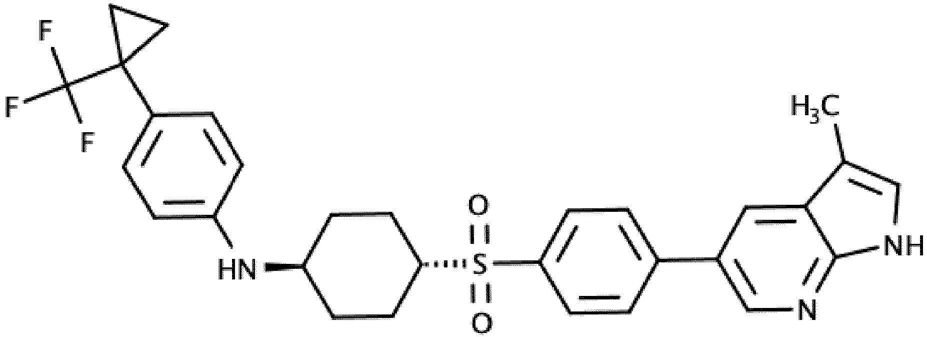

MS(ES⁺) *m*/*z* 554.4 (M+H)⁺

¹H NMR (400 MHz, DMSO) δ 11.55 - 11.43 (d, *J* = 2.5 Hz, 1H), 8.65 - 8.59 (d, *J* = 2.2 Hz, 1H), 8.37 - 8.29 (d, *J=* 2.2 Hz, 1H), 8.12 - 8.01 (m, 2H), 7.99 - 7.90 (m, 2H), 7.34 - 7.29 (dd, *J* = 2.3, 1.2 Hz, 1H), 7.14 - 7.04 (m, 2H), 6.62 - 6.47 (m, 2H), 5.65 - 5.57 (d, *J* = 8.0 Hz, 1H), 3.31 - 3.27 (m, 1H), 3.21 - 3.10 (dt, *J=* 14.8, 5.5 Hz, 1H), 2.40 - 2.28 (s, 3H), 2.10 - 1.93 (m, 4H), 1.63 - 1.46 (m, 2H), 1.26 - 1.09 (m, 4H), 1.00 - 0.89 (m, 2H).

Building block: **step ii:** 5-bromo-3-methyl-1H-pyrrolo[2,3-b]pyridine.

### Example 22: N-[(trans)-4-(4-{3-methyl-1H-pyrazolo[3,4-b]pyridin-5-yl}benzenesulfonyl)cyclohexyl]-4-[1-(trifluoromethyl)cyclopropyl]aniline.

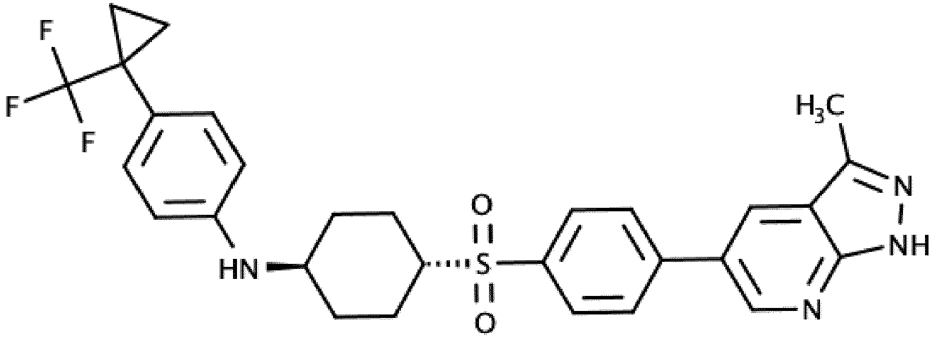

MS(ES⁺) *m*/*z* 555.4 (M+H)⁺

¹H NMR (400 MHz, DMSO) δ 13.47 - 13.30 (m, 1H), 8.96 - 8.86 (d, *J* = 2.2 Hz, 1H), 8.72 - 8.60 (d, *J* = 2.2 Hz, 1H), 8.20 - 8.04 (m, 2H), 8.04 - 7.90 (m, 2H), 7.23 - 7.03 (d, *J* = 8.3 Hz, 2H), 6.62 - 6.46 (m, 2H), 5.69 - 5.51 (d, *J=* 8.0 Hz, 1H), 3.39 - 3.35 (m, 1H), 3.24 - 3.09 (m, 1H), 2.61 - 2.55 (s, 3H), 2.09 - 1.94 (m, 4H), 1.63 - 1.46 (m, 2H), 1.27 - 1.09 (m, 4H), 0.99 - 0.91 (s, 2H).

### Example 23: 1-(6-{[1-(4-{2-methoxy-7-azaspiro[3.5]nonan-7-yl}benzenesulfonyl)piperidin-4-yl]amino}pyridin-3-yl)cyclopropane-1-carbonitrile.

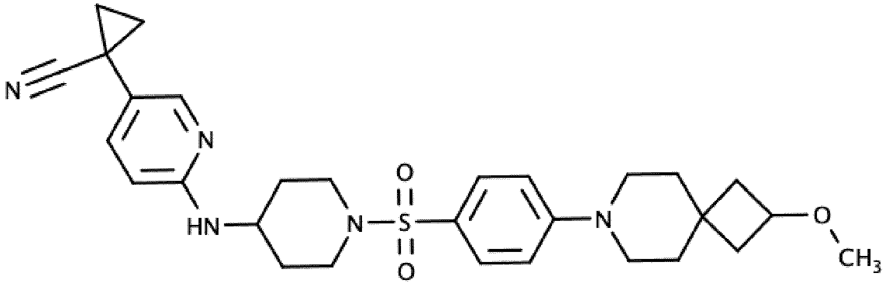

**i)** To a solution of Pd(OAc)₂ (754 mg) en Josiphos SL-J009-1(1.86 g) in THF (250 mL), was added under a nitrogen atmosphere, 1-(6-chloropyridin-3-yl)cyclopropane-1-carbonitrile (6.00 g), NaOt-Bu (4.52 g) and tert-butyl 4-aminopiperidine-1-carboxylate (8.07 g). The reaction mixture was stirred overnight at 100 °C in a sealed vial. After cooling to room temperature, the reaction mixture was poured into water and the product was extracted into ethyl acetate. The combined organic layers were washed with brine, dried over MgSO₄, filtered and concentrated under reduced pressure. The obtained black oil was purified on SiO₂, using 5% to 100% EtOAc in heptane as the eluent to give tert-butyl 4-{[5-(1-cyanocyclopropyl)pyridin-2-yl]amino}piperidine-1-carboxylate (6.52 g) as a white solid.
**ii)** A solution of 5N HCl in 2-propanol (100 mL) was added to a solution of the product obtained in the previous step (6.52 g) in ethyl acetate (130 mL) and the reaction mixture was stirred overnight at room temperature. After completion the reaction mixture was concentrated under reduced pressure to give 1-{6-[(piperidin-4-yl)amino]pyridin-3-yl}cyclopropane-1-carbonitrile hydrochloride (6.3 g) as a light yellow solid, which was used in the next step without further purification.
**iii)** To a solution of the product obtained in the previous step, (1,7 g) and Et₃N (1,3 mL) in ethyl acetate (45 mL) was added 4-fluorobenzene-1-sulfonyl chloride (0.70 g). The reaction mixture was stirred overnight at room temperature. The reaction mixture was quenched by pouring it into water. The product was extracted into CH₂Cl₂ and the combined organic layers were dried over MgSO₄, filtered and concentrated under vacuo to give 1-(6-{[1-(4-fluorobenzenesulfonyl)piperidin-4-yllamino}pyridin-3-yl)cyclopropane-1-carbonitrile (1.0 g) as an off white solid, which was used in the next step without any further purification.
**iv)** In a capped microwave vial, a mixture of the product obtained in the previous step (75 mg), 2-methoxy-7-azaspiro[3.5]nonane hydrochloride (63 mg) and K₂CO (91 mg) in NMP (2 mL) was stirred overnight at 130 °C. After cooling down to r.t., the reaction mixture was poured into water and the product was extracted into ethyl acetate. The combined organic layers were dried over MgSO₄, filtered and concentrated under vacuo and the obtained oil was purified on C18, using 20% to 60% acetonitrile in water as the eluent to give the title compound 1-(6-{[1-(4-{2-methoxy-7-azaspiro[3.5]nonan-7-yl }benzenesulfonyl)piperidin-4-yl]amino}pyridin-3-yl)cyclopropane-1-carbonitrile (42 mg) as a white solid. MS(ES⁺) *m*/*z* 536.4 (M+H)⁺

¹H NMR (400 MHz, DMSO) δ 7.95 - 7.91 (d, J = 2.6 Hz, 1H), 7.53 - 7.45 (d, J = 9.0 Hz, 2H), 7.35 - 7.29 (dd, J = 8.8, 2.6 Hz, 1H), 7.10 - 7.03 (d, J = 9.2 Hz, 2H), 6.67 - 6.60 (d, J = 7.3 Hz, 1H), 6.48 - 6.40 (dd, J = 8.8, 0.8 Hz, 1H), 3.94 - 3.84 (p, J = 6.9 Hz, 1H), 3.69 - 3.55 (td, J = 8.8, 7.9, 5.1 Hz, 1H), 3.50 - 3.40 (m, 2H), 3.30 - 3.23 (m, 2H), 3.15 - 3.10 (s, 3H), 2.46 - 2.37 (m, 2H), 2.22 - 2.13 (ddd, J = 9.9, 7.1, 2.7 Hz, 2H), 1.97 - 1.87 (dd, J = 12.7, 3.9 Hz, 2H), 1.69 - 1.54 (m, 8H), 1.51 - 1.38 (m, 2H), 1.34 - 1.29 (m, 2H).

Following a procedure analogous to that described for **Example 23,** using in **step iv** the appropriate heterocycle, **Examples 24 - 29** have been prepared.

### Examples 24 - 29

**Example 24:** 2-(1-{4-[(4-{[5-(1-cyanocyclopropyl)pyridin-2-yl]amino}piperidin-1-yl)sulfonyl]phenyl}piperidin-4-yl)acetamide.

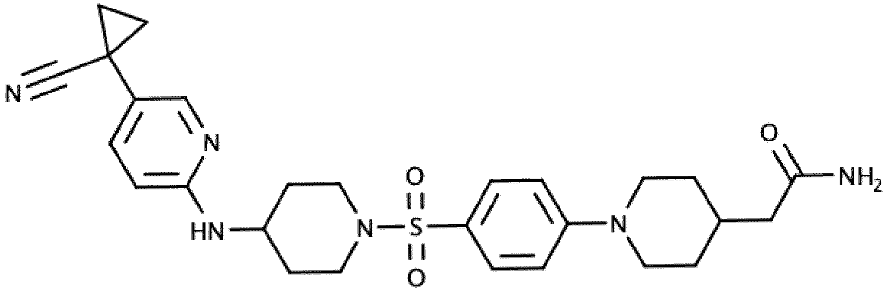

MS(ES⁺) *m*/*z* 523.4 (M+H)⁺

¹H NMR (400 MHz, DMSO) δ 7.95 - 7.91 (d, *J* = 2.7 Hz, 1H), 7.53 - 7.45 (d, *J* = 9.0 Hz, 2H), 7.35 - 7.30 (dd, *J* = 8.8, 2.6 Hz, 1H), 7.30 - 7.25 (s, 1H), 7.08 - 7.03 (d, *J=* 9.2 Hz, 2H), 6.79 - 6.74 (s, 1H), 6.70 - 6.62 (s, 1H), 6.47 - 6.41 (d, *J=* 8.8 Hz, 1H), 3.94 - 3.86 (dt, *J=* 13.0, 3.3 Hz, 2H), 3.68 - 3.56 (m, 1H), 3.50 - 3.40 (m, 2H), 2.90 - 2.79 (td, *J=* 12.7, 2.6 Hz, 2H), 2.46 - 2.37 (td, *J=* 11.7, 11.3, 2.8 Hz, 2H), 2.04 - 1.98 (d, *J =* 7.1 Hz, 2H), 1.97 - 1.86 (m, 3H), 1.78 - 1.69 (d, 2H), 1.60 - 1.55 (m, 2H), 1.50 - 1.39 (m, 2H), 1.34 - 1.28 (m, 2H), 1.27 - 1.14 (m, 2H).

**Building block:** step iv: 2-(piperidin-4-yl)acetamide.

### Example 25: [(1-{4-[(4-{[5-(1-cyanocyclopropyl)pyridin-2-yl]amino}piperidin-1-yl)sulfonyl]phenyl}piperidin-4-yl)methyl]urea.

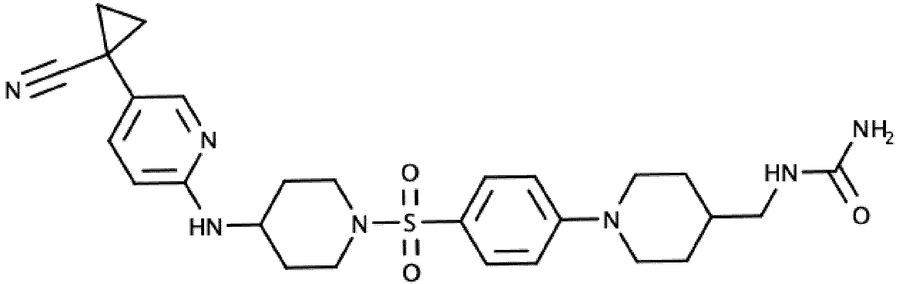

MS(ES⁺) *m*/*z* 538.4 (M+H)⁺

¹H NMR (400 MHz, DMSO) δ 7.94 - 7.92 (d, *J=* 2.5 Hz, 1H), 7.52 - 7.44 (d, *J* = 9.1 Hz, 2H), 7.37 - 7.27 (dd, *J* = 8.7, 2.6 Hz, 1H), 7.09 - 7.01 (d, *J=* 9.2 Hz, 2H), 6.66 - 6.59 (d, *J* = 7.3 Hz, 1H), 6.47 - 6.40 (d, *J* = 8.8 Hz, 1H), 6.07 - 5.99 (t, *J* = 5.9 Hz, 1H), 5.40 - 5.32 (s, 2H), 3.99 - 3.88 (dd, *J* = 9.8, 3.5 Hz, 2H), 3.69 - 3.56 (m, 1H), 3.51 - 3.39 (m, 2H), 2.94 - 2.87 (t, *J=* 6.3 Hz, 2H), 2.86 - 2.75 (m, 2H), 2.47 - 2.37 (m, 2H), 2.00 - 1.85 (m, 2H), 1.78 - 1.66 (d, *J=* 12.7 Hz, 2H), 1.62 - 1.53 (m, 2H), 1.51 - 1.37 (m, 2H), 1.35 - 1.26 (m, 2H), 1.24 - 1.08 (m, 2H).

**Building block:** step iv: [(piperidin-4-yl)methyl]urea.

### Example 26: 1-(6-{[1-(4-{8,10-dioxo-3,9-diazaspiro[5.5]undecan-3-yl}benzenesulfonyl)piperidin-4-yl]amino}pyridin-3-yl)cyclopropane-1-carbonitrile.

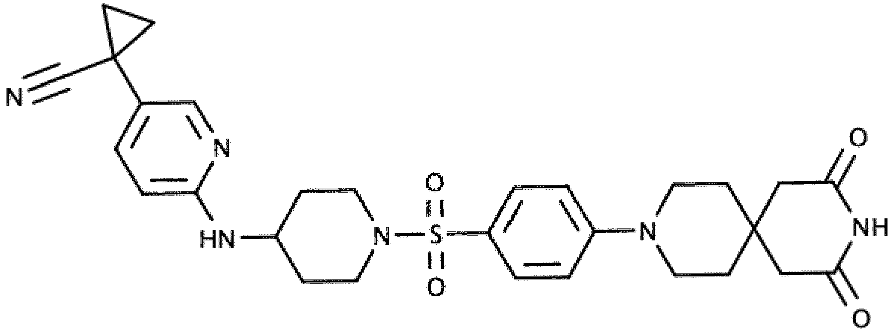

MS(ES⁺) *m*/*z* 563.5 (M+H)⁺

¹H NMR (400 MHz, DMSO) δ 10.84 - 10.70 (s, 1H), 7.94 - 7.91 (d, *J* = 2.6 Hz, 1H), 7.53 - 7.45 (d, *J* = 9.0 Hz, 2H), 7.34 - 7.28 (dd, *J =* 8.8, 2.6 Hz, 1H), 7.09 - 7.01 (d, *J* = 9.1 Hz, 2H), 6.65 - 6.60 (d, *J* = 7.2 Hz, 1H), 6.46 - 6.40 (d, *J* = 8.8 Hz, 1H), 3.67 - 3.55 (m, 1H), 3.48 - 3.41 (m, 2H), 3.41 - 3.35 (m, 4H), 2.60 - 2.54 (s, 4H), 2.47 - 2.37 (td, *J* = 11.7, 2.8 Hz, 2H), 1.95 - 1.87 (m, 2H), 1.59 - 1.50 (m, 6H), 1.50 - 1.39 (m, 2H), 1.34 - 1.28 (m, 2H).

**Building block:** step iv: 3,9-diazaspiro[5.5]undecane-2,4-dione.

### Example 27: 1-(6-{[1-(4-{2-oxo-3-oxa-1,8-diazaspiro[4.5]decan-8-yl}benzenesulfonyl)piperidin-4-yl]amino}pyridin-3-yl)cyclopropane-1-carbonitrile.

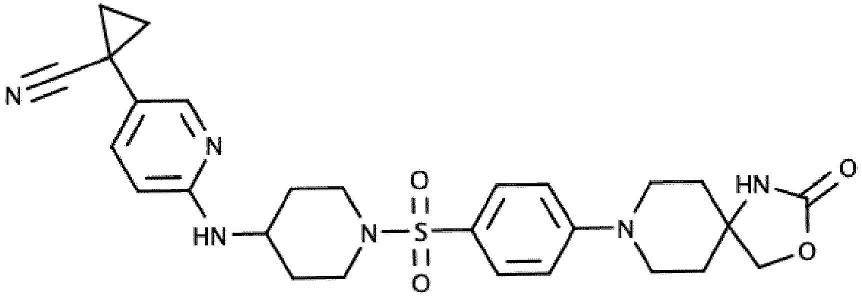

MS(ES⁺) *m*/*z* 537.5 (M+H)⁺

¹H NMR (400 MHz, DMSO) δ 8.15 - 8.11 (s, 1H), 7.93 - 7.92 (d, *J* = 2.4 Hz, 1H), 7.54 - 7.46 (d, *J=* 9.0 Hz, 2H), 7.34 - 7.29 (dd, *J =* 8.7, 2.6 Hz, 1H), 7.13 - 7.05 (d, *J= 9.1* Hz, 2H), 6.66 - 6.60 (d, *J=* 7.3 Hz, 1H), 6.45 - 6.41 (d, *J =* 8.7 Hz, 1H), 4.14 - 4.10 (s, 2H), 3.67 - 3.56 (m, 1H), 3.53 - 3.37 (m, 6H), 2.45 - 2.37 (td, *J=* 11.4, 2.7 Hz, 2H), 1.96 - 1.88 (dd, *J* = 13.2, 3.7 Hz, 2H), 1.80 - 1.65 (m, 4H), 1.60 - 1.54 (m, 2H), 1.50 - 1.39 (m, 2H), 1.33 - 1.28 (m, 2H).

**Building block:** step iv: 3-oxa-1,8-diazaspiro[4.5]decan-2-one.

### Example 28: 1-(6-{[1-(4-{1-oxo-2,8-diazaspiro[4.5]decan-8-yl}benzenesulfonyl)piperidin-4-yl]amino}pyridin-3-yl)cyclopropane-1-carbonitrile.

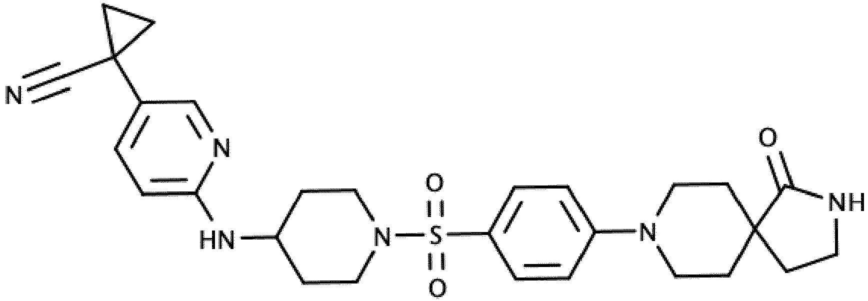

MS(ES⁺) *m*/*z* 535.5 (M+H)⁺

¹H NMR (400 MHz, DMSO) δ 7.94 - 7.92 (d, *J=* 2.5 Hz, 1H), 7.64 - 7.59 (s, 1H), 7.54 - 7.47 (d, *J* = 9.0 Hz, 2H), 7.34 - 7.28 (dd, *J =* 8.8, 2.6 Hz, 1H), 7.12 - 7.03 (d, *J =* 9.1 Hz, 2H), 6.65 - 6.60 (d, *J=* 7.3 Hz, 1H), 6.47 - 6.42 (d, *J=* 8.8 Hz, 1H), 3.90 - 3.81 (dt, *J=* 13.3, 4.0 Hz, 2H), 3.70 - 3.56 (m, 1H), 3.49 - 3.40 (m, 2H), 3.24 - 3.17 (t, *J* = 6.8 Hz, 2H), 3.09 - 2.99 (m, 2H), 2.48 - 2.39 (m, 2H), 2.06 - 2.00 (t, *J=* 6.8 Hz, 2H), 1.97 - 1.87 (m, 2H), 1.77 - 1.67 (td, *J =* 12.7, 4.1 Hz, 2H), 1.59 - 1.54 (m, 2H), 1.52 - 1.39 (dd, *J =* 13.1, 3.7 Hz, 4H), 1.34 - 1.28 (m, 2H).

**Building block:** step iv: 2,8-diazaspiro[4.5]decan-1-one.

### Example 29: 1-(6-{[1-(4-{2-oxa-7-azaspiro[3.5]nonan-7-yl}benzenesulfonyl)piperidin-4-yl]amino}pyridin-3-yl)cyclopropane-1-carbonitrile.

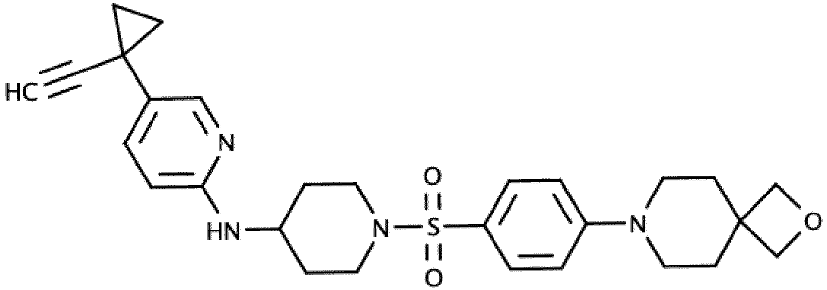

MS(ES⁺) *m*/*z* 508.5 (M+H)⁺

¹H NMR (400 MHz, DMSO) δ 7.95 - 7.90 (d, *J* = 2.6 Hz, 1H), 7.54 - 7.46 (d, *J* = 9.0 Hz, 2H), 7.34 - 7.28 (dd, *J* = 8.7, 2.6 Hz, 1H), 7.12 - 7.04 (d, *J* = 9.1 Hz, 2H), 6.66 - 6.60 (d, *J=* 7.3 Hz, 1H), 6.46 - 6.40 (d, *J=* 8.7 Hz, 1H), 4.39 - 4.32 (s, 4H), 3.66 - 3.55 (m, 1H), 3.49 - 3.40 (m, 2H), 3.31 - 3.26 (d, *J* = 5.4 Hz, 3H), 2.46 - 2.36 (td, *J* = 11.6, 2.8 Hz, 2H), 1.96 - 1.88 (m, 2H), 1.88 - 1.81 (m, 4H), 1.60 - 1.53 (m, 2H), 1.50 - 1.37 (m, 2H), 1.33 - 1.27 (m, 3H)

**Building block:** step iv: 2-oxa-7-azaspiro[3.5]nonane.

### Example 30: 1-(6-{[(trans)-4-{4-[3-(propan-2-yl)-[1,2,4]triazolo[4,3-a]pyridin-6-yl]benzenesulfonyl}cyclohexyl]amino}pyridin-3-yl)cyclopropane-1-carbonitrile.

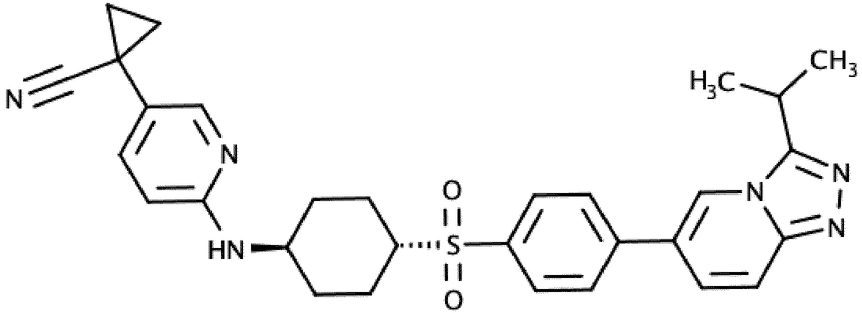

**i)** To a solution of tert-butyl N-[(cis)-4-hydroxycyclohexyl]carbamate (25 g) en triethyl amine (21 mL) in CH₂Cl₂ (100 mL), was added at 0 °C, a solution of methanesulfonyl chloride (12 mL) in CH₂Cl₂. The reaction mixture was stirred overnight at room temperature and after completion was poured into a saturated NaHCO₃ solution in water. The product was extracted into ethyl acetate and the combined organic layers were washed with brine, dried over MgSO₄, filtered and concentrated under reduced pressure to give tert-butyl N-[(*cis*)-4-(methanesulfonyloxy)cyclohexyl]carbamate (36.3 g) as a white solid, which was used in the next step without further purification.
**ii)** To a suspension of the product obtained in the previous step (34.1 g) and Cs₂CO₃ (94.6 mL) in acetone (550 mL) was added 4-bromothiophenol (39.5 g). The reaction mixture was stirred overnight at 60 °C. After removal the organic solvent under reduced pressure, the resulting suspension diluted with water and the product was extracted into ethyl acetate. The combined organic layers were washed with brine, dried over MgSO₄, filtered and concentrated under vacuo to give tert-butyl N-[(*trans*)-4-[(4-bromophenyl)sulfanyl]cyclohexyl]carbamate (25.3 g) as an off white solid, which was used in the next step without any further purification.
**iii)** To a solution of the product obtained in the previous step (25.3 g) in ethyl acetate (250 mL) was added at 0 °C, portion wise mCPBA (56.5 g), and the reaction mixture was stirred overnight at room temperature. After completion, the reaction mixture was washed with an aqueous 1N NaOH solution, water, brine, dried over MgSO₄, filtered concentrated under reduced pressure to give tert-butyl *N-[(trans)-4-(4-*bromobenzenesulfonyl)cyclohexyl]carbamate (23 g) as a white solid, which was used in the next step without further purification.
**iv)** To a suspension of the product obtained in the previous step (2.0 g), bis(pinacolato)diboron (1.73 g) and potassium acetate (1.11 g) in cyclopentyl methyl ether (15 mL), purged with N₂ gas, was added PdCl₂(dppf).CH₂Cl₂ (166 mg). The reaction mixture was heated overnight at 100 °C in a microwave. After cooling to room temperature, the reaction mixture was poured into water and the product was extracted into CH₂Cl₂. The organic layer was washed with brine, dried over MgSO₄ and concentrated under reduced pressure giving tert-butyl N-[(*trans*)-4-[4-(4,4,5,5-tetramethyl-1,3,2-dioxaborolan-2-yl)benzenesulfonyl]cyclohexyl]carbamate (2 g) as a black solid, which was used in the next step without further purification
**v)** Under a nitrogen atmosphere, Pd(PPh₃)₄ (248 mg) was added to a suspension of the product obtained in the previous step (2 g), 6-bromo-3-(propan-2-yl)-[1,2,4]triazolo[4,3-a]pyridine (1.43 g), and NaHCO₃ (1.44 g) in a mixture of 1,4-dioxane (40 mL) and water (10 mL). The reaction mixture was stirred over the weekend at 95 °C. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 10% to 100% CH₃CN in water as the eluent, to give tert-butyl N-[(*trans*)-4-{4-[3-(propan-2-yl)-[1,2,4]triazolo[4,3-a]pyridin-6-yl]benzenesulfonyl}cyclohexyl]carbamate (2.2 g) as a white solid.
**vi)** A solution of 5N HCl in 2-propanol (4.8 mL) was added to a solution of the product obtained in the previous step (1.2 g) in ethyl acetate (10 mL) and the reaction mixture was stirred overnight at room temperature. After completion the reaction mixture was concentrate d under reduced pressure to give (*trans*)-4-{4-[3-(propan-2-yl)-[1,2 4]triazolo[4,3-a]pyridin-6-yl]benzenesulfonyl}cyclohexan-1-amine (1.1 g) as a brown solid, which was used in the next step without further purification.
**vii)** To a solution of Pd(OAc)₂ (38 mg) en Josiphos SL-J009-1 (93 mg) in THF (5 mL), was added under a nitrogen atmosphere, 1-(6-chloro-3-pyridyl)cyclopropanecarbonitrile (150 mg), NaOt-Bu (202 mg) and the product obtained in the previous step (365 mg). The reaction mixture was stirred overnight at 100 °C in a sealed vial. After cooling to room temperature, the reaction mixture was poured into water and the product was extracted into ethyl acetate. The combined organic layers were washed with brine, dried over MgSO₄, filtered and concentrated under reduced pressure. The obtained black oil was purified on SiO₂, using 5% to 100% CH₃OH in CH₂Cl₂ as the eluent to give the title product 1-(6-{[(*trans*)-4-{4-[3-(propan-2-yl)-[1,2,4]triazolo[4,3-a]pyridin-6-yl]benzenesulfonyl}cyclohexyl]amino}pyridin-3-yl)cyclopropane-1-carbonitrile (31 mg) as a white solid. MS(ES⁺) *m*/*z* 541.5 (M+H)⁺

¹H NMR (400 MHz, DMSO) δ 8.96 - 8.91 (t, *J=* 1.4 Hz, 1H), 8.19 - 8.10 (m, 2H), 8.05 - 7.97 (m, 2H), 7.96 - 7.92 (m, 1H), 7.92 - 7.89 (d, *J* = 2.4 Hz, 1H), 7.89 - 7.84 (m, 1H), 7.78 - 7.72 (d, *J* = 9.1 Hz, 1H), 6.94 - 6.85 (d, *J=* 9.2 Hz, 1H), 3.82 - 3.71 (dt, *J=* 13.6, 6.8 Hz, 1H), 3.66 - 3.52 (m, 1H), 3.50 - 3.39 (m, 1H), 2.07 - 2.00 (m, 4H), 1.70 - 1.64 (m, 2H), 1.57 - 1.47 (m, 2H), 1.46 - 1.44 (s, 3H), 1.44 - 1.44 (m, 2H), 1.44 - 1.42 (s, 3H), 1.37 - 1.24 (m, 2H).

### Example 31: 1-{6-[(1-{4-[3-(propan-2-yl)-[1,2,4]triazolo[4,3-a]pyridin-6-yl]benzenesulfonyl}piperidin-4-yl)amino]pyridin-3-yl}cyclopropane-1-carbonitrile.

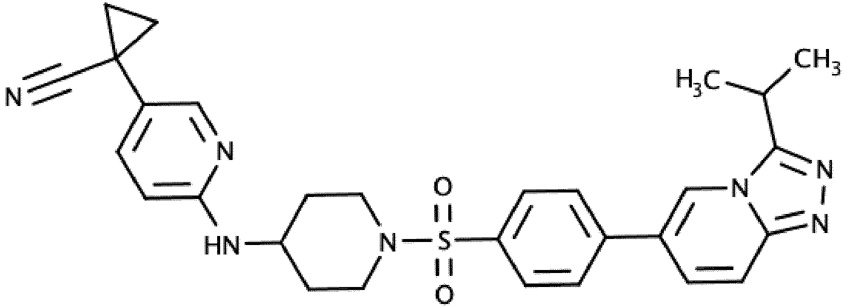

**i)** To a solution of 1-{6-[(piperidin-4-yl)amino]pyridin-3-yl}cyclopropane-1-carbonitrile hydrochloride **(example 23, step ii,** 1.82 g) and DiPEA (4.45 mL) in CH₂Cl₂ (30 mL) was added 4-bromobenzene-1-sulfonyl chloride (1.96 g). The reaction mixture was stirred overnight at room temperature. After completion, the reaction mixture was concentrated under reduced pressure and the residue was dissolved in ethyl acetate, washed with water, dried over MgSO₄, filtered and concentrated under vacuo. The obtained brown solid was purified on SiO₂, using 0% to 30% ethyl acetate in heptane as the eluent to give 1-(6-{[1-(4-bromobenzenesulfonyl)piperidin-4-yl]amino}pyridin-3-yl)cyclopropane-1-carbonitrile (1.4 g) as a white solid.
**ii)** Under a nitrogen atmosphere, PdCl₂(dppf).CH₂Cl₂ (78 mg) was added to a suspension of the product obtained in the previous step (900 mg), bis(pinacolato)diboron (728 mg) and potassium acetate (469 mg) in cyclo pentyl methyl ether (15 mL). The reaction mixture was stirred for 3 hours at 110 °C. After cooling to room temperature, the reaction mixture was poured into water and the product was extracted into ethyl acetate. The organic layer was washed with brine, dried over MgSO₄ and concentrated under reduced pressure giving 1-[6-({1-[4-(4,4,5,5-tetramethyl-1,3,2-dioxaborolan-2-yl)benzenesulfonyl]piperidin-4-yl}amino)pyridin-3-yl]cyclopropane-1-carbonitrile (937 mg) as a brown solid, which was used in the next step without further purification.
**iii)** Under a nitrogen atmosphere, Pd(PPh₃)₄ (34 mg) was added to a suspension of the product obtained in the previous step (150 mg), 6-bromo-3-(propan-2-yl)-[1,2,4]triazolo[4,3-a]pyridine (90 mg), and NaHCO₃ (74 mg) in a mixture of 1,4-dioxane (10 mL) and water (4 mL). The reaction mixture was stirred overnight at 110 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 0% to 100% CH₃CN in water as the eluent, to give the title compound 1-{6-[(1-{4-[3-(propan-2-yl)-[1,2.4]triazolo[4,3-alpyridin-6-yl]benzenesulfonyl}piperidin-4-yl)amino]pyridin-3-yl}cyclopropane-1-carbonitrile (34 mg) as a white solid. MS(ES⁺) *m*/*z* 542.4 (M+H)⁺.

¹H NMR (400 MHz, DMSO) δ 8.89 - 8.82 (m, 1H), 8.16 - 8.05 (m, 2H), 7.96 - 7.92 (dd, *J* = 2.6, 0.8 Hz, 1H), 7.91 - 7.85 (m, 3H), 7.80 - 7.75 (m, 1H), 7.36 - 7.29 (dd, *J* = 8.7, 2.6 Hz, 1H), 6.68 - 6.65 (d, *J=* 7.3 Hz, 1H), 6.46 - 6.42 (dd, *J=* 8.7, 0.8 Hz, 1H), 3.80 - 3.71 (dt, *J=* 13.9, 6.9 Hz, 1H), 3.70 - 3.64 (m, 1H), 3.62 - 3.53 (m, 2H), 2.64 - 2.55 (m, 2H), 2.00 - 1.91 (dd, *J=* 13.2, 3.9 Hz, 2H), 1.61 - 1.54 (m, 2H), 1.51 - 1.46 (m, 2H), 1.46 - 1.43 (s, 3H), 1.43 - 1.40 (s, 3H), 1.34 - 1.28 (m, 2H).

Following a procedure analogous to that described for **Example 31,** using in **step iii** the appropriate (hetero)aryl halide, **Examples 32 - 34** have been prepared.

### Example 32: 1-{6-[(1-{4-[8-methyl-3-(propan-2-yl)-[1,2,4]triazolo[4,3-a]pyridin-6-yl]benzenesulfonyl}piperidin-4-yl)amino]pyridin-3-yl}cyclopropane-1-carbonitrile.

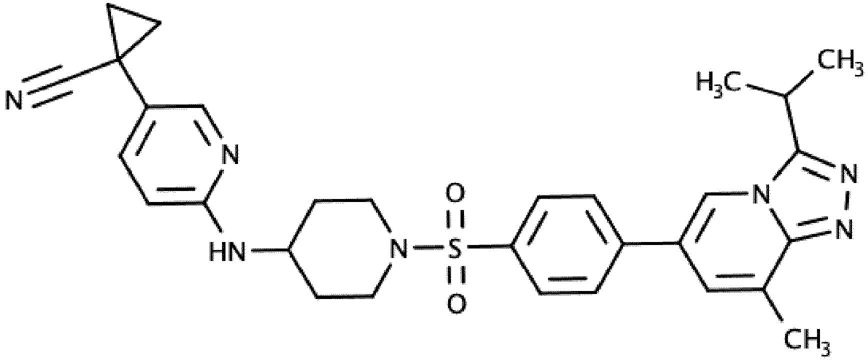

MS(ES⁺) *m*/*z* 556.4 (M+H)⁺

¹H NMR (400 MHz, DMSO) δ 8.73 - 8.69 (t, *J* = 1.1 Hz, 1H), 8.12 - 8.04 (m, 2H), 7.96 - 7.90 (dd, *J* = 2.6, 0.7 Hz, 1H), 7.90 - 7.84 (m, 2H), 7.63 - 7.57 (t, *J* = 1.4 Hz, 1H), 7.37 - 7.30 (dd, *J* = 8.7, 2.6 Hz, 1H), 6.70 - 6.62 (d, *J=* 7.3 Hz, 1H), 6.48 - 6.40 (dd, *J=* 8.7, 0.8 Hz, 1H), 3.78 - 3.68 (p, *J* = 6.7 Hz, 1H), 3.68 - 3.63 (m, 1H), 3.63 - 3.53 (m, 2H), 2.64 - 2.59 (m, 3H), 2.59 - 2.53 (m, 2H), 2.02 - 1.89 (m, 2H), 1.60 - 1.54 (m, 2H), 1.54 - 1.46 (m, 2H), 1.46 - 1.43 (s, 3H), 1.43 - 1.40 (s, 3H), 1.33 - 1.28 (m, 2H).

**Building block:** step iv: 6-bromo-8-methyl-3-(propan-2-yl)-[1,2,4]triazolo[4,3-a]pyridine.

### Example 33: 1-(6-{[1-(4-{imidazo[1,2-a]pyridin-6-yl}benzenesulfonyl)piperidin-4-yl]amino}pyridin-3-yl)cyclopropane-1-carbonitrile.

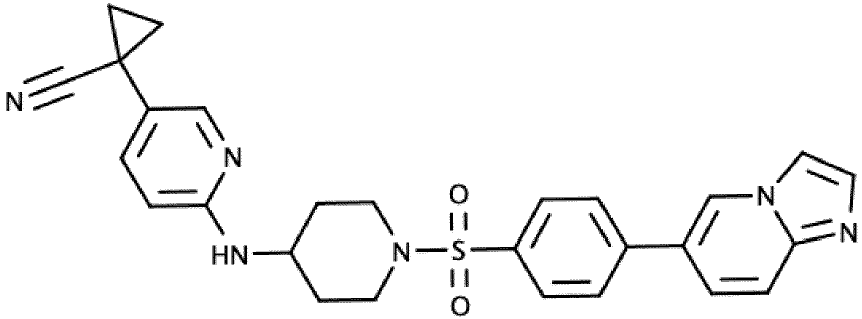

MS(ES⁺) *m*/*z* 499.4 (M+H)⁺

¹H NMR (400 MHz, DMSO) δ 9.12 - 9.08 (dd, *J* = 2.0, 1.0 Hz, 1H), 8.04 - 7.98 (m, 3H), 7.94 - 7.92 (m, 1H), 7.89 - 7.84 (m, 2H), 7.74 - 7.70 (m, 1H), 7.70 - 7.65 (m, 2H), 7.35 - 7.29 (dd, *J* = 8.7, 2.6 Hz, 1H), 6.70 - 6.63 (d, *J =* 7.4 Hz, 1H), 6.48 - 6.40 (dd, *J =* 8.8, 0.8 Hz, 1H), 3.75 - 3.64 (m, 1H), 3.63 - 3.52 (m, 2H), 2.65 - 2.56 (m, 2H), 2.01 - 1.90 (m, 2H), 1.62 - 1.56 (m, 2H), 1.56 - 1.43 (m, 2H), 1.35 - 1.28 (m, 2H).

**Building block:** step iv: 6-bromoimidazo[1,2-a]pyridine.

### Example 34: 1-{6-[(1-{4-[3-(propan-2-yl)imidazo[1,2-a]pyridin-6-yl]benzenesulfonyl}piperidin-4-yl)amino]pyridin-3-yl}cyclopropane-1-carbonitrile.

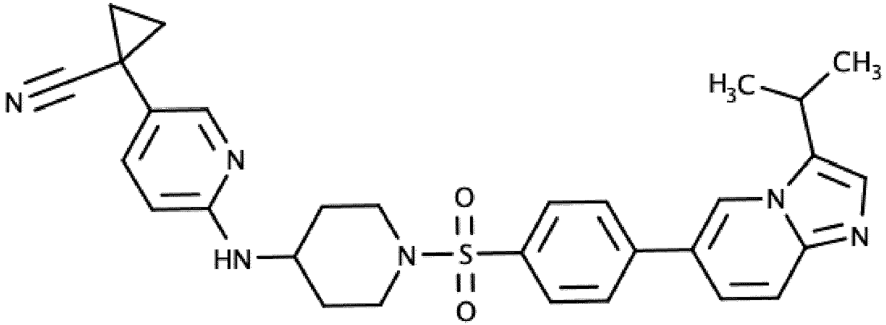

MS(ES⁺) *m*/*z* 541.4 (M+H)⁺

¹H NMR (400 MHz, DMSO) δ 8.76 - 8.70 (d, *J=* 1.7 Hz, 1H), 8.12 - 8.05 (m, 2H), 7.96 - 7.90 (d, *J=* 2.5 Hz, 1H), 7.90 - 7.83 (m, 2H), 7.74 - 7.66 (m, 1H), 7.67 - 7.59 (m, 1H), 7.51 7.42 (s, 1H), 7.37 - 7.29 (dd, *J* = 8.8, 2.6 Hz, 1H), 6.72 - 6.62 (d, *J* = 7.3 Hz, 1H), 6.50 - 6.40 (d, *J=* 8.8 Hz, 1H), 3.75 - 3.64 (m, 1H), 3.64 - 3.56 (m, 2H), 3.56 - 3.47 (m, 1H), 2.64 - 2.54 (m, 2H), 2.02 - 1.91 (m, 2H), 1.61 - 1.55 (m, 2H), 1.54 - 1.43 (m, 2H), 1.40 - 1.37 (s, 3H), 1.37 - 1.34 (s, 3H), 1.34 - 1.28 (m, 2H).

**Building block:** step iv: 6-bromo-3-(propan-2-yl)imidazo[1,2-a]pyridine.

### Example 35: 1-(6-{[1-(4-{3-oxo-2H,3H-[1,2,4]triazolo[4,3-a]pyridin-6-yl}benzenesulfonyl)piperidin-4-yl]amino}pyridin-3-yl)cyclopropane-1-carbonitrile.

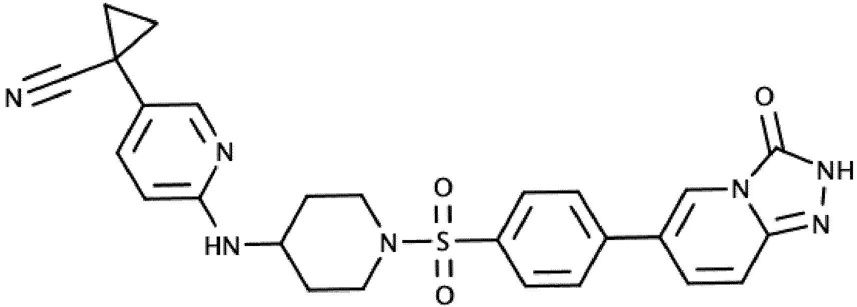

**i)** Following a procedure analogous to that described for **Example 31,** using in **step iii** 6-bromo-2-[(4-methoxyphenyl)methyl]-2H,3H-[1,2,4]triazolo[4,3-a]pyridin-3-one (109 mg), 1-(6-{[1-(4-{2-[(4-methoxyphenyl)methyl]-3-oxo-2H,3H-[1,2,4]triazolo[4,3-a]pyridin-6-yl}benzenesulfonyl)piperidin-4-yl]amino}pyridin-3-yl)cyclopropane-1-carbonitrile (71 mg) has been prepared.
**ii)** To a solution of the product obtained in the previous step (71 mg) in TFA (5 mL) was added L-cysteïne (20 g). The reaction mixture was stirred overnight at 70 °C. After completion, the reaction mixture was concentrated under reduced pressure. Tiihe residue was dissolved in ethyl acetate, washed with water, dried over MgSO₄, filtered and concentrated under vacuo. The obtained brown solid was purified on C18, using 10% to 100% CH₃CN in H₂O as the eluent to give the title product 1-(6-1[1-(4-13-oxo-2H,3H-[1,2,4]triazolo[4,3-ali)yridin-6-yl}benzenesulfonyl)piperidin-4-yl]amino}pyridin-3-yl)cyclopropane-1-carbonitrile (28 mg) as a white solid. MS(ES⁺) *m*/*z* 516.4 (M+H)⁺.

¹H NMR (400 MHz, DMSO) δ 12.74 - 12.50 (s, 1H), 8.32 - 8.16 (t, *J* = 1.5 Hz, 1H), 8.07 - 7.98 (m, 2H), 7.96 - 7.90 (d, *J* = 2.5 Hz, 1H), 7.86 - 7.78 (m, 2H), 7.70 - 7.64 (dd, *J=* 9.9, 1.8 Hz, 1H), 7.43 - 7.36 (dd, *J=* 9.9, 1.0 Hz, 1H), 7.36 - 7.29 (dd, *J=* 8.7, 2.6 Hz, 1H), 6.69 - 6.62 (d, *J=* 7.4 Hz, 1H), 6.47 - 6.40 (m, 1H), 3.75 - 3.62 (m, 1H), 3.62 - 3.49 (m, 2H), 2.64 - 2.54 (m, 2H), 2.01 - 1.88 (m, 2H), 1.61 - 1.54 (m, 2H), 1.54 - 1.42 (m, 2H), 1.34 - 1.27 (m, 2H).

### Example 36: N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine.

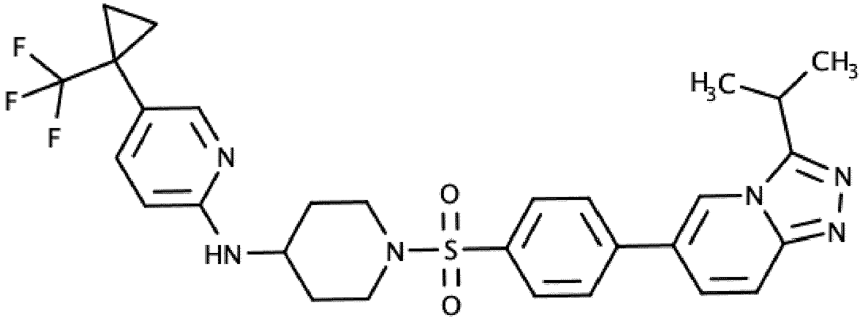

**i)** To a solution of Pd(OAc)₂ (99 mg) en Josiphos SL-J009-1 (238 mg) in THF (20 mL), was added under a nitrogen atmosphere, 2-chloro-5-[1-(trifluoromethyl)cyclopropyl]pyridine (850 mg), NaOt-Bu (579 mg) and tert-butyl 4-aminopiperidine-1-carboxylate (860 mg). The reaction mixture was stirred overnight at 100 °C in a sealed vial. After cooling to room temperature, the reaction mixture was poured into water and the product was extracted into ethyl acetate. The combined organic layers were washed with brine, dried over MgSO₄, filtered and concentrated under reduced pressure. The obtained brown oil was purified on SiO₂, using 0% to 70% EtOAc in heptane as the eluent to give tert-butyl 4-({5-[1-(trifluoromethyl)cyclopropyl]pyridin-2-yl}amino)piperidine-1-carboxylate (993 mg) as a white solid.
**ii)** A solution of 5N HCl in 2-propanol (10 mL) was added to a solution of the product obtained in the previous step (993 mg) in ethyl acetate (20 mL) and the reaction mixture was stirred overnight at room temperature. After completion the reaction mixture was concentrated under reduced pressure to give N-(piperidin-4-yl)-5-[1-(trifluoromethyl)cyclopropyl]pyridin-2-amine hydrochloride (925 mg) as a light yellow solid, which was used in the next step without further purification.
**iii)** To a solution of the product obtained in the previous step (925 mg) and triethyl amine (10 mL) in CH₂Cl₂ (30 mL) was added 4-bromobenzene-1-sulfonyl chloride (802 mg). The reaction mixture was stirred overnight at room temperature. After completion, the reaction mixture was concentrated under reduced pressure and the residue was dissolved in ethyl acetate, washed with water, dried over MgSO₄, filtered, and concentrated under vacuo. The obtained brown solid was purified on SiO₂, using 0% to 40% ethyl acetate in heptane as the eluent to give N-[1-(4-bromobenzenesulfonyl)piperidin-4-yl]-5-[1-(trifluoromethyl)cyclopropyl]pyridin-2-amine (1.21 g) as a white solid.
**iv)** Under a nitrogen atmosphere, PdCl₂(dppf).CH₂Cl₂ (36 mg) was added to a suspension of the product obtained in the previous step (500 mg), bis(pinacolato)diboron (377 mg) and potassium acetate (291 mg) in 1,4-dioxane (10 mL). The reaction mixture was stirred overnight at 110 °C. After cooling to room temperature, the reaction mixture was poured into water and the product was extracted into ethyl acetate. The organic layer was washed with brine, dried over MgSO₄ and concentrated under reduced pressure giving N-{1-[4-(4,4,5,5-tetramethyl-1,3,2-dioxaborolan-2-yl)benzenesulfonyl|piperidin-4-yl}-5-[1-(trifluoromethyl)cyclopropyl]pyridin-2-amine (550 mg) as a brown solid, which was used in the next step without further purification.
**v)** Under a nitrogen atmosphere, Pd(PPh₃)₄ (13 mg) was added to a suspension of the product obtained in the previous step (109 mg), 6-bromo-3-(propan-2-yl)-[1,2,4]triazolo[4,3-a]pyridine (73 mg), and NaHCO₃ (90 mg) in a mixture of 1,4-dioxane (4 mL) and water (1 mL). The reaction mixture was stirred overnight at 110 °C in a microwave. After cooling to room temperature, the reaction mixture was diluted with water and ethyl acetate and the product was extracted into ethyl acetate. The combined organic layers were washed with water, brine, dried over MgSO₄, filtered, and concentrated under reduced pressure. The residue was purified on C18, using 30% to 95% CH₃CN in water as the eluent, to give the title compound N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine (60 mg) as a white solid. MS(ES⁺) m/z 585.4 (M+H)⁺.

¹H NMR (400 MHz, DMSO) δ 8.87 - 8.83 (t, *J* = 1.4 Hz, 1H), 8.14 - 8.06 (m, 2H), 7.97 - 7.92 (d, *J=* 2.4 Hz, 1H), 7.91 - 7.85 (m, 3H), 7.80 - 7.75 (m, 1H), 7.41 - 7.35 (dd, *J* = 8.6, 2.4 Hz, 1H), 6.72 - 6.65 (d, *J* = 7.3 Hz, 1H), 6.45 - 6.39 (dd, *J=* 8.7, 0.8 Hz, 1H), 3.79 - 3.71 (m, 1H), 3.71 - 3.65 (m, 1H), 3.62 - 3.53 (m, 2H), 2.63 - 2.55 (m, 2H), 2.00 - 1.92 (m, 2H), 1.56 - 1.46 (m, 2H), 1.45 - 1.44 (s, 3H), 1.44 - 1.40 (s, 3H), 1.26 - 1.20 (m, 2H), 1.01 - 0.95 (m, 2H).

Following a procedure analogous to that described for **Example 36,** using in **step** v the appropriate (hetero)aryl halide, **Examples 37** - **39** have been prepared.

### Example 37: N-[1-(4-{imidazo[1,2-a]pyridin-6-yl}benzenesulfonyl)piperidin-4-yl]-5-[1-(trifluoromethyl)cyclopropyl]pyridin-2-amine.

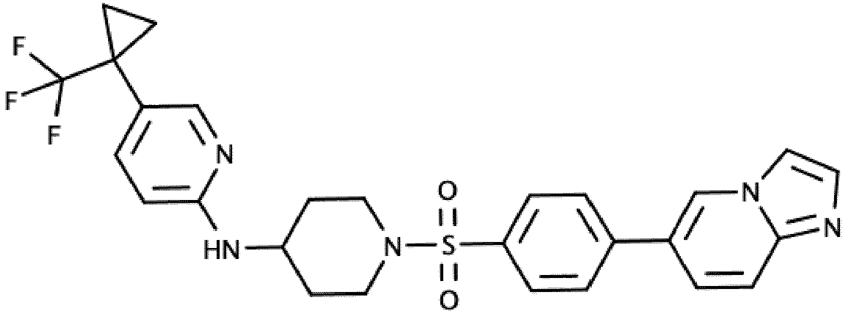

MS(ES⁺) *m*/*z* 542.4 (M+H)⁺

¹H NMR (400 MHz, DMSO) δ 9.12 - 9.08 (dd, *J=* 1.8, 1.1 Hz, 1H), 8.04 - 7.98 (m, 3H), 7.97 - 7.93 (d, *J* = 2.3 Hz, 1H), 7.89 - 7.84 (m, 2H), 7.74 - 7.69 (m, 1H), 7.69 - 7.64 (m, 2H), 7.40 - 7.35 (dd, *J* = 8.6, 2.4 Hz, 1H), 6.71 - 6.64 (d, *J* = 7.3 Hz, 1H), 6.47 - 6.39 (dd, *J* = 8.7, 0.8 Hz, 1H), 3.76 - 3.63 (m, 1H), 3.62 - 3.51 (m, 2H), 2.65 - 2.56 (m, 2H), 2.02 - 1.90 (m, 2H), 1.56 - 1.42 (m, 2H), 1.28 - 1.18 (m, 2H), 1.01 - 0.93 (m, 2H).

**Building block:** step v: 6-bromoimidazo[1,2-a]pyridine.

### Example 38: N-(1-{4-[3-(propan-2-yl)imidazo[1,2-a]pyridin-6-yl]benzenesulfonyl}piperidin-4-yl)-5-[1-(trifluoromethyl)cyclopropyl]pyridin-2-amine.

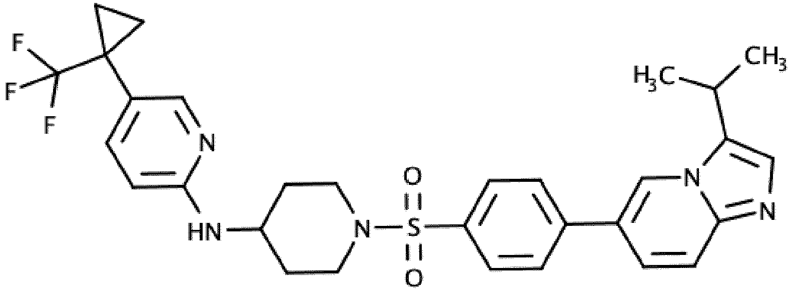

MS(ES⁺) *m*/*z* 584.4 (M+H)⁺

¹H NMR (400 MHz, DMSO) δ 8.74 - 8.70 (dd, *J* = 1.8, 1.0 Hz, 1H), 8.11 - 8.05 (m, 2H), 7.97 - 7.92 (d, *J* = 2.3 Hz, 1H), 7.90 - 7.83 (m, 2H), 7.72 - 7.66 (m, 1H), 7.66 - 7.61 (m, 1H), 7.48 - 7.45 (d, *J* = 0.7 Hz, 1H), 7.42 - 7.34 (dd, *J* = 8.7, 2.4 Hz, 1H), 6.72 - 6.65 (d, *J* = 7.3 Hz, 1H), 6.46 - 6.39 (dd, *J* = 8.7, 0.8 Hz, 1H), 3.76 - 3.63 (m, 1H), 3.62 - 3.55 (m, 2H), 3.55 - 3.46 (m, 1H), 2.64 - 2.54 (m, 2H), 2.01 - 1.91 (m, 2H), 1.57 - 1.43 (m, 2H), 1.26 - 1.21 (m, 2H), 1.04 - 0.95 (m, 2H).

**Building block:** step v: 6-bromo-3-(propan-2-yl)imidazo[1,2-a]pyridine.

### Example 39: N-(1-{4-[3-(propan-2-yl)-[1,2,4]triazolo[4,3-a]pyrazin-6-yl]benzenesulfonyl}piperidin-4-yl)-5-[1-(trifluoromethyl)cyclopropyl]pyridin-2-amine.

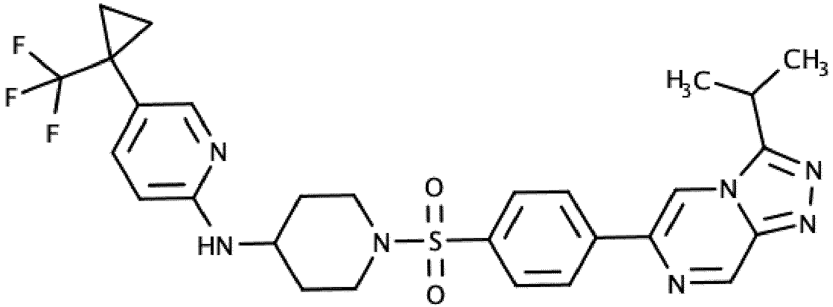

MS(ES⁺) *m*/*z* 586.4 (M+H)⁺

¹H NMR (400 MHz, DMSO) δ 9.58 - 9.47 (d, *J=* 1.5 Hz, 1H), 9.30 - 9.18 (d, *J=* 1.6 Hz, 1H), 8.47 - 8.36 (m, 2H), 7.96 - 7.93 (d, *J* = 2.4 Hz, 1H), 7.93 - 7.88 (m, 2H), 7.40 - 7.34 (dd, *J=* 8.7, 2.5 Hz, 1H), 6.70 - 6.65 (d, *J=* 7.3 Hz, 1H), 6.44 - 6.39 (m, 1H), 3.85 - 3.75 (dt, *J=* 13.7, 6.9 Hz, 1H), 3.75 - 3.63 (m, 1H), 3.62 - 3.52 (m, 2H), 2.66 - 2.57 (m, 2H), 2.00 - 1.90 (m, 2H), 1.55 - 1.49 (m, 2H), 1.48 - 1.46 (s, 2H), 1.46 - 1.45 (s, 3H), 1.26 - 1.20 (m, 2H), 1.01 - 0.95 (m, 2H).

**Building block:** step v: 6-bromo-3-(propan-2-yl)-[1,2,4]triazolo[4,3-a]pyrazine.

Following a procedure analogous to that described for **Example 10,** using in **step ii** the appropriate (hetero)aryl halide, **Example 40 - 43** have been prepared.

### Example 40: 1-{4-[3-(propan-2-yl)-[1,2,4]triazolo[4,3-a]pyridin-6-yl]benzenesulfonyl}-N-{4-[1-(trifluoromethyl)cyclopropyl]phenyl}piperidin-4-amine.

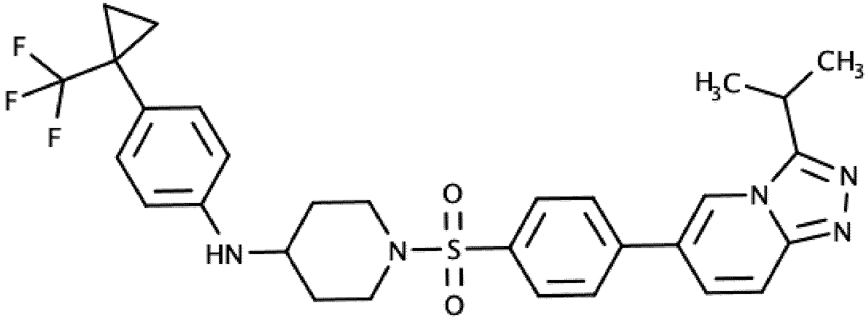

MS(ES⁺) *m*/*z* 584.4 (M+H)⁺

¹H NMR (400 MHz, DMSO) δ 8.88 - 8.82 (t, *J* = 1.4 Hz, 1H), 8.14 - 8.07 (m, 2H), 7.95 - 7.84 (dd, *J* = 8.5, 1.7 Hz, 3H), 7.83 - 7.75 (dd, *J* = 9.6, 1.6 Hz, 1H), 7.16 - 7.05 (m, 2H), 6.57 - 6.45 (m, 2H), 5.70 - 5.63 (d, *J* = 8.0 Hz, 1H), 3.82 - 3.67 (hept, *J* = 6.8 Hz, 1H), 3.67 - 3.56 (m, 2H), 3.31 - 3.22 (m, 1H), 2.62 - 2.54 (m, 2H), 2.02 - 1.91 (m, 2H), 1.46 - 1.44 (s, 3H), 1.44 - 1.42 (s, 3H), 1.42 - 1.36 (m, 2H), 1.23 - 1.17 (m, 2H), 0.99 - 0.90 (m, 2H).

**Building block:** step v: 6-bromo-3-(propan-2-yl)-[1,2,4]triazolo[4,3-a]pyridine.

### Example 41: 1-{4-[3-(propan-2-yl)-[1,2,4]triazolo[4,3-a]pyrazin-6-yl]benzenesulfonyl}-N-{4-[1-(trifluoromethyl)cyclopropyl]phenyl}piperidin-4-amine.

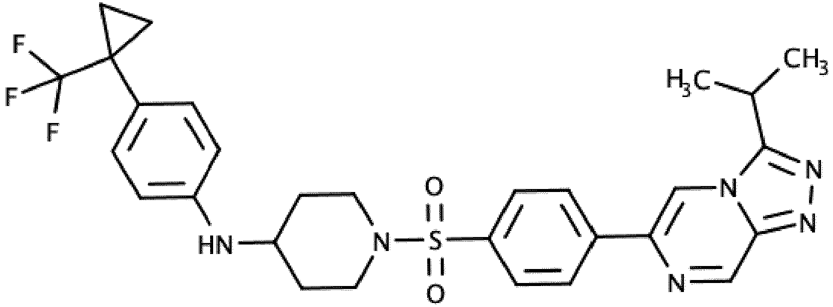

MS(ES⁺) *m*/*z* 585.4 (M+H)⁺

¹H NMR (400 MHz, DMSO) δ 9.57 - 9.49 (d, *J=* 1.5 Hz, 1H), 9.27 - 9.19 (d, *J=* 1.6 Hz, 1H), 8.49 - 8.40 (m, 2H), 7.93 - 7.88 (m, 2H), 7.11 - 7.04 (d, *J* = 8.3 Hz, 2H), 6.56 - 6.47 (m, 2H), 5.73 - 5.63 (d, *J=* 8.0 Hz, 1H), 3.85 - 3.75 (dt, *J=* 13.7, 6.9 Hz, 1H), 3.67 - 3.55 (m, 2H), 3.30 - 3.21 (m, 1H), 2.61 - 2.54 (m, 2H), 1.99 - 1.91 (m, 2H), 1.49 - 1.47 (s, 3H), 1.47 - 1.44 (s, 3H), 1.44 - 1.35 (m, 2H), 1.23 - 1.16 (m, 2H), 0.98 - 0.90 (m, 2H).

**Building block:** step v: 6-bromo-3-(propan-2-yl)-[1,2,4]triazolo[4,3-a]pyrazine.

### Example 42: 1-(4-{3-methyl-[1,2,4]triazolo[4,3-a]pyridin-6-yl}benzenesulfonyl)-N-{4-[1-(trifluoromethyl)cyclopropyl]phenyl}piperidin-4-amine.

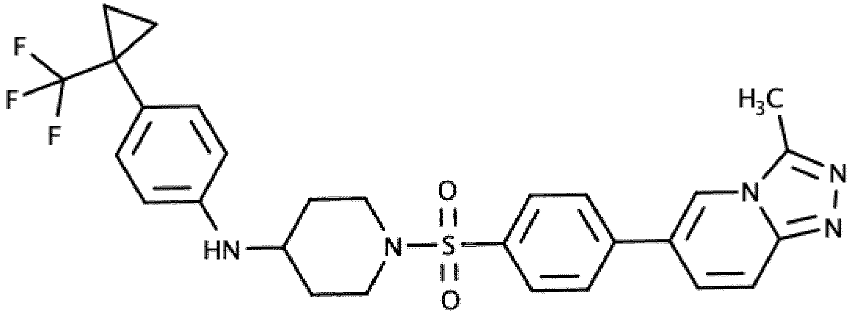

MS(ES⁺) *m*/*z* 556.4 (M+H)⁺

¹H NMR (400 MHz, DMSO) δ 8.83 - 8.78 (t, *J* = 1.3 Hz, 1H), 8.16 - 8.08 (m, 2H), 7.93 - 7.86 (m, 3H), 7.86 - 7.78 (m, 1H), 7.13 - 7.06 (dd, *J* = 6.7, 1.8 Hz, 2H), 6.56 - 6.48 (m, 2H), 5.92 - 5.42 (s, 1H), 3.65 - 3.58 (m, 2H), 3.32 - 3.21 (m, 1H), 2.82 - 2.75 (s, 3H), 2.63 - 2.53 (m, 2H), 2.02 - 1.89 (m, 2H), 1.50 - 1.36 (m, 2H), 1.25 - 1.14 (m, 2H), 1.00 - 0.85 (m, 2H).

**Building block:** step v: 6-bromo-3-methyl-[1,2,4]triazolo[4,3-a]pyridine.

### Example 43: 6-(4-{[4-({4-[1-(trifluoromethyl)cyclopropyl]phenyl}amino)piperidin-1-yl]sulfonyl}phenyl)-2H,3H-[1,2,4]triazolo[4,3-a]pyridin-3-one.

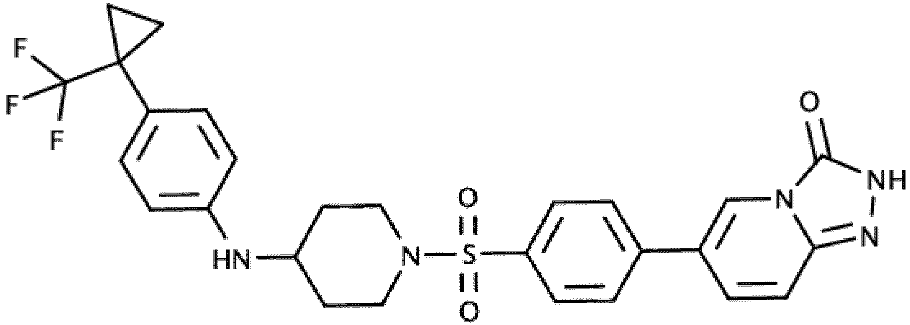

**i)** Following a procedure analogous to that described for **Example 10,** using in **step v** 6-bromo-2-[(4-methoxyphenyl)methyl]-2H,3H-[1,2,4]triazolo[4,3-a]pyridin-3-one (200 mg), 2-[(4-methoxyphenyl)methyl]-6-(4-{[4-({4-[1-(trifluoromethyl)cyclopropyl]phenyl}amino)piperidin-1-yl]sulfonyl}phenyl)-2H,3H-[1,2,4]triazolo[4,3-a]pyridin-3-one (91 mg) has been prepared.
**ii)** To a solution of the product obtained in the previous step (91 mg) in TFA (4 mL) was added L-cysteine (23 mg). The reaction mixture was stirred overnight at 70 °C. After completion, the reaction mixture was concentrated under reduced pressure. The residue was dissolved in ethyl acetate, washed with water, dried over MgSO₄, filtered, and concentrated under vacuo. The obtained brown solid was purified on C18, using 10% to 100% CH₃CN in H₂O as the eluent to give the title product 6-(4-{[4-({4-[1-(trifluoromethyl)cyclopropyl]phenyl}amino)piperidin-1-yl]sulfonyl}phenyl)-2H,3H-[1,2,4]triazolo[4,3-a]pyridin-3-one (42 mg) as a white solid. MS(ES⁺) *m*/*z* 558.4 (M+H)⁺.

¹H NMR (400 MHz, DMSO) δ 12.72 - 12.50 (s, 1H), 8.28 - 8.17 (t, *J* = 1.5 Hz, 1H), 8.08 - 8.00 (m, 2H), 7.86 - 7.77 (m, 2H), 7.71 - 7.63 (dd, *J=* 9.8, 1.8 Hz, 1H), 7.46 - 7.34 (dd, *J=* 9.9, 1.0 Hz, 1H), 7.13 - 7.02 (m, 2H), 6.56 - 6.48 (dd, *J* = 6.9, 1.7 Hz, 2H), 5.71 - 5.62 (d, *J=* 8.1 Hz, 1H), 3.64 - 3.54 (m, 2H), 3.29 - 3.20 (m, 1H), 2.60 - 2.54 (m, 2H), 1.98 - 1.90 (m, 2H), 1.50 - 1.34 (m, 2H), 1.23 - 1.18 (m, 2H), 0.98 - 0.90 (m, 2H).

Following a procedure analogous to that described for **Example 36,** using in **step v** the appropriate (hetero)aryl halide, **Examples 44 - 46** have been prepared.

### Example 44: N-(1-{4-[3-(difluoromethyl)-[1,2,4]triazolo[4,3-a]pyridin-6-yl]benzenesulfonyl}piperidin-4-yl)-5-[1-(trifluoromethyl)cyclopropyl]pyridin-2-amine.

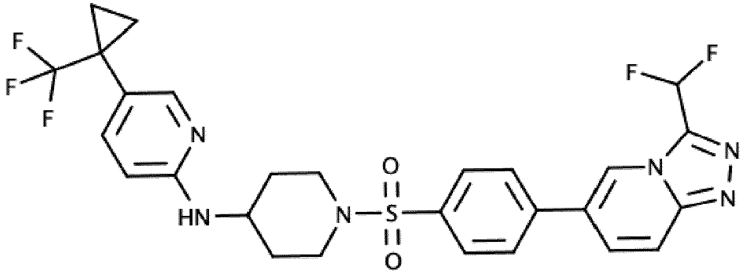

MS(ES⁺) *m*/*z* 593.4 (M+H)⁺

¹H NMR (400 MHz, MeOD-*d*₄) δ = 8.82 (s, 1H), 8.05 - 7.94 (m, 6H), 7.92 (d, *J=* 2.0 Hz, 1H), 7.68 - 7.41 (m, 2H), 6.46 (d, *J=* 8.8 Hz, 1H), 3.78 - 3.71 (m, 2H), 3.70 - 3.63 (m, 1H), 2.66 - 2.61 (m, 2H), 2.08 - 2.04 (m, 2H), 1.63 - 1.52 (m, 2H), 1.30 - 1.25 (m, 2H), 0.96 (s, 2H)

**Building block:** step v: 6-bromo-3-(difluoromethyl)-[1,2,4]triazolo[4,3-a]pyridine.

### Example 45: 4-(4-{[4-({5-[1-(trifluoromethyl)cyclopropyl]pyridin-2-yl}amino)piperidin-1-yl]sulfonyl}phenyl)pyridine-2-carboxamide.

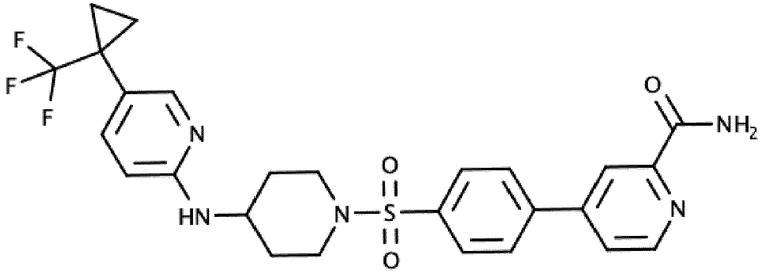

MS(ES⁺) *m*/*z* 546.4 (M+H)⁺

¹H NMR (400 MHz, MeOD-*d*₄) δ = 8.76 (d, *J* = 5.2 Hz, 1H), 8.45 (s, 1H), 8.07 - 8.03 (m, 2H), 7.98 - 7.94 (m, 2H), 7.93 - 7.90 (m, 2H), 7.44 (dd, *J* = 2.4, 8.8 Hz, 1H), 6.45 (d, *J* = 8.8 Hz, 1H), 3.74 - 3.71 (m, 2H), 3.69 - 3.63 (m, 1H), 2.70 - 2.61 (m, 2H), 2.10 - 2.01 (m, 2H), 1.64 - 1.52 (m, 2H), 1.29 - 1.25 (m, 2H), 0.95 (s, 2H).

**Building block:** step v: 4-bromopyridine-2-carboxamide.

### Example 46: N-(1-{4-[8-methyl-3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine.

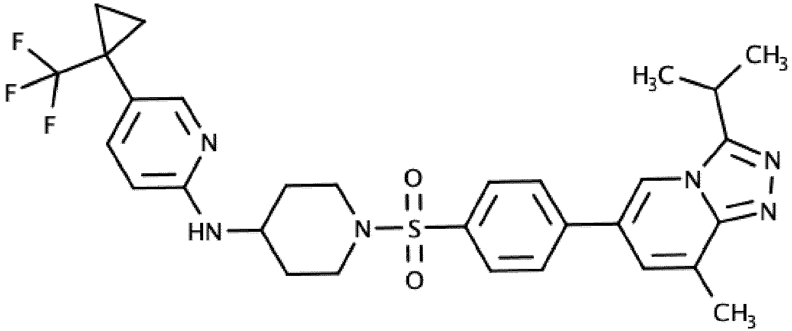

MS(ES⁺) *m*/*z* 599.4 (M+H)⁺

¹H NMR (400 MHz, MeOD-*d*₄) δ = 8.54 (s, 1H), 8.02 - 7.97 (m, 2H), 7.95 - 7.90 (m, 3H), 7.61 (s, 1H), 7.44 (dd, *J* = 2.4, 8.8 Hz, 1H), 6.45 (d, *J=* 8.8 Hz, 1H), 3.78 - 3.71 (m, 2H), 3.67 (d, *J* = 6.4, 13.6 Hz, 2H), 2.69 (s, 3H), 2.67 - 2.58 (m, 2H), 2.11 - 2.02 (m, 2H), 1.64 - 1.55 (m, 2H), 1.53 (d, *J =* 7.2 Hz, 6H), 1.31 - 1.24 (m, 2H), 0.96 (s, 2H).

**Building block:** step v: 6-bromo-8-methyl-3-(propan-2-yl)-[1,2,4]triazolo[4,3-a]pyridine.

### Example 47: N-[1-(4-{3-methyl-[1,2,4]triazolo[4,3-a]pyridin-6-yl}benzenesulfonyl)piperidin-4-yl]-5-[1-(trifluoromethyl)cyclopropyl]pyridin-2-amine.

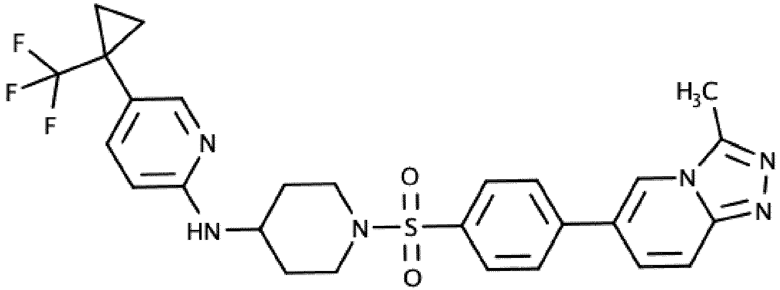

MS(ES⁺) *m*/*z* 557.4 (M+H)⁺

¹H NMR (400 MHz, DMSO-*d*₆) δ = 8.77 (s, 1H), 8.09 (d, *J =* 8.4 Hz, 2H), 7.94 (d, *J =* 2.0 Hz, 1H), 7.91 - 7.83 (m, 3H), 7.81 - 7.73 (m, 1H), 7.37 (dd, *J =* 2.0, 8.4 Hz, 1H), 6.67 (d, *J=* 7.2 Hz, 1H), 6.41 (d, *J =* 8.8 Hz, 1H), 3.73 - 3.62 (m, 1H), 3.61 - 3.52 (m, 2H), 2.77 (s, 3H), 2.62 - 2.53 (m, 2H), 2.00 - 1.91 (m, 2H), 1.55 - 1.43 (m, 2H), 1.28 - 1.18 (m, 2H), 0.97 (s, 2H).

**Building block:** step v: 6-bromo-3-methyl-[1,2,4]triazolo[4,3-a]pyridine.

### HUMAN CCR6-CRE REPORTER ASSAY

293FT cells were transfected with 2 constructs using TransIT-293 (Mirus). The first construct (pGL4.29, Promega) expressed the luciferase reporter gene luc2P in response to the binding of cAMP-induced CREB to the CRE element in its promoter. The second construct contained the open reading frame of human CCR6in the pUNO1 backbone under the control of an enhanced CMV promoter (InVivoGen). 48h after transfection, cells were harvested and diluted to 750,000 cells/ml in culture medium (DMEM, 10% FBS, 1x pen/strep (Gibco 15140)). Compounds were 2.5-fold serially diluted in DMSO. Further dilutions were made in assay medium (DMEM, 10% FBS, 1x pen/strep, 6µM Forskolin, 6nM CCL20). 4µl of the compound solution was added to a white MW384 plate, followed by 20µl of the cell suspension. Final DMSO concentration in the assay was 0.1%. The MW384 plate was placed in an incubator at37° C, 5% CO2 for 5h. Luciferase activity was determined by addition of 24 µl of a 2.5x diluted BriteLite luciferase solution (Perkin Elmer), followed by luminescence measurement using a VICTOR Plate reader (Perkin Elmer). To deselect compounds that did not specifically bind CCR6, the assay was also performed with transfected 293FT cells in which CCR6 was replaced with an empty vector using the same backbone. The relative -LogIC50 was determined with GraphPad Prism 9 using a four-parameter dose-response model.

### CHO-K1 CCR6 CHEMOTAXIS ASSAY TOWARDS CCL20

Cells overexpressing human CCR6 were used in the chemotaxis assay (DiscoverX, cAMP Hunter^{™} CHO-K1 CCR6 Gi Cell Line). Growth medium of these cells consisted of DMEM/F12, non-essential Amino acids (1xNEAA, Gibco 11140050), 10% FBS and 1x pen/strep (Gibco 15140). For the chemotaxis assay, Corning Transwell plates were used with a pore size of 8 µm (Corning 351164). Cells were harvested using trypsin and diluted to 4*10^6 cells/ml in chemotaxis medium (DMEM, 0.25% BSA, pen/strep). Compounds were four-fold serially diluted in DMSO. Further dilutions were made in chemotaxis medium. Cells were incubated with test compounds for 30 min at 37 °C prior to initiation of the assay (2*10^6 cells/ml). Final DMSO concentration in the assay was 0.2%. Wells of the Transwell plates were filled with 200 µl chemotaxis medium containing 100 ng/ml CCL20 (R&D systems 360-MP). 50 µl of the cell/compound suspension was added to the inserts of the Transwell plates (100,000 cells/insert). The Transwell plates were placed in an incubator at 37 °C, 5% CO2 for 4h. After 4h, the number of migrated cells was quantified using CellTiter Glo (Promega). Percentage inhibition values were calculated based on the low and high signal obtained with 0.2% DMSO in chemotaxis medium without and with CCL20, respectively. The relative -LogIC50 was determined with GraphPad Prism 9 using a four-parameter dose-response model.

### CD4+ T CELL CHEMOTAXIS ASSAY TOWARDS CCL20

Human CD4+ T cells were isolated from buffy coats of healthy donors using a CD4+T Cell Isolation Kit (Miltenyi Biotec130-096-533). Isolated T cells were stimulated overnight with anti-CD3 and anti-CD28 antibodies (Biolegend 300314/302934) in growth medium (RPMI1640, 10% heat-inactivated FBS, 1x pen/strep (Gibco 15140)). For the chemotaxis assay, Corning Transwell plates were used with a pore size of 5 µm (Corning 3388). T cells were counted and diluted to 6*10^6 cells/ml in chemotaxis medium (RPMI1640, 1% BSA, pen/strep). Compounds were four-fold serially diluted in DMSO. Further dilutions were made in chemotaxis medium. Cells were incubated with test compounds for 30 min at 37° C prior to initiation of the assay (3*10^6 cells/ml). Final DMSO concentration in the assay was 0.2%. Wells of the Transwell plates were filled with 200 µl chemotaxis medium containing 150 ng/ml CCL20 (R&D systems 360-MP). 50 µl of the cell/compound suspension was added to the inserts of the Transwell plates (150,000 cells/insert). The Transwell plates were placed in an incubator at 37 °C, 5% CO2 for 3 h. After 3 h, the number of migrated cells was quantified using Cell Titer Glo (Promega). Percentage inhibition values were calculated based on the low and high signal obtained with 0.2% DMSO in chemotaxis medium without and with CCL20, respectively. The relative -LogIC50 was determined with GraphPad Prism 9 using a four-parameter dose-response model.

The tables below show the data for selected compounds:

**Table 1: CCR6-CRE reporter assay**

| **Example Number** | **CCR6-CRE reporter** |
|---|---|
| 1 | 6.3 |
| 2 | 7.1 |
| 3 | 7.1 |
| 4 | 7.1 |
| 5 | 6.8 |
| 6 | 6.9 |
| 7 | 6.7 |
| 8 | 7.4 |
| 9 | 7.0 |
| 10 | 7.0 |
| 11 | 6.3 |
| 12 | 6.6 |
| 13 | 6.6 |
| 14 | 6.6 |
| 15 | 6.8 |
| 16 | 7.7 |
| 17 | 7.0 |
| 18 | 6.4 |
| 19 | 7.2 |
| 20 | 7.0 |
| 21 | 7.2 |
| 22 | 7.0 |
| 23 | 5.9 |
| 24 | 5.3 |
| 25 | 4.9 |
| 26 | 5.6 |
| 27 | 5.1 |
| 28 | 4.4 |
| 29 | 5.0 |
| 30 | 6.7 |
| 31 | 6.7 |
| 32 | 6.7 |
| 33 | 6.1 |
| 34 | 6.7 |
| 35 | 6.4 |
| 36 | 7.5 |
| 37 | 7.0 |
| 38 | 7.2 |
| 39 | 7.2 |
| 40 | 7.3 |
| 41 | 7.2 |
| 42 | 6.9 |
| 43 | 7.0 |
| 44 | 7.3 |
| 45 | 7.5 |
| 46 | 7.2 |

**Table 2: CHO-K1 CCR6 chemotaxis assay**

| **Example Number** | **CHO-K1 CCR6 chemotaxis** |
|---|---|
| 1 | |
| 2 | |
| 3 | 7.2 |
| 4 | 7.6 |
| 5 | 6.8 |
| 6 | 6.9 |
| 7 | 6.9 |
| 8 | |
| 9 | |
| 10 | |
| 11 | |
| 12 | 6.6 |
| 13 | |
| 14 | |
| 15 | |
| 16 | 8.3 |
| 17 | 7.4 |
| 18 | |
| 19 | 6.6 |
| 20 | 8.4 |
| 40 | 7.8 |
| 44 | 8.2 |
| 45 | 7.7 |
| 46 | 7.8 |

**Table 3: CD4+ T cell chemotaxis assay**

| **Example Number** | **CD4+ T-cell chemotaxis** |
|---|---|
| 1 | |
| 2 | 8.0 |
| 3 | |
| 4 | |
| 5 | |
| 6 | |
| 7 | |
| 8 | 8.5 |
| 9 | |
| 10 | |
| 11 | |
| 12 | |
| 13 | |
| 14 | |
| 15 | |
| 16 | |
| 17 | |
| 18 | |
| 19 | |
| 20 | |
| 21 | 8.8 |
| 22 | 8.6 |
| 23 | |
| 24 | |
| 25 | |
| 26 | |
| 27 | |
| 28 | |
| 29 | |
| 30 | 6.6 |
| 31 | 7.1 |
| 32 | 6.9 |
| 33 | |
| 34 | |
| 35 | 5.8 |
| 36 | 8.5 |
| 37 | 8.3 |
| 38 | |
| 39 | |
| 40 | 9.2 |
| 41 | 8.4 |
| 42 | 8.2 |
| 43 | 7.9 |
| 44 | 8.2 |
| 45 | 8.1 |
| 46 | 8.7 |
| 47 | 7.8 |

## Claims

1. A compound of formula (I) 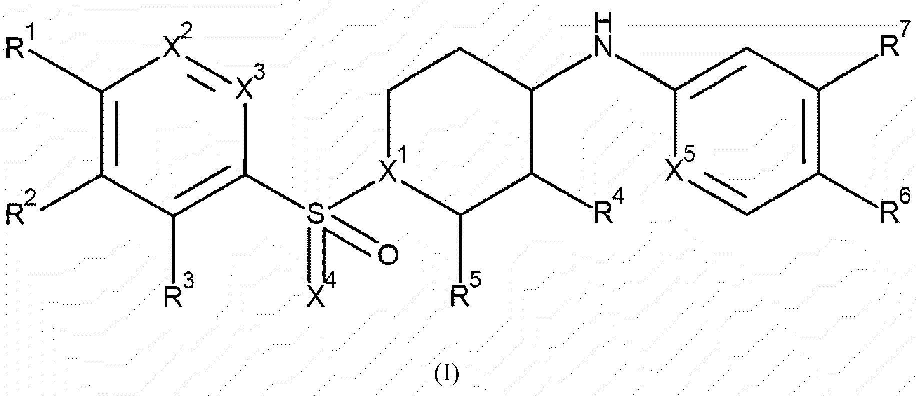 wherein
X¹ is CH or N;
X² is CH or N;
X³ is CH or N;
X⁴ is O or NH;
X⁵ is CH or N;
R¹ is aryl, heterocyclyl, or heteroaryl, wherein aryl, heterocyclyl, and heteroaryl are optionally substituted with one or two, in particular one R^{1a};
R^{1a} is C₁₋₆alkyl, C₁₋₆haloalkyl, oxo, cyano, -CONHR^{1b}, C₃₋₆cycloalkyl, or heterocyclyl;
R^{1b} is C₁₋₆alkyl, or hydrogen;
R² is hydrogen or halogen;
R³ is hydrogen or halogen;
R⁴ is hydrogen, halogen or C₁₋₆alkyl;
R⁵ is hydrogen, halogen or C₁₋₆alkyl;
R⁶ is C₃₋₆cycloalkyl or heterocyclyl, wherein C₃₋₆cycloalkyl and heterocyclyl are optionally substituted with one or two, in particular one R^{6a};
R^{6a} is C₁₋₆haloalkyl, cyano, or halogen
R⁷ is hydrogen;
and wherein
aryl refers to a cyclic aromatic hydrocarbon moiety having a mono-, bi- or tricyclic aromatic ring of 5 to 14 carbon ring atoms;
heteroaryl refers to an aromatic heterocyclic mono-, bi- or tricyclic ring system of 5 to 14 ring atoms, comprising 1, 2, 3 or 4 heteroatoms selected from N, O and S, the remaining ring atoms being carbon;
heterocyclyl refers to a 3, 4, 5, 6, 7, 8, 9, 10-membered monocyclic, 7, 8, 9 and 10-membered bicyclic (including bridged bicyclic and cycloalkyl spiro moieties) or 10, 11, 12, 13, 14 and 15-membered bicyclic heterocyclic moiety that is saturated or partially unsaturated, and has one or more (e.g., 1, 2, 3 or 4) heteroatoms selected from oxygen, nitrogen and sulfur in the ring with the remaining ring atoms being carbon.
or a pharmaceutically acceptable salt thereof.

2. The compound of claim 1 wherein
X² is CH;
X³ is CH;
X⁵ is CH;
R² is hydrogen;
R³ is hydrogen;
R⁴ is hydrogen;
R⁵ is hydrogen.

3. The compound of any one of claims 1 or 2 wherein R¹ is phenyl, pyridyl, or N containing bicyclic heteroaryl, wherein phenyl, pyridyl, and N containing bicyclic heteroaryl are optionally substituted with one or two, in particular one R^{1a}.

4. The compound of any one of claims 1 to 3, wherein R1a is methyl, isopropyl, cyano, - CONH₂, -CONH(Me), or difluoromethyl.

5. The compound of any one of claims 1 to 4 R^{1b} is methyl, or hydrogen.

6. The compound of any one of claims 1 to 5 wherein R⁶ is cyclopropyl, optionally substituted with one R^{6a}.

7. The compound of any one of claims 1 to 6 wherein R^{6a} is trifluoromethyl or cyano.

8. The compound of any one of claims 1 to 7 wherein the compound is of formula (I') 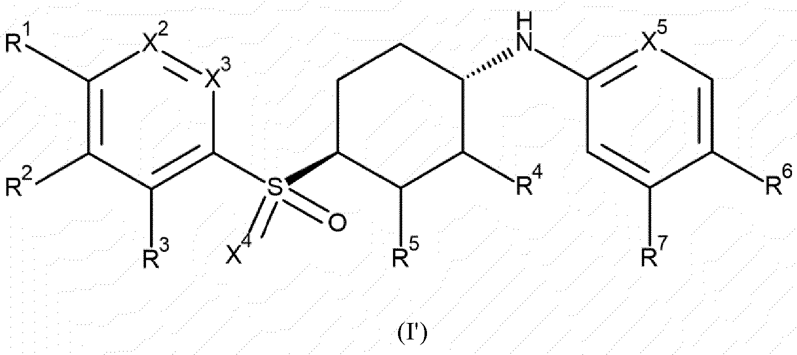 or a pharmaceutically acceptable salt thereof and R¹, R², R³, R⁴, R⁵, R⁶, R⁷, X², X³, X⁴, and X⁵ are as defined in any one of claims 1 to 7.

9. The compound of any one of claims 1 to 8 wherein the compound is of formula (I"') 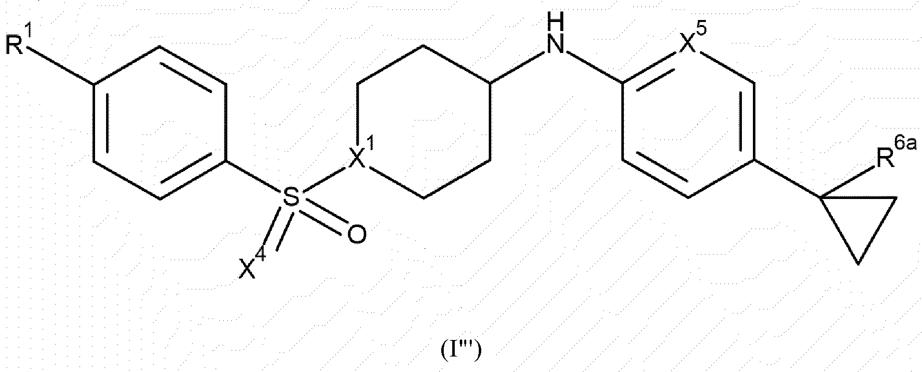 wherein R¹, R^{6a}, X¹, X⁴, and X⁵ are as defined in any one of claims 1 to 8.

10. The compound of any one of claims 1 to 9 selected from:
4'-[(4-{[4-(1-cyanocyclopropyl)phenyl]Amino}piperidin-1-yl)sulfonyl]-[1,1'-biphenyl]-4-carbonitrile;
1-[4-(3-methyl-1H-indazol-5-yl)benzenesulfonyl]-N-{4-[1-(trifluoromethyl)cyclopropyl]phenyl}piperidin-4-amine;
1-(4-{3-methylimidazo[1,2-a]pyridin-6-yl}benzenesulfonyl)-N-{4-[1-(trifluoromethyl)cyclopropyl]phenyl}piperidin-4-amine;
N-methyl-4'-{[4-({4-[1-(trifluoromethyl)cyclopropyl]phenyl}Amino)piperidin-1-yl]sulfonyl}-[1,1'-biphenyl]-3-carboxamide;
N-methyl-4'-{[4-({4-[1-(trifluoromethyl)cyclopropyl]phenyl}Amino)piperidin-1-yl]sulfonyl}-[1,1'-biphenyl]-4-carboxamide;
N-methyl-5-(4-{[4-({4-[1-(trifluoromethyl)cyclopropyl]phenyl}Amino)piperidin-1-yl]sulfonyl}phenyl)pyridine-2-carboxamide;
4'-{[4-({4-[1-(trifluoromethyl)cyclopropyl]phenyl}Amino)piperidin-1-yl]sulfonyl}-[1,1'-biphenyl]-4-carboxamide;
5-(4-{[4-({4-[1-(trifluoromethyl)cyclopropyl]phenyl}Amino)piperidin-1-yl]sulfonyl}phenyl)-1H-indole-3-carbonitrile;
1-(4-{imidazo[1,2-a]pyridin-6-yl}benzenesulfonyl)-N-{4-[1-(trifluoromethyl)cyclopropyl]phenyl}piperidin-4-amine;
1-{4-[3-(propan-2-yl)imidazo[1,2-a]pyridin-6-yl]benzenesulfonyl}-N-{4-[1-(trifluoromethyl)cyclopropyl]phenyl}piperidin-4-amine;
N-(4-cyclopropylphenyl)-1-[4-(3-methyl-1H-indazol-5-yl)benzenesulfonyl]piperidin-4-amine;
5-[4-({4-[(4-cyclopropylphenyl)Amino]piperidin-1-yl}sulfonyl)phenyl]-1H-indole-3-carbonitrile;
(4-{imidazo[1,2-a]pyridin-6-yl}phenyl)[trans-4-({4-[1-(trifluoromethyl)cyclopropyl]phenyl}Amino)cyclohexyl](imino)-λ⁶-sulfanone;
4-(2,2-difluorocyclopropyl)-N-[trans-4-(4-{imidazo[1,2-a]pyridin-6-yl}benzenesulfonyl)cyclohexyl]aniline;
1-(4-{[trans-4-(4-{imidazo[1,2-a]pyridin-6-yl}benzenesulfonyl)cyclohexyl]Amino}phenyl)cyclopropane-1-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
4-(oxetan-3-yl)-N-[trans-4-(4-{imidazo[1,2-a]pyridin-6-yl}benzenesulfonyl)cyclohexyl]aniline;
5-(4-{[trans-4-{[4-(oxetan-3-yl)phenyl]Amino}cyclohexyl]sulfonyl}phenyl)-1H-indole-3-carbonitrile;
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline;
N-[(trans)-4-(4-{3-methyl-1H-pyrrolo[2,3-b]pyridin-5-yl}benzenesulfonyl)cyclohexyl]-4-[1-(trifluoromethyl)cyclopropyl]aniline;
N-[(trans)-4-(4-{3-methyl-1H-pyrazolo[3,4-b]pyridin-5-yl}benzenesulfonyl)cyclohexyl]-4-[1-(trifluoromethyl)cyclopropyl]aniline;
1-(6-{[1-(4-{2-methoxy-7-azaspiro[3.5nonan-7-yl}benzenesulfonyl)piperidin-4-yl]amino}pyridin-3-yl)cyclopropane-1-carbonitrile;
2-(1-{4-[(4-{[5-(1-cyanocyclopropyl)pyridin-2-yl]amino}piperidin-1-yl)sulfonyl]phenyl}piperidin-4-yl)acetamide;
[(1-{4-[(4-{[5-(1-cyanocyclopropyl)pyridin-2-yl]amino}piperidin-1-yl)sulfonyl]phenyl}piperidin-4-yl)methyl]urea;
1-(6-{[1-(4-{8,10-dioxo-3,9-diazaspiro[5.5]undecan-3-yl}benzenesulfonyl)piperidin-4-yl]amino}pyridin-3-yl)cyclopropane-1-carbonitrile;
1-(6-{[1-(4-{2-oxo-3-oxa-1,8-diazaspiro[4.5]decan-8-yl}benzenesulfonyl)piperidin-4-yl]amino}pyridin-3-yl)cyclopropane-1-carbonitrile;
1-(6-{[1-(4-{1-oxo-2,8-diazaspiro[4.5]decan-8-yl}benzenesulfonyl)piperidin-4-yl]amino}pyridin-3-yl)cyclopropane-1-carbonitrile;
1-(6-{[1-(4-{2-oxa-7-azaspiro[3.5]nonan-7-yl}benzenesulfonyl)piperidin-4-yl]amino}pyridin-3-yl)cyclopropane-1-carbonitrile;
1-(6-{[(trans)-4-{4-[3-(propan-2-yl)-[1,2,4]triazolo[4,3-a]pyridin-6-yl]benzenesulfonyl}cyclohexyl]amino}pyridin-3-yl)cyclopropane-1-carbonitrile;
1-{6-[(1-{4-[3-(propan-2-yl)-[1,2,4]triazolo[4,3-a]pyridin-6-yl]benzenesulfonyl}piperidin-4-yl)amino]pyridin-3-yl}cyclopropane-1-carbonitrile;
1-{6-[(1-{4-[8-methyl-3-(propan-2-yl)-[1,2,4]triazolo[4,3-a]pyridin-6-yl]benzenesulfonyl}piperidin-4-yl)amino]pyridin-3-yl}cyclopropane-1-carbonitrile;
1-(6-{[1-(4-{imidazo[1,2-a]pyridin-6-yl}benzenesulfonyl)piperidin-4-yl]amino}pyridin-3-yl)cyclopropane-1-carbonitrile;
1-{6-[(1-{4-[3-(propan-2-yl)imidazo[1,2-a]pyridin-6-yl]benzenesulfonyl}piperidin-4-yl)amino]pyridin-3-yl}cyclopropane-1-carbonitrile;
1-(6-{[1-(4-{3-oxo-2H,3H-[1,2,4]triazolo[4,3-a]pyridin-6-yl}benzenesulfonyl)piperidin-4-yl]amino}pyridin-3-yl)cyclopropane-1-carbonitrile;
N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine;
N-[1-(4-{imidazo[1,2-a]pyridin-6-yl}benzenesulfonyl)piperidin-4-yl]-5-[1-(trifluoromethyl)cyclopropyl]pyridin-2-amine;
N-(1-{4-[3-(propan-2-yl)imidazo[1,2-a]pyridin-6-yl]benzenesulfonyl}piperidin-4-yl)-5-[1-(trifluoromethyl)cyclopropyl]pyridin-2-amine;
N-(1-{4-[3-(propan-2-yl)-[1,2,4]triazolo[4,3-a]pyrazin-6-yl]benzenesulfonyl}piperidin-4-yl)-5-[1-(trifluoromethyl)cyclopropyl]pyridin-2-amine;
1-{4-[3-(propan-2-yl)-[1,2,4]triazolo[4,3-a]pyridin-6-yl]benzenesulfonyl}-N-{4-[1-(trifluoromethyl)cyclopropyl]phenyl}piperidin-4-amine;
1-{4-[3-(propan-2-yl)-[1,2,4]triazolo[4,3-a]pyrazin-6-yl]benzenesulfonyl}-N-{4-[1-(trifluoromethyl)cyclopropyl]phenyl}piperidin-4-amine;
1-(4-{3-methyl-[1,2,4]triazolo[4,3-a]pyridin-6-yl}benzenesulfonyl)-N-{4-[1-(trifluoromethyl)cyclopropyl]phenyl}piperidin-4-amine;
6-(4-{[4-({4-[1-(trifluoromethyl)cyclopropyl]phenyl}amino)piperidin-1-yl]sulfonyl}phenyl)-2H,3H-[1,2,4]triazolo[4,3-a]pyridin-3-one
N-(1-{4-[3-(difluoromethyl)-[1,2,4]triazolo[4,3-a]pyridin-6-yl]benzenesulfonyl}piperidin-4-yl)-5-[1-(trifluoromethyl)cyclopropyl]pyridin-2-amine
4-(4-{[4-({5-[1-(trifluoromethyl)cyclopropyl]pyridin-2-yl}amino)piperidin-1-yl]sulfonyl}phenyl)pyridine-2-carboxamide
N-(1-{4-[8-methyl-3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine
or
N-[1-(4-{3-methyl-[1,2,4]triazolo[4,3-a]pyridin-6-yl}benzenesulfonyl)piperidin-4-yl]-5-[1-(trifluoromethyl)cyclopropyl]pyridin-2-amine.

11. The compound of any one of claims 1 to 10 selected from:
1-[4-(3-methyl-1H-indazol-5-yl)benzenesulfonyl]-N-{4-[1-(trifluoromethyl)cyclopropyl]phenyl}piperidin-4-amine;
5-(4-{[4-({4-[1-(trifluoromethyl)cyclopropyl]phenyl}Amino)piperidin-1-yl]sulfonyl}phenyl)-1H-indole-3-carbonitrile;
5-(4-{[trans-4-({4-[1-(trifluoromethyl)cyclopropyl]phenyl}Amino)cyclohexyl]sulfonyl}phenyl)-1H-indole-3-carbonitrile;
N-[trans-4-(4-{imidazo[1,2-a]pyridin-6-yl}benzenesulfonyl)cyclohexyl]-4-[1-(trifluoromethyl)cyclopropyl]aniline;
N-[trans-4-{4-[3-(propan-2-yl)imidazo[1,2-a]pyridin-6-yl]benzenesulfonyl}cyclohexyl]-4-[1-(trifluoromethyl)cyclopropyl]aniline;
N-(1-{4-[3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine;
1-{4-[3-(propan-2-yl)-[1,2,4]triazolo[4,3-a]pyridin-6-yl]benzenesulfonyl}-N-{4-[1-(trifluoromethyl)cyclopropyl]phenyl}piperidin-4-amine;
1-{4-[3-(propan-2-yl)-[1,2,4]triazolo[4,3-a]pyrazin-6-yl]benzenesulfonyl}-N-{4-[1-(trifluoromethyl)cyclopropyl]phenyl}piperidin-4-amine;
1-(4-{3-methyl-[1,2,4]triazolo[4,3-a]pyridin-6-yl}benzenesulfonyl)-N-{4-[1-(trifluoromethyl)cyclopropyl]phenyl}piperidin-4-amine
N-(1-{4-[3-(difluoromethyl)-[1,2,4]triazolo[4,3-a]pyridin-6-yl]benzenesulfonyl}piperidin-4-yl)-5-[1-(trifluoromethyl)cyclopropyl]pyridin-2-amine
N-(1-{4-[8-methyl-3-(propan-2-yl)-[1,2,4]triazolo[4,3-a]pyridin-6-yl]benzenesulfonyl}piperidin-4-yl)-5-[1-(trifluoromethyl)cyclopropyl]pyridin-2-amine
or
N-[1-(4-{3-methyl-[1,2,4]triazolo[4,3-a]pyridin-6-yl}benzenesulfonyl)piperidin-4-yl]-5-[1-(trifluoromethyl)cyclopropyl]pyridin-2-amine.

12. A compound according to any one of claims 1 to 11, or a pharmaceutically acceptable salt thereof, for use as a therapeutically active substance.

13. A pharmaceutical composition comprising a compound according to any one of claims 1 to 11, or a pharmaceutically acceptable salt thereof, and a pharmaceutically acceptable excipient.

14. A compound according to any one of claims 1 to 11, or a pharmaceutically acceptable salt thereof, for use in the treatment, prevention and/or delay of progression of an inflammatory autoimmune disease.

15. A compound according to any one of claims 1 to 11, or a pharmaceutically acceptable salt thereof, for use in the treatment, prevention and/or delay of progression of psoriatic diseases, asthma, Crohn's disease, ulcerative colitis, rheumatoid arthritis, systemic lupus erythematosus and multiple sclerosis.

## Patentansprüche

1. Verbindung der Formel (I) 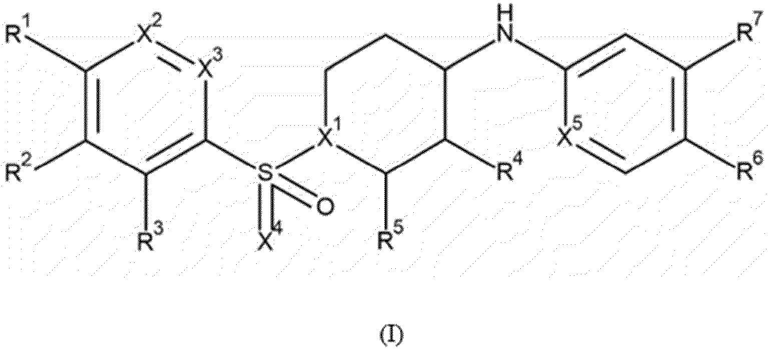 wobei
X¹ CH oder N ist;
X² CH oder N ist;
X³ CH oder N ist;
X⁴ O oder NH ist;
X⁵ CH oder N ist;
R¹ Aryl, Heterocyclyl oder Heteroaryl ist, wobei Aryl, Heterocyclyl und Heteroaryl gegebenenfalls mit einem oder zwei, insbesondere einem, R^{1a} substituiert sind;
R^{1a} C₁₋₆-Alkyl, C₁₋₆-Halogenalkyl, Oxo, Cyano, -CONHR^{1b}, C₃₋₆-Cycloalkyl oder Heterocyclyl ist;
R^{1b} C₁₋₆-Alkyl oder Wasserstoff ist;
R² Wasserstoff oder Halogen ist;
R³ Wasserstoff oder Halogen ist;
R⁴ Wasserstoff, Halogen oder C₁₋₆-Alkyl ist;
R⁵ Wasserstoff, Halogen oder C₁₋₆-Alkyl ist;
R⁶ C₃₋₆-Cycloalkyl oder Heterocyclyl ist, wobei C₃₋₆-Cycloalkyl und Heterocyclyl gegebenenfalls mit einem oder zwei, insbesondere einem, R^{6a} substituiert sind;
R^{6a} C₁₋₆-Halogenalkyl, Cyano oder Halogen ist;
R⁷ Wasserstoff ist;
und wobei
Aryl eine cyclische aromatische Kohlenwasserstoffeinheit mit einem mono-, bi- oder tricyclischen aromatischen Ring mit 5 bis 14 Kohlenstoffringatomen bezeichnet;
Heteroaryl ein aromatisches heterocyclisches mono-, bi- oder tricyclisches Ringsystem mit 5 bis 14 Ringatomen bezeichnet, umfassend 1, 2, 3 oder 4 Heteroatom(e), ausgewählt aus N, O und S, wobei die übrigen Ringatome Kohlenstoff sind;
Heterocyclyl eine 3-, 4-, 5-, 6-, 7-, 8-, 9-, 10-gliedrige monocyclische, 7-, 8-, 9- und 10-gliedrige bicyclische (einschließlich verbrückter bicyclischer und Cycloalkylspiroeinheiten) oder 10-, 11-, 12-, 13-, 14- und 15-gliedrige bicyclische heterocyclische Einheit bezeichnet, die gesättigt oder teilweise ungesättigt ist und ein oder mehrere (z. B. 1, 2, 3 oder 4) Heteroatom(e) in dem Ring aufweist, ausgewählt aus Sauerstoff, Stickstoff und Schwefel, wobei die übrigen Ringatome Kohlenstoff sind;
oder ein pharmazeutisch verträgliches Salz davon.

2. Verbindung nach Anspruch 1, wobei
X² CH ist;
X³ CH ist;
X⁵ CH ist;
R² Wasserstoff ist;
R³ Wasserstoff ist;
R⁴ Wasserstoff ist;
R⁵ Wasserstoff ist.

3. Verbindung nach einem der Ansprüche 1 oder 2, wobei R¹ Phenyl, Pyridyl oder N-haltiges bicyclisches Heteroaryl ist, wobei Phenyl, Pyridyl und N-haltiges bicyclisches Heteroaryl gegebenenfalls mit einem oder zwei, insbesondere einem, R^{1a} substituiert sind.

4. Verbindung nach einem der Ansprüche 1 bis 3, wobei R^{1a} Methyl, Isopropyl, Cyano, -CONH₂, -CONH(Me) oder Difluormethyl ist.

5. Verbindung nach einem der Ansprüche 1 bis 4, wobei R^{1b} Methyl oder Wasserstoff ist.

6. Verbindung nach einem der Ansprüche 1 bis 5, wobei R⁶ Cyclopropyl ist, gegebenenfalls substituiert mit einem R^{6a}.

7. Verbindung nach einem der Ansprüche 1 bis 6, wobei R^{6a} Trifluormethyl oder Cyano ist.

8. Verbindung nach einem der Ansprüche 1 bis 7, wobei die Verbindung die Formel (I') aufweist 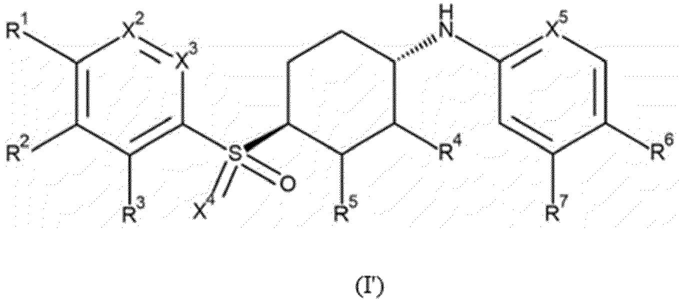 oder ein pharmazeutisch verträgliches Salz davon, und R¹, R², R³, R⁴, R⁵, R⁶, R⁷, X², X³, X⁴ und X⁵ wie in einem der Ansprüche 1 bis 7 definiert sind.

9. Verbindung nach einem der Ansprüche 1 bis 8, wobei die Verbindung die Formel (I"') aufweist 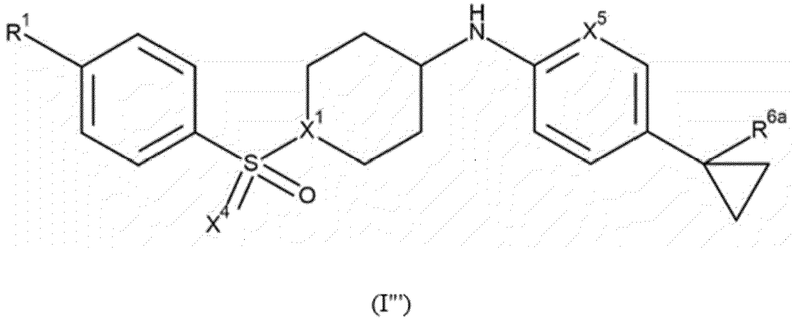 wobei R¹, R^{6a}, X¹, X⁴ und X⁵ wie in einem der Ansprüche 1 bis 8 definiert sind.

10. Verbindung nach einem der Ansprüche 1 bis 9, ausgewählt aus:
4'-[(4-{[4-(1-Cyanocyclopropyl)phenyl]amino}piperidin-1-yl)sulfonyl]-[1,1'-biphenyl]-4-carbonitril;
1-[4-(3-Methyl-1H-indazol-5-yl)benzolsulfonyl]-N-{4-[1-(trifluormethyl)cyclopropyl]phenyl}piperidin-4-amin;
1-(4-{3-Methylimidazo[1,2-a]pyridin-6-yl}benzolsulfonyl)-N-{4-[1-(trifluormethyl)cyclopropyl]phenyl}piperidin-4-amin;
N-Methyl-4'-{[4-({4-[1-(trifluormethyl)cyclopropyl]phenyl}amino)piperidin-1-yl]sulfonyl}-[1,1'-biphenyl]-3-carboxamid;
N-Methyl-4'-{[4-({4-[1-(trifluormethyl)cyclopropyl]phenyl}amino)piperidin-1-yl]sulfonyl}-[1,1'-biphenyl]-4-carboxamid;
N-Methyl-5-(4-{[4-({4-[1-(trifluormethyl)cyclopropyl]phenyl}amino)piperidin-1-yl]sulfonyl}phenyl)pyridin-2-carboxamid;
4'-{[4-({4-[1-(Trifluormethyl)cyclopropyl]phenyl}amino)piperidin-1-yl]sulfonyl}-[1,1'-biphenyl]-4-carboxamid;
5-(4-{[4-({4-[1-(Trifluormethyl)cyclopropyl]phenyl}amino)piperidin-1-yl]sulfonyl}phenyl)-1H-indol-3-carbonitril;
1-(4-{Imidazo[1,2-a]pyridin-6-yl}benzolsulfonyl)-N-{4-[1-(trifluormethyl)cyclopropyl]phenyl}piperidin-4-amin;
1-{4-[3-(Propan-2-yl)imidazo[1,2-a]pyridin-6-yl]benzolsulfonyl}-N-{4-[1-(trifluormethyl)cyclopropyl]phenyl}piperidin-4-amin;
N-(4-Cyclopropylphenyl)-1-[4-(3-methyl-1H-indazol-5-yl)benzolsulfonyl]piperidin-4-amin;
5-[4-({4-[(4-Cyclopropylphenyl)amino]piperidin-1-yl}sulfonyl)phenyl]-1H-indol-3-carbonitril;
(4-{Imidazo[1,2-a]pyridin-6-yl}phenyl)[trans-4-({4-[1-(trifluormethyl)cyclopropyl]phenyl}amino)cyclohexyl](imino)-λ⁶-sulfanon;
4-(2,2-Difluorcyclopropyl)-N-[trans-4-(4-{imidazo[1,2-a]pyridin-6-yl}benzolsulfonyl)cyclohexyl]anilin;
1-(4-{[trans-4-(4-{Imidazo[1,2-a]pyridin-6-yl}benzolsulfonyl)cyclohexyl]amino}phenyl)cyclopropan-1-carbonitril;
5-(4-{[trans-4-({4-[1-(Trifluormethyl)cyclopropyl]phenyl}amino)cyclohexyl]sulfonyl}phenyl)-1H-indol-3-carbonitril;
N-[trans-4-(4-{Imidazo[1,2-a]pyridin-6-yl}benzolsulfonyl)cyclohexyl]-4-[1-(trifluormethyl)cyclopropyl]anilin;
4-(Oxetan-3-yl)-N-[trans-4-(4-{imidazo[1,2-a]pyridin-6-yl}benzolsulfonyl)cyclohexyl]anilin;
5-(4-{[trans-4-{[4-(Oxetan-3-yl)phenyl]amino}cyclohexyl]sulfonyl}phenyl)-1H-indol-3-carbonitril;
N-[trans-4-{4-[3-(Propan-2-yl)imidazo[1,2-a]pyridin-6-yl]benzolsulfonyl}cyclohexyl]-4-[1-(trifluormethyl)cyclopropyl]anilin;
N-[(trans)-4-(4-{3-Methyl-1H-pyrrolo[2,3-b]pyridin-5-yl}benzolsulfonyl)cyclohexyl]-4-[1-(trifluormethyl)cyclopropyl]anilin;
N-[(trans)-4-(4-{3-Methyl-1H-pyrazolo[3,4-b]pyridin-5-yl}benzolsulfonyl)cyclohexyl]-4-[1-(trifluormethyl)cyclopropyl]anilin;
1-(6-{[1-(4-{2-Methoxy-7-azaspiro[3.5]nonan-7-yl}benzolsulfonyl)piperidin-4-yl]amino}pyridin-3-yl)cyclopropan-1-carbonitril;
2-(1-{4-[(4-{[5-(1-Cyanocyclopropyl)pyridin-2-yl]amino}piperidin-1-yl)sulfonyl]phenyl}piperidin-4-yl)acetamid;
[(1-{4-[(4-{[5-(1-Cyanocyclopropyl)pyridin-2-yl]amino}piperidin-1-yl)sulfonyl]phenyl}piperidin-4-yl)methyl]harnstoff;
1-(6-{[1-(4-{8,10-Dioxo-3,9-diazaspiro[5.5]undecan-3-yl}benzolsulfonyl)piperidin-4-yl]amino}pyridin-3-yl)cyclopropan-1-carbonitril;
1-(6-{[1-(4-{2-Oxo-3-oxa-1,8-diazaspiro[4.5]decan-8-yl}benzolsulfonyl)piperidin-4-yl]amino}pyridin-3-yl)cyclopropan-1-carbonitril;
1-(6-{[1-(4-{1-Oxo-2,8-diazaspiro[4.5]decan-8-yl}benzolsulfonyl)piperidin-4-yl]amino}pyridin-3-yl)cyclopropan-1-carbonitril;
1-(6-{[1-(4-{2-Oxa-7-azaspiro[3.5]nonan-7-yl}benzolsulfonyl)piperidin-4-yl]amino}pyridin-3-yl)cyclopropan-1-carbonitril;
1-(6-{[(trans)-4-{4-[3-(Propan-2-yl)-[1,2,4]triazolo[4,3-a]pyridin-6-yl]benzolsulfonyl}cyclohexyl]amino}pyridin-3-yl)cyclopropan-1-carbonitril;
1-{6-[(1-{4-[3-(Propan-2-yl)-[1,2,4]triazolo[4,3-a]pyridin-6-yl]benzolsulfonyl}piperidin-4-yl)amino]pyridin-3-yl}cyclopropan-1-carbonitril;
1-{6-[(1-{4-[8-Methyl-3-(propan-2-yl)-[1,2,4]triazolo[4,3-a]pyridin-6-yl]-benzolsulfonyl}piperidin-4-yl)amino]pyridin-3-yl}cyclopropan-1-carbonitril;
1-(6-{[1-(4-{Imidazo[1,2-a]pyridin-6-yl}benzolsulfonyl)piperidin-4-yl]amino}pyridin-3-yl)cyclopropan-1-carbonitril;
1-{6-[(1-{4-[3-(Propan-2-yl)imidazo[1,2-a]pyridin-6-yl]benzolsulfonyl}piperidin-4-yl)-amino]pyridin-3-yl}cyclopropan-1-carbonitril;
1-(6-{[1-(4-{3-Oxo-2H,3H-[1,2,4]triazolo[4,3-a]pyridin-6-yl}benzolsulfonyl)piperidin-4-yl]amino}pyridin-3-yl)cyclopropan-1-carbonitril;
N-(1-{4-[3-(Propan-2-yl)-[1,2,4]triazolo[4,3-a]pyridin-6-yl]benzolsulfonyl}piperidin-4-yl)-5-[1-(trifluormethyl)cyclopropyl]pyridin-2-amin;
N-[1-(4-{Imidazo[1,2-a]pyridin-6-yl}benzolsulfonyl)piperidin-4-yl]-5-[1-(trifluormethyl)cyclopropyl]pyridin-2-amin;
N-(1-{4-[3-(Propan-2-yl)imidazo[1,2-a]pyridin-6-yl]benzolsulfonyl}piperidin-4-yl)-5-[1-(trifluormethyl)cyclopropyl]pyridin-2-amin;
N-(1-{4-[3-(Propan-2-yl)-[1,2,4]triazolo[4,3-a]pyrazin-6-yl]benzolsulfonyl}piperidin-4-yl)-5-[1-(trifluormethyl)cyclopropyl]pyridin-2-amin;
1-{4-[3-(Propan-2-yl)-[1,2,4]triazolo[4,3-a]pyridin-6-yl]benzolsulfonyl}-N-{4-[1-(trifluormethyl)cyclopropyl]phenyl}piperidin-4-amin;
1-{4-[3-(Propan-2-yl)-[1,2,4]triazolo[4,3-a]pyrazin-6-yl]benzolsulfonyl}-N-{4-[1-(trifluormethyl)cyclopropyl]phenyl}piperidin-4-amin;
1-(4-{3-Methyl-[1,2,4]triazolo[4,3-a]pyridin-6-yl}benzolsulfonyl)-N-{4-[1-(trifluormethyl)cyclopropyl]phenyl}piperidin-4-amin;
6-(4-{[4-({4-[1-(Trifluormethyl)cyclopropyl]phenyl}amino)piperidin-1-yl]sulfonyl}phenyl)-2H,3H-[1,2,4]triazolo[4,3-a]pyridin-3-on
N-(1-{4-[3-(Difluormethyl)-[1,2,4]triazolo[4,3-a]pyridin-6-yl]benzolsulfonyl}piperidin-4-yl)-5-[1-(trifluormethyl)cyclopropyl]pyridin-2-amin
4-(4-{[4-({5-[1-(Trifluormethyl)cyclopropyl]pyridin-2-yl}amino)piperidin-1-yl]sulfonyl}phenyl)pyridin-2-carboxamid
N-(1-{4-[8-Methyl-3-(propan-2-yl)-[1,2,4]triazolo[4,3-a]pyridin-6-yl]benzolsulfonyl}piperidin-4-yl)-5-[1-(trifluormethyl)cyclopropyl]pyridin-2-amin
oder
N-[1-(4-{3-Methyl-[1,2,4]triazolo[4,3-a]pyridin-6-yl}benzolsulfonyl)piperidin-4-yl]-5-[1-(trifluormethyl)cyclopropyl]pyridin-2-amin.

11. Verbindung nach einem der Ansprüche 1 bis 10, ausgewählt aus:
1-[4-(3-Methyl-1H-indazol-5-yl)benzolsulfonyl]-N-{4-[1-(trifluormethyl)cyclopropyl]phenyl}piperidin-4-amin;
5-(4-{[4-({4-[1-(Trifluormethyl)cyclopropyl]phenyl}amino)piperidin-1-yl]sulfonyl}phenyl)-1H-indol-3-carbonitril;
5-(4-{[trans-4-({4-[1-(Trifluormethyl)cyclopropyl]phenyl}amino)cyclohexyl]sulfonyl}phenyl)-1H-indol-3-carbonitril;
N-[trans-4-(4-{Imidazo[1,2-a]pyridin-6-yl}benzolsulfonyl)cyclohexyl]-4-[1-(trifluormethyl)cyclopropyl]anilin;
N-[trans-4-{4-[3-(Propan-2-yl)imidazo[1,2-a]pyridin-6-yl]benzolsulfonyl}cyclohexyl]-4-[1-(trifluormethyl)cyclopropyl]anilin;
N-(1-{4-[3-(Propan-2-yl)-[1,2,4]triazolo[4,3-a]pyridin-6-yl]benzolsulfonyl}piperidin-4-yl)-5-[1-(trifluormethyl)cyclopropyl]pyridin-2-amin;
1-{4-[3-(Propan-2-yl)-[1,2,4]triazolo[4,3-a]pyridin-6-yl]benzolsulfonyl}-N-{4-[1-(trifluormethyl)cyclopropyl]phenyl}piperidin-4-amin;
1-{4-[3-(Propan-2-yl)-[1,2,4]triazolo[4,3-a]pyrazin-6-yl]benzolsulfonyl}-N-{4-[1-(trifluormethyl)cyclopropyl]phenyl}piperidin-4-amin;
1-(4-{3-Methyl-}1,2,4]triazolo[4,3-a]pyridin-6-yl}benzolsulfonyl)-N-{4-[1-(trifluormethyl)cyclopropyl]phenyl}piperidin-4-amin
N-(1-{4-[3-(Difluormethyl)-[1,2,4]triazolo[4,3-a]pyridin-6-yl]benzolsulfonyl}piperidin-4-yl)-5-[1-(trifluormethyl)cyclopropyl]pyridin-2-amin
N-(1-{4-[8-Methyl-3-(propan-2-yl)-[1,2,4]triazolo[4,3-a]pyridin-6-yl]benzolsulfonyl}piperidin-4-yl)-5-[1-(trifluormethyl)cyclopropyl]pyridin-2-amin
oder
N-[1-(4-{3-Methyl-[1,2,4]triazolo[4,3-a]pyridin-6-yl}benzolsulfonyl)piperidin-4-yl]-5-[1-(trifluormethyl)cyclopropyl]pyridin-2-amin.

12. Verbindung nach einem der Ansprüche 1 bis 11 oder ein pharmazeutisch verträgliches Salz davon zur Verwendung als therapeutisch wirksame Substanz.

13. Pharmazeutische Zusammensetzung, umfassend eine Verbindung nach einem der Ansprüche 1 bis 11 oder ein pharmazeutisch verträgliches Salz davon und einen pharmazeutisch verträglichen Hilfsstoff.

14. Verbindung nach einem der Ansprüche 1 bis 11 oder ein pharmazeutisch verträgliches Salz davon zur Verwendung bei der Behandlung, Prävention und/oder Verzögerung des Fortschreitens einer entzündlichen Autoimmunerkrankung.

15. Verbindung nach einem der Ansprüche 1 bis 11 oder ein pharmazeutisch verträgliches Salz davon zur Verwendung bei der Behandlung, Prävention und/oder Verzögerung des Fortschreitens von Psoriasiserkrankungen, Asthma, Morbus Crohn, Colitis ulcerosa, rheumatoider Arthritis, systemischem Lupus erythematodes und multipler Sklerose.

## Revendications

1. Composé de formule (I) 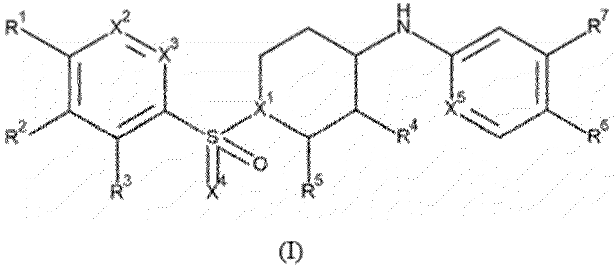 dans lequel
X¹ représente CH ou N ;
X² représente CH ou N ;
X³ représente CH ou N ;
X⁴ représente O ou NH ;
X⁵ représente CH ou N ;
R¹ représente un aryle, un hétérocyclyle ou un hétéroaryle, l'aryle, l'hétérocyclyle et l'hétéroaryle étant éventuellement substitués par un ou deux, en particulier un R^{1a} ;
R^{1a} représente un alkyle en C₁₋₆, un halogénoalkyle en C₁₋₆, un oxo, un cyano, -CONHR^{1b}, un cycloalkyle en C₃₋₆ ou un hétérocyclyle ;
R^{1b} représente un alkyle en C₁₋₆ ou un hydrogène ;
R² représente un hydrogène ou un halogène ;
R³ représente un hydrogène ou un halogène ;
R⁴ représente un hydrogène, un halogène ou un alkyle en C₁₋₆ ;
R⁵ représente un hydrogène, un halogène ou un alkyle en C₁₋₆ ;
R⁶ représente un cycloalkyle en C₃₋₆ ou un hétérocyclyle, le cycloalkyle en C₃₋₆ et l'hétérocyclyle étant éventuellement substitués par un ou deux, en particulier un R^{6a} ;
R^{6a} représente un halogénoalkyle en C₁₋₆, un cyano ou un halogène
R⁷ représente un hydrogène ;
et dans lequel
l'aryle fait référence à une fraction hydrocarbonée aromatique cyclique ayant un cycle aromatique mono-, bi- ou tricyclique de 5 à 14 atomes de carbone sur le cycle ;
l'hétéroaryle fait référence à un système cyclique mono-, bi- ou tricyclique hétérocyclique aromatique de 5 à 14 atomes sur le cycle, comprenant 1, 2, 3 ou 4 hétéroatomes choisis parmi N, O et S, les atomes du cycle restants étant des carbones ;
l'hétérocyclyle fait référence à une fraction hétérocyclique monocyclique à 3, 4, 5, 6, 7, 8, 9 ou 10 chaînons, bicyclique à 7, 8, 9 ou 10 chaînons (y compris des fractions bicycliques pontées et spiro cycloalkyliques) ou bicyclique à 10, 11, 12, 13, 14 ou 15 chaînons qui est saturée ou partiellement insaturée et possède dans le cycle un ou plusieurs (par ex. 1, 2, 3 ou 4) hétéroatomes choisis parmi l'oxygène, l'azote et le soufre, les atomes du cycle restants étant des carbones
ou un sel pharmaceutiquement acceptable de celui-ci.

2. Composé selon la revendication 1 dans lequel
X² représente CH ;
X³ représente CH ;
X⁵ représente CH ;
R² représente un hydrogène ;
R³ représente un hydrogène ;
R⁴ représente un hydrogène ;
R⁵ représente un hydrogène.

3. Composé selon l'une quelconque des revendications 1 ou 2 dans lequel R¹ représente un phényle, un pyridyle ou un hétéroaryle bicyclique contenant N, le phényle, le pyridyle et l'hétéroaryle bicyclique contenant N étant éventuellement substitués par un ou deux, en particulier un R^{1a}.

4. Composé selon l'une quelconque des revendications 1 à 3, dans lequel R^{1a} représente un méthyle, un isopropyle, un cyano, -CONH₂, -CONH(Me) ou un difluorométhyle.

5. Composé selon l'une quelconque des revendications 1 à 4 dans lequel R^{1b} représente un méthyle ou un hydrogène.

6. Composé selon l'une quelconque des revendications 1 à 5 dans lequel R⁶ représente un cyclopropyle, éventuellement substitué par un R^{6a}.

7. Composé selon l'une quelconque des revendications 1 à 6 dans lequel R^{6a} représente un trifluorométhyle ou un cyano.

8. Composé selon l'une quelconque des revendications 1 à 7 dans lequel le composé est de formule (I') 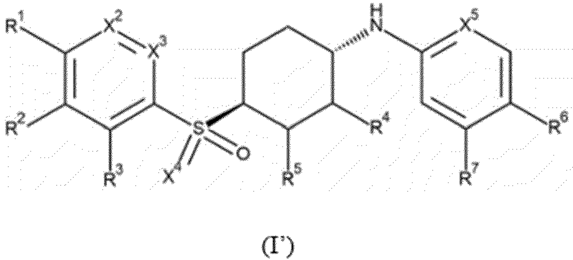 ou un sel pharmaceutiquement acceptable de celui-ci et R¹, R², R³, R⁴, R⁵, R⁶, R⁷, X², X³, X⁴ et X⁵ sont tels que définis dans l'une quelconque des revendications 1 à 7.

9. Composé selon l'une quelconque des revendications 1 à 8 dans lequel le composé est de formule (I‴) 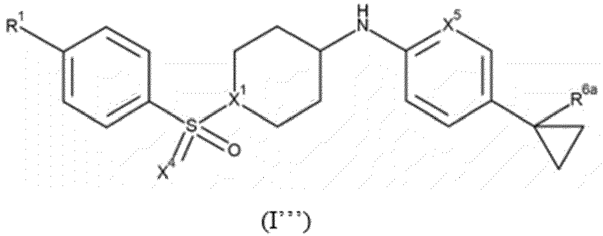 dans lequel R¹, R^{6a}, X¹, X⁴ et X⁵ sont tels que définis dans l'une quelconque des revendications 1 à 8.

10. Composé selon l'une quelconque des revendications 1 à 9 choisi parmi :
le 4'-[(4-{[4-(1-cyanocyclopropyl)phényl]amino}pipéridin-1-yl)sulfonyl]-[1,1'-biphényl]-4-carbonitrile ;
la 1-[4-(3-méthyl-1H-indazol-5-yl)benzènesulfonyl]-N-{4-[1-(trifluorométhyl)cyclopropyl]phényl}pipéridin-4-amine ;
la 1-(4-{3-méthylimidazo[1,2-a]pyridin-6-yl}benzènesulfonyl)-N-{4-[1-(trifluorométhyl)cyclopropyl]phényl}pipéridin-4-amine ;
le N-méthyl-4'-{[4-({4-[1-(trifluorométhyl)cyclopropyl]phényl}amino)pipéridin-1-yl]sulfonyl}-[1,1'-biphényl]-3-carboxamide ;
le N-méthyl-4'-{[4-({4-[1-(trifluorométhyl)cyclopropyl]phényl}amino)pipéridin-1-yl]sulfonyl}-[1,1'-biphényl]-4-carboxamide ;
le N-méthyl-5-(4-{[4-({4-[1-(trifluorométhyl)cyclopropyl]phényl}amino)pipéridin-1-yl]sulfonyl}phényl)pyridine-2-carboxamide ;
le 4'-{[4-({4-[1-(trifluorométhyl)cyclopropyl]phényl}amino)pipéridin-1-yl]sulfonyl}-[1,1'-biphényl]-4-carboxamide ;
le 5-(4-{[4-({4-[1-(trifluorométhyl)cyclopropyl]phényl}amino)pipéridin-1-yl]sulfonyl}phényl)-1H-indole-3-carbonitrile ;
la 1-(4-{imidazo[1,2-a]pyridin-6-yl}benzènesulfonyl)-N-{4-[1-(trifluorométhyl)cyclopropyl]phényl}pipéridin-4-amine ;
la 1-{4-[3-(propan-2-yl)imidazo[1,2-a]pyridin-6-yl]benzènesulfonyl}-N-{4-[1-(trifluorométhyl)cyclopropyl]phényl}pipéridin-4-amine ;
la N-(4-cyclopropylphényl)-1-[4-(3-méthyl-1H-indazol-5-yl)benzènesulfonyl]pipéridin-4-amine ;
le 5-[4-({4-[(4-cyclopropylphényl)amino]pipéridin-1-yl}sulfonyl)phényl]-1H-indole-3-carbonitrile ;
la (4-{imidazo[1,2-a]pyridin-6-yl}phényl)[trans-4-({4-[1-(trifluorométhyl)cyclopropyl]phényl}amino)cyclohexyl](imino)-λ⁶-sulfanone ;
la 4-(2,2-difluorocyclopropyl)-N-[trans-4-(4-{imidazo[1,2-a]pyridin-6-yl}benzènesulfonyl)cyclohexyl]aniline ;
le 1-(4-{[trans-4-(4-{imidazo[1,2-a]pyridin-6-yl}benzènesulfonyl)cyclohexyl]amino}phényl)cyclopropane-1-carbonitrile ;
le 5-(4-{[trans-4-({4-[1-(trifluorométhyl)cyclopropyl]phényl}amino)cyclohexyl]sulfonyl}phényl)-1H-indole-3-carbonitrile ;
la N-[trans-4-(4-{imidazo[1,2-a]pyridin-6-yl}benzènesulfonyl)cyclohexyl]-4-[1-(trifluorométhyl)cyclopropyl]aniline ;
la 4-(oxétan-3-yl)-N-[trans-4-(4-{imidazo[1,2-a]pyridin-6-yl}benzènesulfonyl)cyclohexyl]aniline ;
le 5-(4-{[trans-4-{[4-(oxétan-3-yl)phényl]amino}cyclohexyl]sulfonyl}phényl)-1H-indole-3-carbonitrile ;
la N-[trans-4-{4-[3-(propan-2-yl)imidazo[1,2-a]pyridin-6-yl]benzènesulfonyl}cyclohexyl]-4-[1-(trifluorométhyl)cyclopropyl]aniline ;
la N-[(trans)-4-(4-{3-méthyl-1H-pyrrolo[2,3-b]pyridin-5-yl}benzènesulfonyl)cyclohexyl]-4-[1-(trifluorométhyl)cyclopropyl]aniline ;
la N-[(trans)-4-(4-{3-méthyl-1H-pyrazolo[3,4-b]pyridin-5-yl}benzènesulfonyl)cyclohexyl]-4-[1-(trifluorométhyl)cyclopropyl]aniline ;
le 1-(6-{[1-(4-{2-méthoxy-7-azaspiro[3.5]nonan-7-yl}benzènesulfonyl)pipéridin-4-yl]amino}pyridin-3-yl)cyclopropane-1-carbonitrile ;
le 2-(1-{4-[(4-{[5-(1-cyanocyclopropyl)pyridin-2-yl]amino}pipéridin-1-yl)sulfonyl]phényl}pipéridin-4-yl)acétamide ;
la [(1-{4-[(4-{[5-(1-cyanocyclopropyl)pyridin-2-yl]amino}pipéridin-1-yl)sulfonyl]phényl}pipéridin-4-yl)méthyl]urée ;
le 1-(6-{[1-(4-{8,10-dioxo-3,9-diazaspiro[5.5]undécan-3-yl}benzènesulfonyl)pipéridin-4-yl]amino}pyridin-3-yl)cyclopropane-1-carbonitrile ;
le 1-(6-{[1-(4-{2-oxo-3-oxa-1,8-diazaspiro[4.5]décan-8-yl}benzènesulfonyl)pipéridin-4-yl]amino}pyridin-3-yl)cyclopropane-1-carbonitrile ;
le 1-(6-{[1-(4-{1-oxo-2,8-diazaspiro[4.5]décan-8-yl}benzènesulfonyl)pipéridin-4-yl]amino}pyridin-3-yl)cyclopropane-1-carbonitrile ;
le 1-(6-{[1-(4-{2-oxa-7-azaspiro[3.5]nonan-7-yl}benzènesulfonyl)pipéridin-4-yl]amino}pyridin-3-yl)cyclopropane-1-carbonitrile ;
le 1-(6-{[(trans)-4-{4-[3-(propan-2-yl)-[1,2,4]triazolo[4,3-a]pyridin-6-yl]benzènesulfonyl}cyclohexyl]amino}pyridin-3-yl)cyclopropane-1-carbonitrile ;
le 1-{6-[(1-{4-[3-(propan-2-yl)-[1,2,4]triazolo[4,3-a]pyridin-6-yl]benzènesulfonyl}pipéridin-4-yl)amino]pyridin-3-yl}cyclopropane-1-carbonitrile ;
le 1-{6-[(1-{4-[8-méthyl-3-(propan-2-yl)-[1,2,4]triazolo[4,3-a]pyridin-6-yl]benzènesulfonyl}pipéridin-4-yl)amino]pyridin-3-yl}cyclopropane-1-carbonitrile ;
le 1-(6-{[1-(4-{imidazo[1,2-a]pyridin-6-yl}benzènesulfonyl)pipéridin-4-yl]amino}pyridin-3-yl)cyclopropane-1-carbonitrile ;
le 1-{6-[(1-{4-[3-(propan-2-yl)imidazo[1,2-a]pyridin-6-yl]benzènesulfonyl}pipéridin-4-yl)amino]pyridin-3-yl}cyclopropane-1-carbonitrile ;
le 1-(6-{[1-(4-{3-oxo-2H,3H-[1,2,4]triazolo[4,3-a]pyridin-6-yl}benzènesulfonyl)pipéridin-4-yl]amino}pyridin-3-yl)cyclopropane-1-carbonitrile ;
la N-(1-{4-[3-(propan-2-yl)-[1,2,4]triazolo[4,3-a]pyridin-6-yl]benzènesulfonyl}pipéridin-4-yl)-5-[1-(trifluorométhyl)cyclopropyl]pyridin-2-amine ;
la N-[1-(4-{imidazo[1,2-a]pyridin-6-yl}benzènesulfonyl)pipéridin-4-yl]-5-[1-(trifluorométhyl)cyclopropyl]pyridin-2-amine ;
la N-(1-{4-[3-(propan-2-yl)imidazo[1,2-a]pyridin-6-yl]benzènesulfonyl}pipéridin-4-yl)-5-[1-(trifluorométhyl)cyclopropyl]pyridin-2-amine ;
la N-(1-{4-[3-(propan-2-yl)-[1,2,4]triazolo[4,3-a]pyrazin-6-yl]benzènesulfonyl}pipéridin-4-yl)-5-[1-(trifluorométhyl)cyclopropyl]pyridin-2-amine ;
la 1-{4-[3-(propan-2-yl)-[1,2,4]triazolo[4,3-a]pyridin-6-yl]benzènesulfonyl}-N-{4-[1-(trifluorométhyl)cyclopropyl]phényl}pipéridin-4-amine ;
la 1-{4-[3-(propan-2-yl)-[1,2,4]triazolo[4,3-a]pyrazin-6-yl]benzènesulfonyl}-N-{4-[1-(trifluorométhyl)cyclopropyl]phényl}pipéridin-4-amine ;
la 1-(4-{3-méthyl-[1,2,4]triazolo[4,3-a]pyridin-6-yl}benzènesulfonyl)-N-{4-[1-(trifluorométhyl)cyclopropyl]phényl}pipéridin-4-amine ;
la 6-(4-{[4-({4-[1-(trifluorométhyl)cyclopropyl]phényl}amino)pipéridin-1-yl]sulfonyl}phényl)-2H,3H-[1,2,4]triazolo[4,3-a]pyridin-3-one
la N-(1-{4-[3-(difluorométhyl)-[1,2,4]triazolo[4,3-a]pyridin-6-yl]benzènesulfonyl}pipéridin-4-yl)-5-[1-(trifluorométhyl)cyclopropyl]pyridin-2-amine
le 4-(4-{[4-({5-[1-(trifluorométhyl)cyclopropyl]pyridin-2-yl}amino)pipéridin-1-yl]sulfonyl}phényl)pyridine-2-carboxamide
la N-(1-{4-[8-méthyl-3-(propan-2-yl)-[1,2,4]triazolo[4,3-a]pyridin-6-yl]benzènesulfonyl}pipéridin-4-yl)-5-[1-(trifluorométhyl)cyclopropyl]pyridin-2-amine
ou
la N-[1-(4-{3-méthyl-[1,2,4]triazolo[4,3-a]pyridin-6-yl}benzènesulfonyl)pipéridin-4-yl]-5-[1-(trifluorométhyl)cyclopropyl]pyridin-2-amine.

11. Composé selon l'une quelconque des revendications 1 à 10 choisi parmi :
la 1-[4-(3-méthyl-1H-indazol-5-yl)benzènesulfonyl]-N-{4-[1-(trifluorométhyl)cyclopropyl]phényl}pipéridin-4-amine ;
le 5-(4-{[4-({4-[1-(trifluorométhyl)cyclopropyl]phényl}amino)pipéridin-1-yl]sulfonyl}phényl)-1H-indole-3-carbonitrile ;
le 5-(4-{[trans-4-({4-[1-(trifluorométhyl)cyclopropyl]phényl}amino)cyclohexyl]sulfonyl}phényl)-1H-indole-3-carbonitrile ;
la N-[trans-4-(4-{imidazo[1,2-a]pyridin-6-yl}benzènesulfonyl)cyclohexyl]-4-[1-(trifluorométhyl)cyclopropyl]aniline ;
la N-[trans-4-{4-[3-(propan-2-yl)imidazo[1,2-a]pyridin-6-yl]benzènesulfonyl}cyclohexyl]-4-[1-(trifluorométhyl)cyclopropyl]aniline ;
la N-(1-{4-[3-(propan-2-yl)-[1,2,4]triazolo[4,3-a]pyridin-6-yl]benzènesulfonyl}pipéridin-4-yl)-5-[1-(trifluorométhyl)cyclopropyl]pyridin-2-amine ;
la 1-{4-[3-(propan-2-yl)-[1,2,4]triazolo[4,3-a]pyridin-6-yl]benzènesulfonyl}-N-{4-[1-(trifluorométhyl)cyclopropyl]phényl}pipéridin-4-amine ;
la 1-{4-[3-(propan-2-yl)-[1,2,4]triazolo[4,3-a]pyrazin-6-yl]benzènesulfonyl}-N-{4-[1-(trifluorométhyl)cyclopropyl]phényl}pipéridin-4-amine ;
la 1-(4-{3-méthyl-[1,2,4]triazolo[4,3-a]pyridin-6-yl}benzènesulfonyl)-N-{4-[1-(trifluorométhyl)cyclopropyl]phényl}pipéridin-4-amine
la N-(1-{4-[3-(difluorométhyl)-[1,2,4]triazolo[4,3-a]pyridin-6-yl]benzènesulfonyl}pipéridin-4-yl)-5-[1-(trifluorométhyl)cyclopropyl]pyridin-2-amine
la N-(1-{4-[8-méthyl-3-(propan-2-yl)-[1,2,4]triazolo[4,3-a]pyridin-6-yl]benzènesulfonyl}pipéridin-4-yl)-5-[1-(trifluorométhyl)cyclopropyl]pyridin-2-amine
ou
la N-[1-(4-{3-méthyl-[1,2,4]triazolo[4,3-a]pyridin-6-yl}benzènesulfonyl)pipéridin-4-yl]-5-[1-(trifluorométhyl)cyclopropyl]pyridin-2-amine.

12. Composé selon l'une quelconque des revendications 1 à 11, ou un sel pharmaceutiquement acceptable de celui-ci, pour une utilisation comme substance thérapeutiquement active.

13. Composition pharmaceutique comprenant un composé selon l'une quelconque des revendications 1 à 11, ou un sel pharmaceutiquement acceptable de celui-ci, et un excipient pharmaceutiquement acceptable.

14. Composé selon l'une quelconque des revendications 1 à 11, ou un sel pharmaceutiquement acceptable de celui-ci, pour une utilisation dans le traitement, la prévention et/ou le ralentissement de la progression d'une maladie auto-immune inflammatoire.

15. Composé selon l'une quelconque des revendications 1 à 11, ou un sel pharmaceutiquement acceptable de celui-ci, pour une utilisation dans le traitement, la prévention et/ou le ralentissement de la progression de maladies psoriasiques, de l'asthme, de la maladie de Crohn, de la rectocolite hémorragique, de la polyarthrite rhumatoïde, du lupus érythémateux systémique et de la sclérose en plaques.
